# EUROPEAN PATENT SPECIFICATION

(11) **EP 3 474 886 B1**
(45) Date of publication and mention of the grant of the patent: **04.08.2021**
(21) Application number: 16739617.5
(22) Date of filing: 27.06.2016
(51) Int. Cl.: A61K 38/46, A61P 19/00

(54) **METHODS FOR TREATING HYPOPHOSPHATASIA IN CHILDREN AND ADOLESCENTS**
VERFAHREN ZUR BEHANDLUNG VON HYPOPHOSPHATASIE BEI KINDERN UND JUGENDLICHEN
MÉTHODES DE TRAITEMENT DE L'HYPOPHOSPHATASIE CHEZ L'ENFANT ET L'ADOLESCENT

(43) Date of publication of application: 01.05.2019
(73) Proprietor: Alexion Pharmaceuticals, Inc., New Haven, CT 06510 (US)
(72) Inventor: FUJITA, Kenji, Milburn, NJ 07041 (US); VALLEE, Marc, Concord, MA 01742 (US); PHILLIPS, Dawn, Chapel Hill, NC 27517 (US)
(74) Representative: D Young & Co LLP
(86) International application number: PCT/US2016/039595
(87) International publication number: WO 2018/004517

(56) References cited:
- DAWN PHILLIPS ET AL: "A modified performance-oriented mobility assessment tool for assessing clinically relevant gait impairments and change in children with hypophosphatasia: development and validation", BONE ABSTRACTS, 1 July 2015 (2015-07-01), XP055339488, ISSN: 2052-1219, DOI: 10.1530/boneabs.4.P136
- Anonymous: "HIGHLIGHTS OF PRESCRIBING INFORMATION", , 1 October 2015 (2015-10-01), XP055339497, Retrieved from the Internet: URL:http://www.alexion.com/Documents/stren siq_pi-10-2015.aspx [retrieved on 2017-01-26]
- Anonymous: "Gait Assessment in Children with Childhood Hypophosphatasia: Impairments in Muscle Strength and Physical Function", , 1 January 2015 (2015-01-01), XP055339649, Retrieved from the Internet: URL:https://endo.confex.com/endo/2015endo/ webprogram/Paper22842.html [retrieved on 2017-01-27] & Anonymous: "Printing ENDO 2015: Hypophosphatasia in the Spotlight", , 1 January 2015 (2015-01-01), XP055339774, Retrieved from the Internet: URL:http://www.raredr.com/print.php [retrieved on 2017-01-27]
- Anonymous: "NCT02235493 on 2015_11_19: ClinicalTrials.gov Archive", , 19 November 2015 (2015-11-19), XP055339659, Retrieved from the Internet: URL:https://clinicaltrials.gov/archive/NCT 02235493/2015_11_19 [retrieved on 2017-01-27]
- Carla Epps ET AL: "CENTER FOR DRUG EVALUATION AND RESEARCH", , 1 January 2015 (2015-01-01), XP055339606, Retrieved from the Internet: URL:http://www.accessdata.fda.gov/drugsatf da_docs/nda/2015/125513Orig1s000MedR.pdf [retrieved on 2017-01-27]
- Anonymous: "Improvement in bone manifestations and respiratory status in infants and young children with HPP treated with asfotase alfa: an update on the ENB-010-10 trial", , 1 January 2015 (2015-01-01), XP055339614, Retrieved from the Internet: URL:http://www.bone-abstracts.org/ba/0004/ ba0004OC18.htm [retrieved on 2017-01-27]

## Description

### FIELD

The disclosure relates to methods for treating hypophosphatasia (HPP).

### BACKGROUND

Hypophosphatasia (HPP) is a rare, heritable skeletal disease with an incidence of 1 per 100,000 births for the most severe forms of the disease. The disorder results from loss-of-function mutations in the gene coding for tissue-nonspecific alkaline phosphatase (TNSALP). HPP exhibits a remarkable range of symptoms and severity in children adolescents, which may include rickets and proximal muscle weakness. Due to physical impairments associated with HPP, children and adolescent afflicted with HPP often exhibit decreased mobility relative to healthy peers. Mobility is a complex function requiring integration of strength, balance, and coordination, all of which are affected by HPP. Children and adolescents with HPP have functional deficits in mobility including ambulation difficulties, weakness (e.g., proximal muscle weakness), shortened stature, and an inability to perform activities of daily living.

There exists a need for methods that can be used to monitor and guide the treatment of HPP in patients with mobility impairments, particularly gait defects, such as children and adolescents.

### SUMMARY

The invention is defined in the claims. According to the present invention there is provided a composition comprising a soluble alkaline phosphatase (sALP) comprising an amino acid sequence having at least 95% sequence identity to the amino acid sequence of SEQ ID NO: 1 for use in treating hypophosphatasia (HPP) in a subject of 5 to 15 years of age having an average modified Performance-Oriented Mobility Assessment - Gait (mPOMA-G) score of 5 or less, wherein said subject did not exhibit an increase in the average mPOMA-G score of at least 0.6, particularly at least 1.0 or more, after administration of said sALP to the subject at a dose providing 6 mg/kg/week during a treatment period of at least one year, and wherein the dosage of the sALP is increased to 9 mg/kg/week.

Disclosed are (1) methods to identify (a) children having hypophosphatasia (HPP; e.g., children having HPP of about 5 to about 12 years of age) and (b) adolescents having hypophosphatasia (e.g., adolescents having HPP of about 12 to about 15 years of age) for treatment with a soluble alkaline phosphatase (sALP; e.g., SEQ ID NO: 1), and (2) treatment of such patients identified in (1) and (2) with an sALP. The modified Performance-Oriented Mobility Assessment Gait (mPOMA-G) is a useful metric to evaluate the need for or the efficacy of treatment using an sALP. The methods described herein include the use of the mPOMA-G to assess treatment efficacy using an sALP for a patient having HPP, in which improvements relative to a certain score or value demonstrate that the sALP is effective for treating HPP.

The methods described herein can further include the use of the mPOMA-G in combination with one or more other metrics (e.g., the Six Minute Walk Test (6MWT), Child Health Assessment Questionnaire (CHAQ), and/or Pediatric Outcomes Data Collection Instrument (PODCI)) to assess
treatment efficacy using an sALP for a patient having HPP, in which improvements relative to a certain score or value demonstrate that the sALP is effective for treating HPP. Additionally, the methods can further include changing the dosage of and/or the frequency of administration of the sALP (e.g., SEQ ID NO: 1) in order to determine the effective amount of the sALP to administer to a child or an adolescent having HPP.

There is described herein a method of treating HPP in a patient of about 5 to about 15 years of age (e.g., a child or an adolescent having HPP, such as a patient having juvenile-onset HPP) having an average mPOMA-G score of about 8 or less (e.g., about 1, 2, 3, 4, 5, 6, 7, or 8), which includes administering a soluble alkaline phosphatase (sALP) to the patient at a dosage providing about 6 mg/kg/week of the sALP. In particular, the sALP includes an amino acid sequence having at least 95% sequence identity to the amino acid sequence of SEQ ID NO: 1 (e.g., asfotase alfa). Administration of the sALP (SEQ ID NO: 1) for a treatment period of at least one year results in an increase in the average mPOMA-G score of at least about 0.6 (e.g., about 0.7, about 0.8, or about 0.9), particularly at least about 1.0 or more (e.g., about 1, about 1.5, about 2.5, about 3, about 3.5, about 4, about 4.5, about 5, about 5.5, about 6, about 6.5, or about 7). For instance, administration of the sALP for a treatment period of at least one year results in an increase in the average mPOMA-G score of at least about 2.5 or more. The average mPOMA-G score of the patient can be determined relative to an average mPOMA-G score of an untreated subject (e.g., an HPP subject of about 5 to about 15 years of age) or a healthy subject (e.g., a healthy subject of about 5 to about 15 years of age).

The method can further include performing an mPOMA-G analysis, e.g., daily, one or more times per week (e.g., 2, 3, 4, 5, 6, or 7 times per week), weekly, one or more times per month (e.g., 2, 3, 4, 5, 6, 7, 8, 9, 10, 15, 20, 25, or more times per month), monthly, every other month, every three months, every six months, one or more times per year (e.g., 2, 3, 4, 5, 6, 7, 8, 9, 10, 11, 12, or more times per year), yearly, every two years, every three years, or a combination thereof (e.g., weekly combined with every three months, every six months, one or more times per year, or yearly).

For instance, the mPOMA-G analysis includes one or more gait assessments, such as trunk sway, walking stance, step length and height, step symmetry, and step continuity. In particular, trunk sway can include measuring at least one of marked sway, use of walking aids, arm abduction, trunk flexion, and excessive knee flexion; walking stance can include measuring distance of heels; step length and height can include measuring right swing foot, right foot clear, left swing foot, and/or left foot clear; step symmetry can include measuring right step length and left step length and comparing right step length to left step length; and step continuity can include measuring heel off in terminal stance on one foot at the same time as initial contact of heel strike on the opposite foot. For example, the mPOMA-G analysis is performed before or after administration of the sALP (e.g. SEQ ID NO: 1).

For instance, the sALP (e.g., SEQ ID NO: 1) is administered for a treatment period of at least two years, at least three years, at least four years, at least five years, at least six years, at least seven years, at least eight years, at least nine years, at least ten years, or longer (e.g., for the lifetime of the patient having HPP, such as a patient having juvenile-onset HPP). In particular, the average mPOMA-G score of the patient increases to about 7.5, about 8, about 8.5, about 9, about 9.5, about 10, about 10.5, about 11, about 11.5, or about 12 after administration of the sALP, such as to about 9 after administration of the sALP.

In the above aspects, the patient (e.g., a patient of about 5 to about 15 years of age having HPP, such as a patient having juvenile-onset HPP) prior to administration of the sALP can exhibit one or more gait impairments, including, but not limited to, reduced step length, reduced step continuity, reduced foot clearance, foot clearance that exceeds about 1 inch off of the surface, and widened stance. For example, the patient after administration of the sALP exhibits an improvement in one or more gait impairments, such as reduced step length, reduced foot clearance, and widened stance.

The patient having HPP can be a child or an adolescent. Additionally, the patient can be one that has not been previously administered the sALP (e.g., SEQ ID NO: 1).

As a result of the methods, the increase in the average mPOMA-G score is sustained throughout a period during which the patient (e.g., a patient of about 5 to about 15 years of age having HPP, such as a patient having juvenile-onset HPP) is treated with the sALP (e.g., at least one year, at least two years, at least three years, at least four years, at least five years, at least six years, at least seven years, at least eight years, at least nine years, at least ten years, or for the lifetime of the patient). For example, a rate of change per year of the average mPOMA-G score is about 1.0 or more (e.g., about 1, about 1.5, about 2.5, about 3, about 3.5, about 4, about 4.5, about 5, about 5.5, about 6, about 6.5, or about 7).

The method can further include performing at least one of a 6MWT, CHAQ, and/or a PODCI. For example, the patient (e.g., a patient of about 5 to about 15 years of age having HPP, such as a patient having juvenile-onset HPP) can exhibit an increase in activities of daily living (ADL) after administration of the sALP, e.g., in which the increase in ADL is determined from a CHAQ disability index score or PODCI transfer and mobility scale score of the patient. Additionally, the patient can exhibit an improvement in walking ability, e.g., in which the improvement in walking ability is determined from a 6MWT distance of the patient. For instance, the patient also exhibits decreased reliance on an assistive device (e.g., a wheelchair, braces, crutches, or an orthotic) for mobility after administration of the sALP.

Additionally, the method can further include determining sALP activity in at least one of serum and blood from the patient (e.g., a patient of about 5 to about 15 years of age having HPP, such as a patient having juvenile-onset HPP). For example, the sALP activity includes measuring at least one of phosphoethanoiamine (PEA), inorganic pyrophosphate (PPi), and/or pyridoxal 5'-phosphate (PLP) in the serum and/or blood sample from the patient.

In the above aspects, the sALP (e.g., SEQ ID NO: 1) is administered to the patient (e.g., a patient of about 5 to about 15 years of age having HPP, such as a patient having juvenile-onset HPP) daily or weekly, such as twice a week, three times a week, four times a week, five times a week, six times a week, or seven times a week. For example, the sALP (e.g., SEQ ID NO: 1) is administered to the patient at a dosage of 2 mg/kg for administration three times a week, a dosage of 3 mg/kg for administration three times a week, or a dosage of 1 mg/kg for administration six times a week. Moreover, the sALP (e.g., SEQ ID NO: 1) is administered to the patient once daily and/or on consecutive or alternating days. Additionally, the dose of the sALP is increased (e.g., from 6 mg/kg/day to 9 mg/kg/day) if the average mPOMA-G score does not increase by, e.g., at least about 1.0 or more (e.g., about 1, about 1.5, about 2.5, about 3, about 3.5, about 4, about 4.5, about 5, about 5.5, about 6, about 6.5, or about 7) after a treatment period of at least one year (e.g., at least two years, at least three years, at least four years, at least five years, at least six years, at least seven years, at least eight years, at least nine years, at least ten years, or for the lifetime of the patient). In particular, the dose of the sALP can be increased if the average mPOMA-G score does not increase by, e.g., at least about 2.5 or more (e.g., about 3, about 3.5, about 4, about 4.5, about 5, about 5.5, about 6, about 6.5, or about 7) after a treatment period of at least one year.

According to the present invention, the composition comprising the sALP is used to treat HPP in a subject of 5 to 15 years of age having an average modified Performance-Oriented Mobility Assessment - Gait (mPOMA-G) score of 5 or less, wherein said subject did not exhibit an increase in the average mPOMA-G score of at least 0.6, particularly at least 1.0 or more, after administration of said sALP to the subject at a dose providing 6 mg/kg/week during a treatment period of at least one year, and wherein the dosage of the sALP is increased to 9 mg/kg/week.

The patient (e.g., a patient of about 5 to about 15 years of age having HPP, such as a patient having juvenile-onset HPP) can exhibit one or more symptoms of HPP, which can include, but are not limited to, gait disturbance, bone deformity, joint pain, bone pain, bone fracture, muscle weakness, muscle pain, rickets, premature loss of deciduous teeth, incomplete bone mineralization, elevated blood and/or urine levels of phosphoethanolamine (PEA), elevated blood and/or urine levels of inorganic pyrophosphate (PPi), elevated blood and/or urine levels of pyridoxal 5'-phosphate (PLP), hypomineralization, rachitic ribs, hypercalciuria, short stature, HPP-related seizure, inadequate weight gain, craniosynostosis, and/or calcium pyrophosphate dihydrate crystal deposition. In particular, the patient exhibits an improvement in the one or more symptoms of HPP after administration of the sALP (e.g., SEQ ID NO: 1).

In the above aspects, the sALP (e.g., SEQ ID NO: 1) is formulated in a pharmaceutical composition, with at least one pharmaceutically acceptable carrier, such as saline (e.g., sodium chloride and sodium phosphate). For example, the at least one pharmaceutically acceptable carrier includes 150 mM sodium chloride and 25 mM sodium phosphate. Moreover, the pharmaceutical composition is formulated for subcutaneous, intramuscular, intravenous, oral, nasal, sublingual, intrathecal, or intradermal administration. In particular, the pharmaceutical composition is formulated for subcutaneous administration.

In the above aspects, the sALP (e.g., SEQ ID NO: 1) is physiologically active toward PEA, PPi, and PLP, catalytically competent to improve skeletal mineralization in bone, and/or is the soluble extracellular domain of an alkaline phosphatase. In particular, the sALP includes or consists of the amino acid sequence of SEQ ID NO: 1.

### Definitions

As used herein, "a" or "an" means "at least one" or "one or more" unless otherwise indicated. In addition, the singular forms "a," "an," and "the" include plural referents unless the context clearly dictates otherwise.

As used herein, "about" refers to an amount that is ± 10% of the recited value and is preferably ± 5 % of the recited value, or more preferably ± 2 % of the recited value. For instance, the term "about" can be used to modify all dosages or ranges recited herein by ±10% of the recited values or range endpoints.

By "asfotase alfa" is meant a human TNSALP (hTNSALP) fusion protein formulated for the treatment of HPP. Asfotase alfa (STRENSIQ®, Alexion Pharmaceuticals, Inc.) is a fusion protein including a soluble glycoprotein of two identical polypeptide chains, in which each polypeptide chain includes amino acid residues 1-726 of SEQ ID NO: 1. The structure of each polypeptide chain includes the catalytic domain of hTNSALP, the human immunoglobulin G₁ Fc domain, and a deca-aspartate peptide used as a bone targeting domain (the structure hTNSALP-Fc-D₁₀). The two polypeptide chains are covalently linked by two disulfide bonds. Asfotase alfa has been approved under the trade name STRENSIQ® in the United States, Europe, Japan, Canada, Israel, Australia, and Korea.

The terms "activities of daily living" or "ADL," as used herein, refer to routine activities that healthy subjects perform on a daily basis without requiring assistance, such as functional mobility or transferring (e.g., walking), bathing and showering, dressing, self-feeding, and personal hygiene and grooming. As described herein, therapeutic compositions (e.g., compositions including a soluble alkaline phosphatase (sALP), such as asfotase alfa) can be administered to an HPP patient to improve the ability of the patient (e.g., an adolescent or a child) to perform ADL.

As used herein, "average" refers to a numerical value expressing the mean or median of a data set. The mean of a data set is calculated by dividing the sum of the values in the set by their number. The median of a data set is calculated by, e.g., arranging all values in the data set from the lowest value to the highest value and selecting the middle value. In a data set with an even number of values, the median may be calculated, e.g., as the mean of the two middle values.

The term "bone-targeting moiety," as used herein, refers to an amino acid sequence of between 1 and 50 amino acid residues (such as 1, 2, 3, 4, 5, 6, 7, 8, 9, 10, 12, 14, 15, 16, 18, 20, 22, 24, 25, 26, 28, 30, 32, 34, 35, 36, 38, 40, 42, 44, 45, 46, 48, or 50 amino acid residues) in length having an affinity to bone matrix, such that the bone-targeting moiety, singularly, has an *in vivo* binding affinity to bone matrix that is about 10⁻⁶ M to about 10⁻¹⁵ M (e.g., 10⁻⁷ M, 10⁻⁸ M, 10⁻⁹ M, 10⁻¹⁰ M, 10⁻¹¹ M, 10⁻¹² M, 10⁻¹³ M, 10⁻¹⁴ M, or 10⁻¹⁵ M). For example, the bone-targeting moiety can include a series of consecutive aspartate (D) and/or glutamate (E) residues of number "n," in which n = 1 to 50, e.g., n = 3-30, eg., 5-15, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 36, 38, 39, 40, 41, 42, 43, 44, 45, 46, 47, 48, 49, or 50.

The term "catalytically competent," as used herein, refers to an sALP that hydrolyzes the bone mineralization inhibitor inorganic pyrophosphate (PPi) to provide inorganic phosphate (Pi), thereby decreasing the extracellular concentrations of PPi. A catalytically competent sALP improves skeletal mineralization in bone by regulating the concentration of PPi.

The term "dosage" refers to a determined quantity of an active agent (e.g., an sALP, such as asfotase alfa) calculated to produce a desired therapeutic effect (e.g., treatment of HPP, e.g., a reduction in one or many symptoms of HPP, such as a mobility impairment) that is administered to a patient at a particular frequency and/or in a defined amount. A dosage form can include an sALP, such as asfotase alfa, in association with any suitable pharmaceutical excipient, carrier, or diluent.

By "extracellular domain" is meant any functional extracellular portion of a native protein, e.g., alkaline phosphatase. In particular, an extracellular domain lacks a signal peptide.

By "Fc" is meant a fragment crystallizable region of an immunoglobulin, e.g., IgG-1, IgG-2, IgG-3, IgG-3 or IgG-4, including the CH2 and CH3 domains of the immunoglobulin heavy chain. Fc may also include any portion of the hinge region joining the Fab and Fc regions. The Fc can be of any mammal, including human, and may be post-translationaliy modified (e.g., by glycosylation). In a non-limiting example, Fc can be the fragment crystallizable region of human IgG-1 having the amino acid sequence of SEQ ID NO: 20.

By "fragment" is meant a portion of a polypeptide or nucleic acid molecule that contains, preferably, at least 10%, 20%, 30%, 40%, 50%, 60%, 70%, 75%, 80%, 85%, 90%, 95%, 96%, 97%, 98%, 99%, or more, but less than the entire length of, a reference nucleic acid molecule or polypeptide. For example, a polypeptide fragment may contain 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 400, 500, 600, 700, or more amino acid residues of the reference polypeptide. Exemplary sALP fragments have amino acid residues 18-498, 18-499, 18-500, 18-501, 18-502, 18-503, 18-504, 18-505, 18-506, 18-507, 18-508, 18-509, 18-510, 18-511, or 18-512 of an ALP (e.g., SEQ ID NOs: 2-6), and may include additional C-terminal and/or N-terminal potions. Biological activity of such fragments can be tested in standard assays known in the art, e.g., by a non-compartrnental analysis (NCA) to calculate pharmacokinetic parameters of the sALP fragment.

The term "gait," as used herein, refers to the locomotion of a subject (e.g., a child or an adolescent having HPP) achieved through moving on foot, such as by walking, e.g., in which at least one foot is in contact with the ground at all times. In particular, human gait refers to bipedal and biphasic forward propulsion of the center of gravity of the human body over a surface, such as a floor. Normal gait for a human subject is when one limb typically provides support, while the other limb is advanced in preparation to support the other limb The gait cycle (e.g., a stride) is composed of a swing phase and a stance phase, in which the stance phase includes initial double stance, single limb stance, and terminal double. The duration of a gait cycle is the interval between sequential initial surface contacts by the same limb

The term "gait impairment," as used herein, refers to abnormalities in normal gait of a subject (e.g., a human child or adolescent, such as a human child or adolescent with HPP) in which the subject exhibits deficiencies in the ability to stand and perform stepping movements for locomotion. Gait impairments can include, but are not limited to, reduced step length, reduced foot clearance, and a widened stance. For example, gait impairments can result in, e.g., waddling gait, in which the patient exhibits exaggerated alternation of lateral trunk movements with an exaggerated elevation of the hip, or steppage gait, in which the advancing leg is lifted high in order that the toes may clear the ground. Assessments of gait and gait impairments in a patient (e.g., a child or an adolescent having HPP) can include, e.g., determining the step length and height, step symmetry, step continuity, trunk sway, and walking stance of the patient.

The terms "hypophosphatasia" and "HPP," as used herein, refer to a rare, heritable skeletal disorder caused by, e.g., one or more loss-of-function mutations in the ALPL (alkaline phosphatase, liver/bone/kidney) gene, which encodes tissue-nonspecific alkaline phosphatase (TNSALP). HPP may be further characterized as infantile HPP, childhood HPP, perinatal HPP (e.g., benign perinatal HPP or lethal perinatal HPP), odonto-HPP, adolescent HPP, or adult HPP. For instance, "childhood HPP" describes a patient having HPP that is about 5 years of age to about 12 years of age, whereas "adolescent HPP" describes a patient having HPP that is about 12 years of age to about 18 years of age, such as a patient having HPP that is about 12 years of age to about 15 years of age. The age of onset of HPP, such as when the subject exhibits symptoms of HPP, can also be categorized as, e.g., perinatal-onset HPP, infantile-onset HPP, and juvenile-onset HPP (e.g., during childhood).

The term "HPP phenotype," as used herein, refers to any one of gait disturbance, bone deformity, joint pain, bone pain, bone fracture, muscle weakness, muscle pain, rickets (e.g., defects in growth plate cartilage), premature loss of deciduous teeth, incomplete bone mineralization, elevated blood and/or urine levels of phosphoethanolamine (PEA), PPi, pyridoxal 5'-phosphate (PLP), hypomineralization, rachitic ribs, hypercalciuria, short stature, HPP-related seizure, inadequate weight gain, craniosynostosis, and/or calcium pyrophosphate dihydrate crystal deposition (CPPD) in joints leading to, e.g., chondrocalcinosis and premature death. Without being so limited, an HPP phenotype can be documented by one or more of gait deficits, as described herein; growth retardation with a decrease of long bone length (including but not limited to femur, tibia, humerus, radius, and/or ulna); a decrease of the mean density of total bone and a decrease of bone mineralization in bones such as femur, tibia, ribs and metatarsi, and phalange; a decrease in teeth mineralization; and a premature loss of deciduous teeth (e.g., aplasia, hypoplasia, or dysplasia of dental cementum). Without being so limited, correction or prevention of a bone mineralization defect may be observed by one or more of the following: a reduction in gait deficits, an increase of long bone length, an increase of mineralization in bone and/or teeth, a correction of bowing of the legs, a reduction of bone pain, and a reduction of CPPD crystal deposition in joints.

The terms "modified Performance Oriented Mobility Assessment-Gait" and "mPOMA-G," as used herein, refer to a modified version of the Performance Oriented Mobility Assessment - Gait (POMA-G), such as a POMA-G that is modified to provide improved sensitivity for gait impairments in POMA-G components that pertain to HPP patients (e.g., a child or an adolescent with HPP). The POMA is a validated tool that consists of 2 subtests: the POMA-G (Gait) and the POMA-B (Balance). See Tinetti et al. (Am J Med 80:429-434, 1986) for a description of the POMA-G. Gait performance of an HPP patient (e.g., a child or an adolescent with HPP) can be assessed using an mPOMA-G test, e.g., in which the POMA-G analysis has been modified to remove the less appropriate components, i.e., the initiation of gait and path components, of the POMA-G. For example, the mPOMA-G analysis can provide a total score of 12 points, similarly to the POMA-G components, of step length and height, step symmetry, step continuity, trunk sway, and walking stance, in which a score of 12 indicates no gait impairments, and lower scores indicate gait impairments.

By "pharmaceutically acceptable excipient, carrier, or diluent" is meant at least one excipient, carrier, or diluent, respectively, which is physiologically acceptable to the treated patient and which does not alter the therapeutic properties of an active agent (e.g., an sALP, such as asfotase alfa (SEQ ID NO: 1)) with which it is administered. One exemplary pharmaceutically acceptable carrier substance is physiological saline. For instance, the pharmaceutically acceptable carrier can include sodium chloride (e.g., 150 mM sodium chloride) and sodium phosphate (e.g., 25 mM sodium phosphate). Other physiologically acceptable excipients, carriers, and diluents, and their formulations, are known to those skilled in the art and described, e.g., in Remington's Pharmaceutical Sciences (20th edition), A. Gennaro, Ed., 2000, Lippincott, Williams & Wilkins, Philadelphia, PA. For instance, a pharmaceutically acceptable excipient, carrier, or diluent can include dibasic sodium phosphate, heptahydrate; monobasic sodium phosphate, monohydrate; and sodium chloride at a pH between 7.2 and 7.6.

By "pharmaceutical composition" is meant a composition containing an active agent, such as an sALP (e.g., an sALP comprising an amino acid sequence having at least 95% sequence identity to the amino acid sequence of SEQ ID NO: 1, such as asfotase alfa), as described herein, formulated with at least one pharmaceutically acceptable excipient, carrier, or diluent. The pharmaceutical composition may be manufactured or sold with the approval of a governmental regulatory agency as part of a therapeutic regimen for the treatment or prevention of a disease or event (e.g., HPP) in a patient (e.g., an HPP patient, such as a child or adolescent). Pharmaceutical compositions can be formulated, for example, for subcutaneous administration, intravenous administration (e.g., as a sterile solution free of particulate emboli and in a solvent system suitable for intravenous use), for oral administration (e.g., a tablet, capsule, caplet, gelcap, or syrup), or any other formulation described herein, e.g., in unit dosage form. For example, an sALP (e.g., asfotase alfa; SEQ ID NO: 1) can be formulated as a pharmaceutical composition including dibasic sodium phosphate, heptahydrate; monobasic sodium phosphate, monohydrate; and sodium chloride at a pH between 7.2 and 7.6 for administration to a patient in, e.g., a weekly dosage ranging, e.g., from about 0.5 mg/kg/week to about 140 mg/kg/week, e.g., about 0.8 mg/kg/week to about 50 mg/kg/week, or about 1 mg/kg/week to about 10 mg/kg/week (e.g., about 6 or about 9 mg/kg/week).

The term "physiologically active," as used herein, refers to an sALP that hydrolyzes phosphoethanolamine (PEA), inorganic pyrophosphate (PPi), and pyridoxal 5'-phosphate (PLP) to provide Pi, thereby decreasing extracellular concentrations of PEA, PPi, and PLP.

The terms "sALP," "soluble alkaline phosphatase," and "extracellular domain of an alkaline phosphatase" are used interchangeably and refer to a soluble, non-membrane bound ALP or a domain or a biologically active fragment of the soluble, non-membrane bound ALP. sALPs include, for example, an alkaline phosphatase lacking a C-terminal glycolipid anchor (GPI signal sequence, e.g., polypeptides including or consisting of the amino acid residues 18-502 of a human TNSALP (SEQ ID NOs: 2, 3, 4, 5, or 6)). In particular, a TNSALP may include, e.g., a polypeptide including or consisting of amino acid residues 1-485 of SEQ ID NO: 1, such as asfotase alfa, or a polypeptide variant having at least 95% sequence identity to the amino acid residues 1-485 of SEQ ID NO: 1. sALPs further include, for example, mammalian orthologs of human TNSALP, such as a rhesus TNSALP (SEQ ID NO: 7), a rat TNSALP (SEQ ID NO: 8), a canine TNSALP (SEQ ID NO: 9), a porcine TNSALP (SEQ ID NO: 10), a murine TNSALP (SEQ ID NO: 11), a bovine TNSALP (SEQ ID NOs: 12-14), or a feline TNSALP (SEQ ID NO: 15). sALPs also include soluble, non-membrane-bound forms of human PALP (e.g., polypeptides including or consisting of amino acid residues 18-502 of SEQ ID NOs: 16 or 17), GCALP (e.g., polypeptides including or consisting of amino acid residues 18-502 of SEQ ID NO: 18), and IALP (e.g., polypeptides including or consisting of amino acid residues 18-502 of SEQ ID NO: 19), and additional variants and analogs thereof that retain alkaline phosphatase activity, e.g., the ability to hydrolyze PPᵢ. such as variants having at least 90, 95, 97, or 99% sequence identity to any one of SEQ ID NOs: 7-19. An sALP, in particular, lacks the N-terminal signal peptide (e.g., aa 1-17 of SEQ ID NOs: 2-6, 8, 11-13, or 15 or aa 1-25 of SEQ ID NO: 7). The sALP used in the present invention comprises an amino acid sequence having at least 95% sequence identity to the amino acid sequence of SEQ ID NO: 1.

By "sALP fusion polypeptide" is meant a polypeptide having the structure Z-sALP-Y-spacer-X-Wₙ-V, Z-Wₙ-X-spacer-Y-sALP-V, Z-sALP-Y-Wₙ-X-spacer-V, and Z-Wₙ-X-sALP-Y-spacer-V. In particular, the sALP fusion polypeptide can be Z-sALP-Y-spacer-X-Wₙ-V or Z-Wₙ-X-spacer-Y-sALP-V, such as hTNSALP-Fc-D₁₀ (e.g., asfotase alfa; SEQ ID NO: 1). Any one of X, Y, Z, V, the spacer, and/or Wₙ can be absent or an amino acid sequence of at least one amino acid. For example, X, Y, Z, and V may be a dipeptide sequence (e.g., leucine-lysine or aspartic acid-isoleucine), such as a two residue linker at the Y position (e.g., leucine-lysine) and a two residue linker at the X position (e.g., aspartic acid-isoleucine). Spacers include, for example, a Fc region of an immunoglobulin, such as the amino acid sequence of SEQ ID NO: 20. Wₙ can be a bone-targeting moiety as defined herein, e.g., having a series of consecutive aspartate (D) or glutamate (E) residues, in which n = 1 to 50, e.g., n = 3-30, e.g., 5-15, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 36, 38, 39, 40, 41, 42 43, 44, 45, 46, 47, 48, 49, or 50.

By "signal peptide" is meant a short peptide (5-30 amino acids long) at the N-terminus of a polypeptide that directs a polypeptide towards the secretory pathway (e.g., the extracellular space). The signal peptide is typically cleaved during secretion of the polypeptide. The signal sequence may direct the polypeptide to an intracellular compartment or organelle, e.g., the Golgi apparatus. A signal sequence may be identified by homology, or biological activity, to a peptide with the known function of targeting a polypeptide to a particular region of the cell. One of ordinary skill in the art can identify a signal peptide by using readily available software (e.g., Sequence Analysis Software Package of the Genetics Computer Group, University of Wisconsin Biotechnology Center, 1710 University Avenue, Madison, Wis. 53705, BLAST, or PILEUP/PRETTYBOX programs). A signal peptide can be one that is, for example, substantially identical to amino acid residues 1-17 of SEQ ID NOs: 2-6 or amino acid residues 1-25 of SEQ ID NO: 7.

As used herein, when a polypeptide or nucleic acid sequence is referred to as having "at least X% sequence identity" to a reference sequence, wherein "X" is a real number, it is meant that at least X percent of the amino acid residues or nucleotides in the polypeptide or nucleic acid, respectively, are identical to those of the reference sequence when the sequences are optimally aligned An optimal alignment of sequences can be determined in various ways that are within the skill in the art, for instance, the Smith Waterman alignment algorithm (Smith et al., J. Mol. Biol. 147:195-7, 1981) and BLAST (Basic Local Alignment Search Tool; Altschul et al., J. Mol. Biol. 215: 403-10, 1990). These and other alignment algorithms are accessible using publicly available computer software such as "Best Fit" (Smith and Waterman, Advances in Applied Mathematics, 482-489, 1981) as incorporated into GeneMatcher Plus (Schwarz and Dayhoff, Atlas of Protein Sequence and Structure, Dayhoff, M.O., Ed pp 353-358, 1979), BLAST, BLAST-2, BLAST-P, BLAST-N, BLAST-X, WU-BLAST-2, ALIGN, ALIGN-2, CLUSTAL, Megalign (DNASTAR), or other software/hardware for alignment. In addition, those skilled in the art can determine appropriate parameters for measuring alignment, including any algorithms needed to achieve optimal alignment over the length of the sequences being compared

The terms "patient" or "subject" refer to a mammal, including, but not limited to, a human (e.g., a human having HPP, such as a child or adolescent) or a non-human mammal, such as a bovine, equine, canine, ovine, or feline.

"Parenteral administration," "administered parenterally," and other grammatically equivalent phrases, as used herein, refer to a mode of administration other than enteral and topical administration, usually by injection, and include, without limitation, subcutaneous, intradermal, intravenous, intranasal, intraocular, pulmonary, intramuscular, intra-arterial, intrathecal, intracapsular, intraorbital, intracardiac, intradermal, intrapulmonary, intraperitoneal, transtracheal, subcuticular, intraarticular, subcapsular, subarachnoid, intraspinal, epidural, intracerebral, intracranial, intracarotid, and intrasternal injection and infusion.

By "therapeutically effective amount" is meant an amount of an sALP (e.g., an sALP comprising an amino acid sequence having at least 95% sequence identity to the amino acid sequence of SEQ ID NO: 1, such as asfotase alfa) that is sufficient to substantially improve, treat, prevent, delay, suppress, or arrest at least one symptom of HPP (e.g., a mobility impairment, such as a gait impairment). A therapeutically effective amount of an sALP described herein may depend on the severity of HPP and the condition, weight, and general state of the patient and can be determined by an ordinarily-skilled artisan with consideration of such factors. A therapeutically effective amount of a composition described herein can be administered to a patient in a single dose or in multiple doses administered over a period of time.

By "treating," "treat," or "treatment" is meant the medical management of a patient (e.g., a child or an adolescent having HPP) with the intent to cure, ameliorate, stabilize, reduce the likelihood of, or prevent HPP and/or the management of a patient exhibiting or likely to have HPP, e.g., by administering a pharmaceutical composition, such as an sALP (e.g., an sALP comprising an amino acid sequence having at least 95% sequence identity to the amino acid sequence of SEQ ID NO: 1, such as asfotase alfa). Treatment can occur for a treatment period, in which an sALP is administered for a period of time (e.g., days, months, years, or longer) to treat a patient having HPP, such as a child or an adolescent with HPP that exhibits deficits in gait. This term includes active treatment directed toward the improvement of HPP; symptomatic treatment directed toward symptoms of HPP, such as deficits in gait; preventative treatment directed to minimizing the development of HPP, e.g., in a patient who does not yet have HPP, but who is susceptible to or at risk of developing HPP; and supportive treatment employed to supplement another specific therapy directed toward the improvement of HPP.

### BRIEF DESCRIPTION OF THE DRAWINGS

**Figure 1** is an image for gait assessment used in the key for the modified Performance-Oriented Mobility Assessment Gait (mPOMA-G) analysis of a child demonstrating step continuity.
**Figure 2** is an image used in the mPOMA-G analysis of a child demonstrating normal hip/knee flexion during swing for gait assessment.
**Figures 3A-3C** are graphs showing linear regression analysis of the mPOMA-G score relative to scores determined for the Childhood Health Assessment Questionnaire (CHAQ) Disability Index (Fig. 3A), Pediatric Outcomes Data Collection Instrument (PODCI) Transfer and Mobility Scale Normative Parent Score (Fig. 3B), and the Six Minute Walk Test (distance walked in meters) (6MWT; Fig. 3C) for children and adolescents with HPP treated with asfotase alfa.
**Figure 4** is a graph showing the rate of change per year in the mPOMA-G score from baseline to last assessment of asfotase alfa-treated HPP patients (n=8) compared with historical HPP controls (HCs; n=6). The *P*-value based on Wilcoxon rank sum test (exact method) comparing the median mPOMA-G for asfotase alfa-treated HPP patients and HCs is shown. The upper and lower bars represent the maximum and minimum values, respectively; the top and bottom of the box represent the 3rd and 1st quartiles, respectively; the line within the box represents the median; and the 'x' represents the mean.

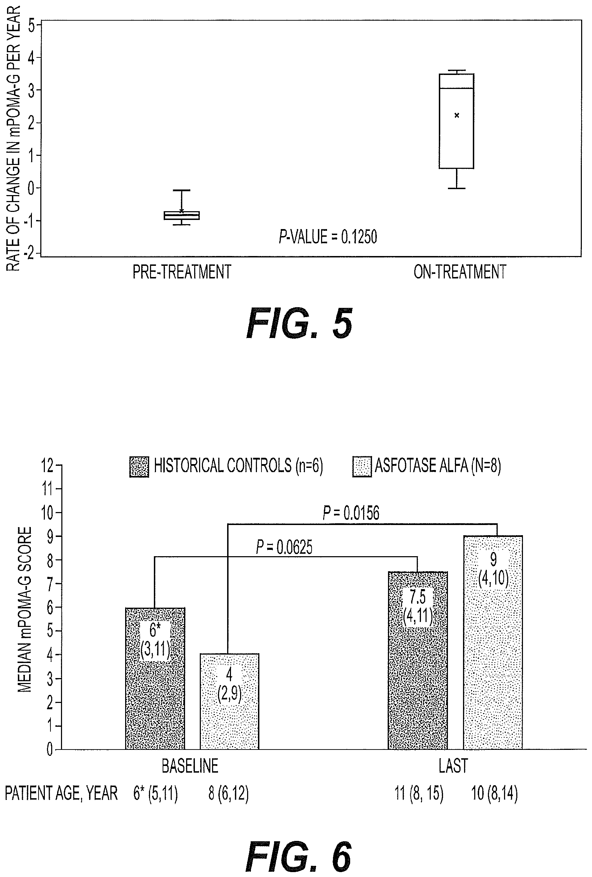

**Figure 5** is a graph showing the rate of change per year in the mPOMA-G score for HPP patients (n=5) prior to treatment with asfotase alfa (pre-treatment) compared to during treatment with asfotase alfa (on-treatment). The *P*-value based on Wilcoxon rank sum test (exact method) comparing the median mPOMA-G score for pre-treatment and on-treatment is shown. The upper and lower bars represent the maximum and minimum values, respectively; the top and bottom of the box represent the 3rd and 1st quartiles, respectively; the line within the box represents the median; and the 'x' represents the mean.
Figure 6 is a graph showing the mPOMA-G score at baseline and at last assessment for HCs and HPP patients treated with asfotase alfa. Median m-POMA score and patient age with minimum and maximum values are shown.
Figures **7A-7B** are graphs showing individual changes in mPOMA-G score by age in historical HPP control patients (Fig. 7A) and in HPP patients prior to and following treatment with asfotase alfa (Fig. 7B).
Figures 8A-8B are graphs showing individual changes in mPOMA-G score by age from the earliest historical gait data to pre-treatment baseline for patients treated with asfotase alfa (Fig. 8A) and from baseline to last on-treatment assessment (Fig. 8B).
**Figure 9** shows the amino acid sequence of asfotase alfa monomer (STRENSIQ®, Alexion Pharmaceuticals, Inc., SEQ ID NO: 1).

### DETAILED DESCRIPTION

Musculoskeletal defects in HPP can lead to compromised physical function, including impaired mobility. We have discovered that asfotase alfa (SEQ ID NO: 1, STRENSIQ®, Alexion Pharmaceuticals, Inc.) can be used effectively to treat hypophosphatasia (HPP), its symptoms, and decreased physical function associated therewith in patients of about 5 to about 15 years of age for an extended period of time (e.g., at least one year, at least two years, at least three years, at least four years, at least five years, at least six years, at least seven years, at least eight years, at least nine years, at least ten years, or longer than ten years, such as for the lifetime of the patient). As described herein, the Performance-Oriented Mobility Assessment Gait (POMA-G), a clinical gait assessment tool for adults, was modified to accurately assess gait impairment and change in gait impairment in children and adolescents with HPP and was utilized during administration of asfotase alfa treatment.

For example, asfotase alfa can be administered to treat HPP patients of about 5 to about 15 years of age exhibiting gait impairments (e.g., reduced step length, reduced foot clearance, and a widened stance) relative to an untreated HPP subject (e.g., an untreated HPP subject of about the same age, same gender, and/or height). Thus, asfotase alfa is effective for the treatment of HPP and gait impairments associated with HPP in both children (e.g., children having HPP of about 5 to about 12 years of age) and adolescents (e.g., adolescents having HPP of about 12 to about 15 years of age).

Methods for administering asfotase alfa (SEQ ID NO: 1) to an HPP patient of about 5 to about 15 years of age (e.g., a child or an adolescent having HPP) that result in an improvement in gait impairments are described. For example, asfotase alfa can be administered to an HPP patient of about 5 to about 15 years of age having an average modified POMA-G (mPOMA-G) score of about 5 or less (e.g., an mPOMA-G score of 0, 1, 2, 3, or 4). For example, asfotase alfa can be administered to an HPP patient of about 5 to about 15 years of age having an average modified POMA-G (mPOMA-G) score of about 9 or less (e.g., an mPOMA-G score of 6, 7, or 8). Following administration of asfotase alfa, the HPP patient of about 5 to about 15 years of age can exhibit an improvement in one or more mPOMA-G gait assessments, such as gait trunk sway, walking stance, step length and height, step symmetry, and/or step continuity. Accordingly, administration of asfotase alfa can result in an increase in the average mPOMA-G score of the HPP patient of about 5 to about 15 years of age. The average increase is an increase of about 1, 2, 3, 4 or more points in the mPOMA-G, relative to an untreated HPP patient of about the same age, gender, and/or height (e.g., a historical control patient).

Given the results described herein using asfotase alfa, other sALPs (such as a polypeptide variant having at least 95% sequence identity to the sequence of SEQ ID NO: 1) can be used to treat HPP patients of about 5 to about 15 years of age (e.g., a child or an adolescent having HPP) for an extended period of time (e.g., at least one year, at least two years, at least three years, at least four years, at least five years, at least six years, at least seven years, at least eight years, at least nine years, at least ten years, or longer than ten years, such as for the lifetime of the patient). In particular, other sALPs can be used to treat gait impairments in these HPP patients for an extended treatment period.

### Methods of Treatment

Described herein are methods for treating an HPP patient of about 5 to about 15 years of age, such as a child having HPP (e.g., a child having HPP of about 5 to about 12 years of age) or an adolescent having HPP (e.g., an adolescent having HPP of about 12 to about 15 years of age). HPP patients can be treated by administering an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) across a range of ages, e.g., about 5 to about 7, about 5 to about 8, about 5 to about 9, about 5 to about 10, about 5 to about 11, about 5 to about 12, about 5 to about 13, about 5 to about 14, about 6 to about 8, about 6 to about 9, about 6 to about 10, about 6 to about 11, about 6 to about 12, about 6 to about 13, about 6 to about 14, about 6 to about 15, about 7 to about 9, about 7 to about 10, about 7 to about 11, about 7 to about 12, about 7 to about 13, about 7 to about 14, about 7 to about 15, about 8 to about 10, about 8 to about 11, about 8 to about 12, about 8 to about 13, about 8 to about 14, about 8 to about 15, about 9 to about 11, about 9 to about 12, about 9 to about 13, about 9 to about 14, about 9 to about 15, about 10 to about 12, about 10 to about 13, about 10 to about 14, about 10 to about 15, about 11 to about 13, about 12 to about 14, about 10 to about 15, about 11 to about 13, about 11 to about 14, about 11 to about 15, about 12 to about 14, about 12 to about 15, or about 13 to about 15 years of age.

Patients of about 5 to about 15 years of age (e.g., children and adolescents) can be diagnosed with HPP prior to administration of an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa). An HPP patient of about 5 to about 15 years of age can exhibit, e.g., gait impairments relative to a healthy subject of about the same age and/or gender. Additionally, the HPP patient of about 5 to about 15 years of age may be one that has not previously been treated with an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa).

The method involves administering an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) to an HPP patient of about 5 to about 15 years of age, such as administering an sALP for a period of least one year (e.g., at least two years, at least three years, at least four years, at least five years, at least six years, at least seven years, at least eight years, at least nine years, at least ten years, or longer than ten years, such as for the lifetime of the patient). In particular, an sALP, such as asfotase alfa, can be administered for a period of time to an HPP patient of about 5 to about 15 years of age previously determined to have an average mPOMA-G score of about 9 or less (e.g., about 1, 2, 3, 4, 5, 6, 7, 8 or 9).

After administration of the sALP, the mPOMA-G score of the HPP patient of about 5 to about 15 years of age can be compared to the mPOMA-G score of the patient prior to administration of the sALP or to an untreated subject having HPP. The mPOMA-G of the HPP patient of about 5 to about 15 years of age can also be compared to the mPOMA-G score of a healthy subject (e.g., a subject without HPP) Alternatively, the methods can include determining the mPOMA-G score prior to administering an sALP, such as asfotase alfa.

Additionally, the method can further include performing an additional assessment, such as one or more of a Six Minute Walk Test (6MWT) to assess the walking ability of the HPP patient of about 5 to about 15 years of age, a Child Health Assessment Questionnaire (CHAQ), and/or a Pediatric Outcomes Data Collection Instrument (PODCI) to assess the ability of the HPP patient of about 5 to about 15 years of age to perform activities of daily living (ADL) either before or after administration of an sALP. The mPOMA-G can then be used in combination with one or more of the 6MWT, CHAQ, and/or PODCI to assess treatment efficacy using an sALP (such as TNSALP, for example the sALP fusion polypeptide of SEQ ID NO: 1 or a polypeptide variant having at least 95% sequence identity to the sequence of SEQ ID NO: 1, e.g., asfotase alfa), in which improvements relative to a certain value or score demonstrate that the sALP is effective for treating HPP.

For example, when administration of an sALP to an HPP patient of about 5 to about 15 years of age (e.g., a child or adolescent having HPP) results in a yearly increase in the average mPOMA-G score of about 2.5 or more (e.g., about 3, about 3.5, about 4, about 4.5, about 5, about 5.5, about 6, about 6.5, or about 7), in which the patient previously had an average mPOMA-G score of about 9 or less (e.g., about 0, 1, 2, 3, 4, 5, 6, 7, 8, or 9)(baseline), then the sALP treatment is determined to be effective at treating gait impairments associated with HPP. Alternatively, when administration of an sALP does not result in an increase in the average mPOMA-G score of about 2.5 or more, the dosage and/or frequency of sALP administration can be changed (e.g., increased), e.g., until an effective amount of the sALP for the HPP patient of about 5 to about 15 years of age (e.g., a child or adolescent having HPP) is identified. For instance, if necessary, the dosage of the sALP such as TNSALP, for example the sALP fusion polypeptide of SEQ ID NO: 1 or a polypeptide variant having at least 95% sequence identity to the sequence of SEQ ID NO: 1, e.g., asfotase alfa) can be increased from about 6 mg/kg/week to, e.g., about 9 mg/kg/week.

For example, when administration of an sALP to an HPP patient of about 5 to about 15 years of age (e.g., a child or adolescent having HPP) results in an increase in the average mPOMA-G score of about 0.6 or about 1.0 or more (e.g., about 0.7, about 0.8, about 0.9, about 1.0, or about 1.1), in which the patient previously had an average mPOMA-G score of about 9 or less (e.g., about 0, 1, 2, 3, 4, 5, 6, 7, 8, or 9)(baseline), then the sALP treatment is determined to be effective at treating gait impairments associated with HPP. Alternatively, when administration of an sALP does not result in an increase in the average mPOMA-G score of about 0.6 or about 1.0 or more, the dosage and/or frequency of sALP administration can be changed (e.g., increased), e.g., until an effective amount of the sALP for the HPP patient of about 5 to about 15 years of age (e.g., a child or adolescent having HPP) is identified.

For instance, if necessary, the dosage of the sALP such as TNSALP, for example the sALP fusion polypeptide of SEQ ID NO: 1 or a polypeptide variant having at least 95% sequence identity to the sequence of SEQ ID NO: 1, e.g., asfotase alfa) can be increased from about 6 mg/kg/week to, e.g., about 9 mg/kg/week.

According to the present invention, the composition comprising the sALP is used to treat HPP in a subject of 5 to 15 years of age having an average modified Performance-Oriented Mobility Assessment - Gait (mPOMA-G) score of 5 or less, wherein said subject did not exhibit an increase in the average mPOMA-G score of at least 0.6, particularly at least 1.0 or more, after administration of said sALP to the subject at a dose providing 6 mg/kg/week during a treatment period of at least one year, and wherein the dosage of the sALP is increased to 9 mg/kg/week.

### Hypophosphatasia in children and adolescents

Asfotase alfa is administered, as described herein, to treat childhood HPP or adolescent HPP. In particular, patients with childhood HPP (e.g., children having HPP of about 5 to about 12 years of age) or adolescent HPP (e.g., adolescents with HPP of about 12 to about 18 years of age, such as adolescents with HPP of about 12 to about 15 years of age) can be treated with an sALP (such as TNSALP, for example the sALP fusion polypeptide of SEQ ID NO: 1 or a polypeptide variant having at least 95% sequence identity to the sequence of SEQ ID NO: 1, e.g., asfotase alfa) for a period of at least one year (e.g., at least two years, at least three years, at least four years, at least five years, at least six years, at least seven years, at least eight years, at least nine years, at least ten years, or longer than ten years (e.g., the lifetime of the patient). We observed that an HPP phenotype of, e.g., childhood HPP or adolescent HPP, such as a mobility impairment (e.g., a gait impairment), as assessed using, e.g., an mPOMA-G, improves following treatment with an sALP (such as TNSALP, for example the sALP fusion polypeptide of SEQ ID NO: 1 or a polypeptide variant having at least 95% sequence identity to the sequence of SEQ ID NO: 1, e.g., asfotase alfa).

Accordingly, the methods described herein are useful for alleviating symptoms of HPP described herein, particularly when the sALP (such as TNSALP, for example the sALP fusion polypeptide of SEQ ID NO: 1 or a polypeptide variant having at least 95% sequence identity to the sequence of SEQ ID NO: 1, e.g., asfotase alfa) is administered for a period of at least one year (e.g., at least two years, at least three years, at least four years, at least five years, at least six years, at least seven years, at least eight years, at least nine years, at least ten years, or longer than ten years, such as for the lifetime of the patient). For instance, the methods are useful for treating symptoms of childhood or adolescent HPP, including, but not limited to, gait disturbance; skeletal deformities, such as bowed legs and enlarged wrists, knees, and ankles as a result of flared metaphyses; joint pain; bone pain; bone fracture; muscle weakness; muscle pain; rickets; premature loss of deciduous teeth; incomplete bone mineralization; elevated blood and/or urine levels of phosphoethanolamine (PEA), inorganic pyrophosphate (PPi), or pyridoxal 5'-phosphate (PLP); hypomineralization; rachitic ribs; hypercalciuria; short stature; HPP-related seizure; inadequate weight gain; craniosynostosis; and/or calcium pyrophosphate dihydrate crystal deposition.

The above mentioned symptoms can be treated with an sALP for a period of at least one year (e.g., at least two years, at least three years, at least four years, at least five years, at least six years, at least seven years, at least eight years, at least nine years, at least ten years, or longer than ten years, such as for the lifetime of the patient). The mPOMA-G is a useful metric for evaluating the need for or the efficacy of treatment using an sALP (such as TNSALP, for example the sALP fusion polypeptide of SEQ ID NO: 1 or a polypeptide variant having at least 95% sequence identity to the sequence of SEQ ID NO: 1, e.g., asfotase alfa) in childhood and adolescent HPP.

### Modified Performance-Oriented Mobility Assessment - Gait (mPOMA-G)

Children with HPP (e.g., children having HPP of about 5 to about 12 years of age) or adolescents with HPP (e.g., adolescents with HPP of about 12 to about 18 years of age, such as adolescents with HPP of about 12 to about 15 years of age) can be identified for treatment with an sALP (such as TNSALP, for example the sALP fusion polypeptide of SEQ ID NO: 1 or a polypeptide variant having at least 95% sequence identity to the sequence of SEQ ID NO: 1, e.g., asfotase alfa) using a modified version of the gait subtest of the Performance Oriented Mobility Assessment (mPOMA). As is described herein, the mPOMA-G can be used to evaluate gait impairments in HPP patients of about 5 to about 15 years of age (e.g., children or adolescents having HPP) to generate an mPOMA-G score for the patient.

The POMA-G originally included a range of tests to evaluate gait impairments of adults, e.g., elderly and community-dwelling adults, using a 12 point rating scale. The gait subtest of the original POMA-G was described in detail in Tinetti et al. (Am J Med 80:429-434, 1986). A rater, such as a physical therapist, may evaluate HPP patients of about 5 to about 15 years of age, using the POMA-G or mPOMA-G (see, e.g., Tables 2 and 3, respectively, herein below) during a clinical visit or can evaluate patients using video recordings of the patient. The POMA-G provides assessments in the following areas: initiation of gait; step length, height, symmetry, and continuity; foot clearance; path alignment; trunk stability; and walking stance. In each POMA-G area, 0 is the lowest score per item, and 1 or 2 is the highest score per item.

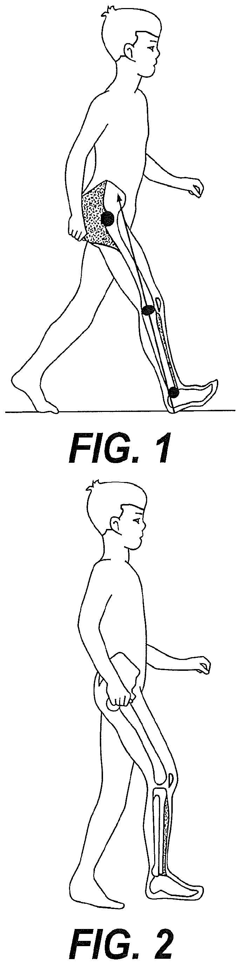

The POMA-G was modified to better assess children and adolescent HPP patients by removing the areas of gait initiation, which can be indicative of neurologic abnormalities instead of mobility impairments, and by removing path, which can be difficult to quantify in children and adolescents, e.g., children and adolescents having HPP. The mPOMA-G was further modified to assess HPP patients of about 5 to about 15 years of age (e.g., children and adolescent HPP patients) in individual areas, such as step length and step continuity, to increase the rating score to a 3 point instead of a 2 point or 1 point scale. In addition, the mPOMA-G can include clarifying descriptions of all items, including trunk sway and walk stance, to increase sensitivity and consistency among raters and/or a scoring key to provide additional details on individual item ratings (see, e.g., Table 3) and examples of patients performing the different areas (see, e.g., Fig. 1 and Fig. 2).

For example, a rater (e.g., a physical therapist) can score an HPP patient of about 5 to about 15 years of age (e.g., a child or adolescent HPP patient) in the mPOMA-G areas of step length and height, step symmetry, step continuity, foot clearance, trunk stability, and walking stance. Step length and height of the HPP patient can be scored by a rater as follows:
a) the right swing foot does not pass the left stance foot with step (0 points), the right heel passes the left stance foot (1 point), or the right foot passes the left stance foot by at least the length of the patient's foot between the stance toe and swing heel (2 points);
b) the right foot does not clear the floor completely with step or the patient raises their foot by more than 1 - 2 inches (0 points) or the right foot completely clears floor (1 point);
c) the left swing foot does not pass the right stance foot with step (0 points), the left heel passes the right stance foot (1 point), or the left foot passes the right stance foot by at least the length of the patient's foot between the stance toe and the swing heel (2 points); and
d) the left foot does not clear the floor completely with step or the patient raises their foot by more than 1 - 2 inches (0 points) or the left foot completely clears the floor (1 point).

Step symmetry of the HPP patient of about 5 to about 15 years of age (e.g., a child or adolescent HPP patient) can be scored by a rater based on the observations that the left foot does not clear the floor completely with step or the patient raises their foot by more than 1 - 2 inches (0 points) or the left foot completely clears the floor (1 point). Stopping or discontinuity between steps of the HPP patient of about 5 to about 15 years of age (e.g., a child or adolescent HPP patient) can be scored by a rater based on the observations that there is stopping or discontinuity between steps (0 points); the steps appear continuous unilaterally (the rater observes the patient raising the heel of one foot as the heel of other foot touches the floor, unilaterally) or there is flat foot contact on the stance limb when the heel of the other foot touches the floor bilaterally with no breaks or stops in stride (1 point); or the steps appear continuous bilaterally (e.g., the rater observes the patient raising the heel of one foot as the heel of other foot touches the floor, bilaterally), with no breaks or stops in stride, and step lengths are equal (2 points).

Trunk stability of the HPP patient of about 5 to about 15 years of age (e.g., a child or adolescent HPP patient) can be scored by a rater based on the observations that there is marked sway (e.g., moderate lateral flexion as the result of instability bilateral or unilateral) or use of walking aids (0 points); no marked sway, but compensatory patterns, such as trunk flexion, knee flexion, arm abduction, or retraction to increase postural stability while walking (1 point); or no sway, no flexion, no use of arms, and no walking aid (2 points).

Walking stance of the HPP patient of about 5 to about 15 years of age (e.g., a child or adolescent HPP patient) can be scored by a rater based on the observations that the heels are always apart and there is a wide base of support utilized to increase postural stability (0 points) or the heels are intermittently apart (1 point).

The individual scores in the mPOMA-G areas of step length and height, step symmetry, step continuity, foot clearance, trunk stability, and walking stance for an HPP patient of about 5 to about 15 years of age (e.g., a child or adolescent HPP patient) can be summed to generate a total mPOMA-G score for the patient ranging from 0 to 12, in which a lower score indicates greater gait impairments and a higher score indicates less or no gait impairments, e.g., gait of a healthy subject (e.g., step length and height points + step symmetry points + step continuity points + foot clearance points + trunk stability points + walking stance points = total mPOMA-G score). The total mPOMA-G score of the HPP patient of about 5 to about 15 years of age (e.g., a child or adolescent HPP patient) can then be compared to the mPOMA-G score of other HPP patients (e.g., a historical control patient) to, e.g., determine the average mPOMA-G score for the HPP patient.

The mPOMA-G score of an HPP patient of about 5 to about 15 years of age (e.g., a child or adolescent HPP patient) will range from about 0 to equal to or less than about 12, in which a higher score (e.g., about 8, 9, 10, 11, or 12) is considered representative of healthy subjects (e.g., subjects without HPP). An HPP patient of about 5 to about 15 years of age (e.g., a child or adolescent HPP patient) with an average mPOMA-G score of less than about 9 (e.g., about 0, 1, 2, 3, 4, 5, 6, 7, 8, or 9) can be treated with an sALP (such as TNSALP, for example the sALP fusion polypeptide of SEQ ID NO: 1 or a polypeptide variant having at least 95% sequence identity to the sequence of SEQ ID NO: 1, e.g., asfotase alfa), such as by administering an sALP for a period of at least one year (e.g., at least two years, at least three years, at least four years, at least five years, at least six years, at least seven years, at least eight years, at least nine years, at least ten years, or longer than ten years, such as for the lifetime of the patient). The mPOMA-G score of the treated patient can then be compared to the mPOMA-G score of an untreated HPP patient, such as an HPP patient of about 5 to about 15 years of age.

The methods can result in an improvement in the mPOMA-G score of an HPP patient of about 5 to about 15 years of age (e.g., a child or adolescent HPP patient). For example, treatment with an sALP (such as TNSALP, for example the sALP fusion polypeptide of SEQ ID NO: 1 or a polypeptide variant having at least 95% sequence identity to the sequence of SEQ ID NO: 1, e.g., asfotase alfa), such as treatment with an sALP for a period of at least one year (e.g., at least two years, at least three years, at least four years, at least five years, at least six years, at least seven years, at least eight years, at least nine years, at least ten years, or longer than ten years, such as for the lifetime of the patient), result in an average yearly increase in the mPOMA-G score of about 2.5 or more (e.g., about 3, about 3.5, about 4, about 4.5, about 5, about 5.5, about 6, about 6.5, or about 7). For example, the mPOMA-G score of the patient of about 5 to about 15 years of age can increase on average to about 7.5 or greater (e.g., to about 8, about 8.5, about 9, about 9.5, about 10, about 10.5, or about 12) after administration of the sALP Accordingly, administration of the sALP to the patient can result in a decrease in one or more gait impairments, such as reduced step length, reduced foot clearance, and/or widened stance.

The increase in the mPOMA-G can be sustained throughout administration of the sALP (such as TNSALP, for example the sALP fusion polypeptide of SEQ ID NO: 1 or a polypeptide variant having at least 95% sequence identity to the sequence of SEQ ID NO: 1, e.g., asfotase alfa) to the HPP patient of about 5 to about 15 years of age (e.g., a child or adolescent HPP patient), e.g., for a period of at least one year (e.g., at least two years, at least three years, at least four years, at least five years, at least six years, at least seven years, at least eight years, at least nine years, at least ten years, or longer than ten years, such as for the lifetime of the patient). For instance, the mPOMA-G score can increase yearly by about 2.5 points and can remain at ± 10% of the increased mPOMA-G score (e.g., about 7.5, about 8, about 8.5, about 9, about 9.5, about 10, about 105, or about 12) during treatment with the sALP.

Likewise, the improvement in gait impairment(s) can be sustained throughout administration of the sALP, e.g., for a period of at least one year (e.g., at least two years, at least three years, at least four years, at least five years, at least six years, at least seven years, at least eight years, at least nine years, at least ten years, or longer than ten years, such as for the lifetime of the patient). For instance, the HPP patient exhibits decreased reliance on an assistive device for walking, such as a wheelchair, a wheeled walker, a cane, or an orthotic during treatment with the sALP.

The mPOMA-G score can also be used to assess treatment efficacy of an sALP (such as TNSALP, for example the sALP fusion polypeptide of SEQ ID NO: 1 or a polypeptide variant having at least 95% sequence identity to the sequence of SEQ ID NO: 1, e.g., asfotase alfa), in which improvements relative to a certain mPOMA-G score demonstrate that the sALP is effective for treating gait impairments associated with HPP. For example, when administration of an sALP to an HPP patient of about 5 to about 15 years of age (e.g., a child or adolescent HPP patient) results in an average increase in the mPOMA-G score of about 2.5 or more (e.g., about 3, about 3.5, about 4, about 4.5, about 5, about 5.5, about 6, about 6.5, or about 7), in which the patient previously had an average mPOMA-G score of about 5 or less (e.g., about 0, 1, 2, 3, or 4), then the sALP is considered to be effective at, e.g., treating a gait impairment(s) associated with HPP.

Alternatively, when administration of an sALP for a treatment period of at least one year (e.g., at least two years, at least three years, at least four years, at least five years, at least six years, at least seven years, at least eight years, at least nine years, at least ten years, or longer than ten years, such as for the lifetime of the patient) does not result in an average increase in the mPOMA-G score of about 2.5 or more (e.g., an increase of about 3, about 3.5, about 4, about 4.5, about 5, about 5.5, about 6, about 6.5, or about 7), the dosage and/or frequency of sALP administration can be changed (e.g., increased), e.g., until an effective amount of the sALP for the HPP patient of about 5 to about 15 years of age (e.g., a child or adolescent HPP patient) is identified. For instance, if necessary, the dosage of the sALP (such as TNSALP, for example the sALP fusion polypeptide of SEQ ID NO: 1 or a polypeptide variant having at least 95% sequence identity to the sequence of SEQ ID NO: 1, e.g., asfotase alfa) can be increased from about 6 mg/kg/week to, e.g., about 9 mg/kg/week.

Additionally, the method can further include performing one or more of a 6MWT, CHAQ, and/or PODCI evaluation in combination with the mPOMA-G. The CHAQ or PODCI can be used in combination with the mPOMA-G to assess the ability of the HPP patient of about 5 to about 15 years of age to perform activity of daily living (ADL) after administration of an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa), as is described in U.S. Application No. 62/305,450. The 6MWT can be used to assess walking ability and physical function of the patient after administration of an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa), as is described in International Application No. PCT/US2016/025721. The mPOMA-G can then be used in combination with one or more of the 6MWT, CHAQ, and/or PODCI analyses to assess treatment efficacy using an sALP (such as TNSALP, for example the sALP fusion polypeptide of SEQ ID NO: 1 or a polypeptide variant having at least 95% sequence identity to the sequence of SEQ ID NO: 1, e.g., asfotase alfa), in which improvements relative to a certain value or score demonstrate that the sALP is effective for treating HPP.

### Alkaline Phosphatase

Asfotase alfa is a human TNSALP (hTNSALP; SEQ ID NO: 1) fusion polypeptide formulated for the treatment of HPP. In particular, asfotase alfa can be used effectively to treat hypophosphatasia (HPP), its symptoms, and gait impairments associated therewith in HPP patients of about 5 to about 15 years of age (e.g., a child or an adolescent having HPP) for an extended period of time (e.g., at least one year, at least two years, at least three years, at least four years, at least five years, at least six years, at least seven years, at least eight years, at least nine years, at least ten years, or longer than ten years, such as for the lifetime of the patient).

Given the results described herein, the treatment methods are not limited to administration of a particular alkaline phosphatase (ALP) or nucleic acid sequence encoding an ALP. Alkaline phosphatases encompass a group of enzymes that catalyze the cleavage of a phosphate moiety (e.g., hydrolysis of pyrophosphate, PPᵢ). There are four known mammalian alkaline phosphatase (ALP) isozymes: tissue nonspecific alkaline phosphatase (TNSALP; described further below), placental alkaline phosphatase (PLALP; e.g., Accession Nos. P05187, NP_112603, and NP_001623), germ cell alkaline phosphatase (GALP; e.g., Accession No. P10696), and intestinal alkaline phosphatase (IALP; e.g., Accession Nos. P09923 and NP_001622). In addition to the exemplary ALPs discussed above, any polypeptide having the identical or similar catalytic site structure and/or enzymatic activity of ALP can be used (e.g., as an sALP or an sALP fusion polypeptide as defined herein) for treating HPP patients, such as HPP patients of about 5 to about 15 years of age (e.g., a child or an adolescent having HPP). Bone delivery conjugates including sALP are further described in PCT publication Nos. WO 2005/103263 and WO 2008/138131.

TNSALPs that can be used according to the methods described herein include, e.g., human TNSALP (Accession Nos. NP_000469, AAI10910, AAH90861, AAH66116, AAH21289, and AAI26166); rhesus TNSALP (Accession No. XP_01109717); rat TNSALP (Accession No. NP_037191); dog TNSALP (Accession No. AAF64516); pig TNSALP (Accession No. AAN64273); mouse (Accession No. NP_031457); cow TNSALP (Accession Nos. NP_789828, NP_776412, AAM 8209, and AAC33858); cat TNSALP (Accession No. NP_001036028); and variants thereof having 90, 95, 97, or 99% sequence identity to any one of SEQ ID NOs: 7-19. In particular, TNSALP can be a recombinant human TNSALP (e.g., SEQ ID NO: 1, asfotase alfa; see U.S. Patent Nos. 7,763,712 and 7,960,529, used for the treatment of HPP patients of about 5 to about 15 years of age (e.g., a child or an adolescent having HPP). The TNSALP can also be one that exhibits at least about 95% sequence identity to the polypeptide or nucleic acid sequence of the above-noted TNSALPs.

### Soluble Alkaline Phosphatases

The ALPs that can be used in the methods include soluble (e.g., extracellular or non-membrane-bound) forms of any of the alkaline phosphatases described herein. The sALP can be, for example, a soluble form of human tissue non-specific alkaline phosphatase (human TNSALP (hTNSALP)). The methods are not limited to a particular sALP and can include any sALP that is physiologically active toward, e.g., phosphoethanolamine (PEA), inorganic pyrophosphate (PPi), and pyridoxal 5'-phosphate (PLP). In particular, an sALP is one that is catalytically competent to improve skeletal mineralization in bone. The methods further include nucleic acids encoding the sALPs described herein that can be used to treat HPP patients of about 5 to about 15 years of age (e.g., a child or an adolescent having HPP).

TNSALP is a membrane-bound protein anchored by a glycolipid moiety at the C-terminal (Swiss-Prot, P05186). This glycolipid anchor (GPI) is added post-translationally after the removal of a hydrophobic C-terminal end, which serves both as a temporary membrane anchor and as a signal for the addition of the GPI. While the GPI anchor is located in the cell membrane, the remaining portions of TNSALP are extracellular. In particular, TNSALP (e.g., human TNSALP (hTNSALP)) can be engineered to replace the first amino acid of the hydrophobic C-terminal sequence (an alanine) with a stop codon, thereby producing an engineered hTNSALP that contains all amino acid residues of the native anchored form of TNSALP and lacks the GPI membrane anchor. One skilled in the art will appreciate that the position of the GPI membrane anchor will vary in different ALPs and can include, e.g., the last 10, 12, 14, 16, 18, 20, 21, 22, 23, 24, 25, 26, 27, 28, 29, 30, 32, 34, 36, 38, 40, 45, 50, or more amino acid residues on the C-terminus of the polypeptide. Recombinant sTNSALP can include, e.g., amino acids 1 to 502 (18 to 502 when secreted), amino acids 1 to 501 (18 to 501 when secreted), amino acids 1 to 504 (18 to 504 when secreted), amino acids 1 to 505 (18-505 when secreted), or amino acids 1 to 502. Thus, the C-terminal end of the native ALP can be truncated by certain amino acids without affecting ALP activity.

In addition to the C-terminal GPI anchor, TNSALP also has an N-terminal signal peptide sequence. The N-terminal signal peptide is present on the synthesized protein when it is synthesized, but cleaved from TNSALP after translocation into the ER. The sALPs include both secreted (i.e., lacking the N-terminal signal) and non-secreted (i.e., having the N-terminal signal) forms thereof. One skilled in the art will appreciate that the position of the N-terminal signal peptide will vary in different alkaline phosphatases and can include, for example, the first 5, 8, 10, 11, 12, 13, 14, 15, 16, 17, 18, 19, 20, 21, 22, 23, 24, 25, 27, 30, or more amino acid residues on the N-terminus of the polypeptide. One of skill in the art can predict the position of a signal sequence cleavage site, e.g., by an appropriate computer algorithm such as that described in Bendtsen et al. (J. Mol. Biol. 340(4):783-795, 2004) and/or at www.cbs.dtu.dk/services/SignaIP/.

The methods can also be performed using sALP consensus sequences derived from the extracellular domain of ALP isozymes (e.g., TNSALP, PALP, GCALP, or IALP). Thus, similar to sTNSALP discussed above, the present disclosure also provides other soluble human ALP isozymes, i.e., without the peptide signal, preferably comprising the extracellular domain of the ALPs. The sALPs also include polypeptide sequences satisfying a consensus sequence derived from the ALP extracellular domain of human ALP isozymes and of mammalian TNSALP orthologs (human, mouse, rat, cow, cat, and dog) or a consensus derived from the ALP extracellular domain of just mammalian TNSALP orthologs (human, mouse, rat, cow, cat, and dog). The sALPs also include those which satisfy similar consensus sequences derived from various combinations of these TNSALP orthologs or human ALP isozymes. Such consensus sequences are given, for example, in WO 2008/138131.

sALPs of the present methods can include not only the wild-type sequence of the sALPs described above, but any polypeptide having at least 50% (e.g., 55%, 60%, 65%, 70%, 75%, 80%, 85%, 86%, 87%, 88%, 89%, 90%, 91%, 92%, 93%, 94%, 95%, 96%, 97%, 98%, 99%, or more) sequence identity to these alkaline phosphatases (e.g., SEQ ID NOs: 1-24; for example the sALP fusion polypeptide of SEQ ID NO: 1 or a polypeptide variant having at least 95% sequence identity to the sequence of SEQ ID NO: 1, e.g., asfotase alfa). According to the preset invention, the sALP comprises an amino acid sequence having at least 95% sequence identity to the amino acid sequence of SEQ ID NO: 1. Examples of mutations that can be introduced into an ALP sequence are described in US Patent Application Publication No. 2013/0323244. An sALP can optionally be glycosylated at any appropriate one or more amino acid residues. In addition, an sALP can have at least 50% (e.g., 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, or more) sequence identity to any of the sALPs described herein (such as TNSALP, for example the sALP fusion polypeptide of SEQ ID NO: 1 or a polypeptide variant having at least 95% sequence identity to the sequence of SEQ ID NO: 1, e.g., asfotase alfa). An sALP can have 1, 2, 3, 4, 5, 6, 7, 8, 9, 10, or more additions, deletions, or substitutions relative to any of the sALPs described herein (such as TNSALP, for example the sALP fusion polypeptide of SEQ ID NO: 1 or a polypeptide variant having at least 95% sequence identity to the sequence of SEQ ID NO: 1, e.g., asfotase alfa).

### sALP Fusion Polypeptides

Any of the sALPs (such as TNSALP, for example the sALP fusion polypeptide of SEQ ID NO: 1 or a polypeptide variant having at least 95% sequence identity to the sequence of SEQ ID NO: 1, e.g., asfotase alfa), linkers, spacers (e.g., Fc regions), and bone-targeting moieties described herein can be combined in a fusion polypeptide, which includes the structures Z-sALP-Y-spacer-X-Wₙ-V, Z-Wₙ-X-spacer-Y-sALP-V, Z-sALP-Y-Wₙ-X-spacer-V, and Z-Wₙ-X-sALP-Y-spacer-V. In particular, the structure of the sALP fusion polypeptide can be Z-sALP-Y-spacer-X-Wₙ-V or Z-Wₙ-X-spacer-Y-sALP-V. The sALP of the sALP fusion polypeptide can be the full-length ALP or functional fragments of ALPs, such as the soluble, extracellular domain of the ALP, as is described herein (e.g., TNSALP, PALP, GCALP and IALP).

Any one of X, Y, Z, and V and/or the spacer can be absent or a linker region including an amino acid sequence of at least one amino acid. For example, X, Y, Z, and V may be a dipeptide sequence (e.g., leucine-lysine or aspartic acid-isoleucine), such as a two residue linker at the Y position (e.g., leucine-lysine) or a two residue linker at the X position (e.g., aspartic acid-isoleucine). For example, sALP fusion polypeptides can have the structure hTNSALP-Fc-D₁₀ (e.g., an sALP fusion polypeptide including the amino acid sequence of SEQ ID NO: 1 or a polypeptide variant having at least 95% sequence identity to the sequence of SEQ ID NO: 1, e.g., asfotase alfa).

The linker region can be of any sequence and length that allows the sALP to remain biologically active, e.g., not sterically hindered. Exemplary linker lengths are between 1 and 200 amino acid residues, e.g., 1-5, 6-10, 11-15, 16-20, 21-25, 26-30, 31-35, 36-40, 41-45, 46-50, 51-55, 56-60, 61-65, 66-70, 71-75, 76-80, 81-85, 86-90, 91-95, 96-100, 101-110, 111-120, 121-130, 131-140, 141-150, 151-160, 161-170, 171-180, 181-190, or 191-200 amino acid residues. For instance, linkers include or consist of flexible portions, e.g., regions without significant fixed secondary or tertiary structure. Exemplary flexible linkers are glycine-rich linkers, e.g., containing at least 50%, 60%, 65%, 70%, 75%, 80%, 85%, 90%, 95%, or even 100% glycine residues. Linkers can also contain, e.g., serine residues. In some cases, the amino acid sequence of linkers consists only of glycine and serine residues. A linker can optionally be glycosylated at any appropriate one or more amino acid residues. Additionally, a linker as described herein can include any other sequence or moiety, attached covalently or non-covalently. The linker can also be absent, in which the spacer (e.g., the Fc region) and the sALP are fused together directly, with no intervening residues.

Useful spacers include, but are not limited to, polypeptides including a Fc region. For example, an sALP can be a fusion polypeptide including an Fc region of an immunoglobulin at the N-terminal or C-terminal domain. An immunoglobulin molecule has a structure that is well known in the art. It includes two light chains (∼23 kD each) and two heavy chains (∼50-70 kD each) joined by inter-chain disulfide bonds. Immunoglobulins are readily cleaved proteolytically (e.g., by papain cleavage) into Fab (containing the light chain and the VH and CH1 domains of the heavy chain) and Fc (containing the CH2 and CH3 domains of the heavy chain, along with adjoining sequences) fragments. Useful Fc fragments as described herein include the Fc fragment of any immunoglobulin molecule, including IgG, IgM, IgA, IgD, or IgE, and their various subclasses (e.g., IgG-1, IgG-2, IgG-3, IgG-4, IgA-1, IgA-2), from any mammal (e.g., human). For instance, the Fc fragment is human IgG-1. The Fc fragments can include, for example, the CH2 and CH3 domains of the heavy chain and any portion of the hinge region. The Fc region can optionally be glycosylated at any appropriate one or more amino acid residues known to those skilled in the art. In particular, the Fc fragment of the fusion polypeptide has the amino acid sequence of SEQ ID NO: 20, or has at least 50% (e.g., 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, or more) sequence identity to SEQ ID NO: 20. Engineered, e.g., non-naturally occurring, Fc regions can be incorporated into the sALP fusion polypeptides described herein, e.g., those described in International Application Pub. No. WO2005/007809. An Fc fragment as described herein can have 1, 2, 3, 4, 5, 6, 7, 8, 9, 10, 11, 12, 13, 14, 15, 16, 17, 18, 19, 20, 25, 30, 35, 40, 50, or more additions, deletions, or substitutions relative to any of the Fc fragments described herein.

Wₙ can be a bone-targeting moiety, e.g., having a series of consecutive aspartate (D) or glutamate (E) residues, in which n = 1 to 50, e.g., n = 3-30, e.g., 5-15, e.g., 1, 2, 3, 4, 5, 6, 7, 8, 9, 10, 11, 12, 13, 14, 15, 16, 17, 18, 19, 20, 21, 22 , 23, 24, 25, 26, 27, 28, 29, 30, 31, 32, 33, 34, 35, 36, 36, 38, 39, 40, 41, 42, 43, 44, 45, 46, 47, 48, 49, or 50. The bone-targeting moiety, if present, can be positioned anywhere in the fusion polypeptide, e.g., at or near the N-terminal or C-terminal end, and/or in the linker region. For instance, the bone-targeting moiety can be present at the C-terminal end of an sALP fusion polypeptide. sALP fusion polypeptides can also lack a bone-targeting moiety.

Additional amino acid residues can be introduced into the polypeptide according to the cloning strategy used to produce the fusion polypeptides. For instance, the additional amino acid residues do not provide an additional GPI anchoring signal so as to maintain the polypeptide in a soluble form. Furthermore, any such additional amino acid residues, when incorporated into the polypeptide of the methods, do not provide a cleavage site for endoproteases of the host cell. The likelihood that a designed sequence would be cleaved by the endoproteases of the host cell can be predicted as described, e.g., by Ikezawa (Biol. Pharm. Bull. 25:409-417, 2002).

The sALP fusion polypeptides (such as a TNSALP, for example the sALP fusion polypeptide of SEQ ID NO: 1 or a polypeptide variant having at least 95% sequence identity to the sequence of SEQ ID NO: 1, e.g., asfotase alfa) can be associated into dimers or tetramers. For example, two sALP-Fc monomers can covalently be linked through two disulfide bonds located in the hinge regions of the Fc fragments. Additionally, the sALP fusion polypeptide (e.g., an sALP or an sALP fusion polypeptide) can be glycosylated or PEGylated.

### Production of Nucleic Acids and Polypeptides

The nucleic acids encoding sALP (such as TNSALP, for example the sALP fusion polypeptide of SEQ ID NO: 1 or a polypeptide variant having at least 95% sequence identity to the sequence of SEQ ID NO: 1, e.g., asfotase alfa) can be produced by any method known in the art. Typically, a nucleic acid encoding the desired polypeptide is generated using molecular cloning methods, and is generally placed within a vector, such as a plasmid or virus. The vector is used to transform the nucleic acid into a host cell appropriate for the expression of the fusion polypeptide. Representative methods are disclosed, for example, in Maniatis et al. (Cold Springs Harbor Laboratory, 1989). Many cell types can be used as appropriate host cells, although mammalian cells are preferable because they are able to confer appropriate post-translational modifications. Host cells can include, e.g., Chinese Hamster Ovary (CHO) cell, L cell, C127 cell, 3T3 cell, BHK cell, COS-7 cell or any other suitable host cell known in the art. For example, the host cell is a Chinese Hamster Ovary (CHO) cell (e.g., a CHO-DG44 cell).

The sALPs (such as TNSALP, for example the sALP fusion polypeptide of SEQ ID NO: 1 or a polypeptide variant having at least 95% sequence identity to the sequence of SEQ ID NO: 1, e.g., asfotase alfa) can be produced under any conditions suitable to effect expression of the sALP polypeptide in the host cell. Such conditions include appropriate selection of a media prepared with components such as a buffer, bicarbonate and/or HEPES, ions like chloride, phosphate, calcium, sodium, potassium, magnesium, iron, carbon sources like simple sugars, amino acids, potentially lipids, nucleotides, vitamins and growth factors like insulin; regular commercially available media like alpha-MEM, DMEM, Ham's-F12, and IMDM supplemented with 2-4 mM L-glutamine and 5% Fetal bovine serum; regular commercially available animal protein free media (i.e., HYCLONE™, GE Healthcare; SFM4CHO, Sigma CHO DHFR⁻; Cambrex POWER™ CHO CD supplemented with 2-4 mM L-glutamine, etc.). These media are desirably prepared without thymidine, hypoxanthine and L-glycine to maintain selective pressure, allowing stable protein-product expression.

### Pharmaceutical compositions and formulations

A composition including an sALP that can be used in the methods (such as TNSALP, for example the sALP polypeptide of SEQ ID NO: 1 or a polypeptide variant having at least 95% sequence identity to the sequence of SEQ ID NO: 1, e.g., asfotase alfa) can be administered by a variety of methods known in the art. For example, asfotase alfa (SEQ ID NO: 1) can be administered at a range of dosages, in a variety of formulations, and in combination with pharmaceutically acceptable carriers or vehicles. In particular, asfotase alfa is a sterile, preservative-free, nonpyrogenic, clear, slightly opalescent or opalescent, colorless to slightly yellow, with few small translucent or white particles, aqueous solution that is formulated for, e.g., subcutaneous administration. Asfotase alfa can be supplied in glass single-use vials containing asfotase alfa in combination with dibasic sodium phosphate, heptahydrate; monobasic sodium phosphate, monohydrate; and sodium chloride at a pH between 7.2 and 7.6.

### Dosage

Any amount of a pharmaceutical composition including an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) can be administered to HPP patients of about 5 to about 15 years of age (e.g., a child or an adolescent having HPP). The dosages will depend on many factors including the mode of administration and the age of the patient. Typically, the amount of the composition including an sALP contained within a single dose will be an amount that is effective to treat HPP as described herein without inducing significant toxicity.

For example, the sALPs (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) can be administered to HPP patients of about 5 to about 15 years of age (e.g., a child or an adolescent having HPP, in particular, those having an mPOMA-G score of less than about 8 (e.g., less than, or about, 5)), in individual doses ranging, e.g., from 0.01 mg/kg to 500 mg/kg (e.g., from 0.05 mg/kg to 500 mg/kg, from 0.1 mg/kg to 20 mg/kg, from 5 mg/kg to 500 mg/kg, from 0.1 mg/kg to 100 mg/kg, from 10 mg/kg to 100 mg/kg, from 0.1 mg/kg to 50 mg/kg, 0.5 mg/kg to 25 mg/kg, 1.0 mg/kg to 10 mg/kg, 1.5 mg/kg to 5 mg/kg, or 2.0 mg/kg to 3.0 mg/kg) or from 1 µg/kg to 1,000 µg/kg (e.g., from 5 µg/kg to 1,000 µg/kg, from 1 µg/kg to 750 µg/kg, from 5 µg/kg to 750 µg/kg, from 10 µg/kg to 750 µg/kg, from 1 µg/kg to 500 µg/kg, from 5 µg/kg to 500 µg/kg, from 10 µg/kg to 500 µg/kg, from 1 µg/kg to 100 µg/kg, from 5 µg/kg to 100 µg/kg, from 10 µg/kg to 100 µg/kg, from 1 µg/kg to 50 µg/kg, from 5 µg/kg to 50 µg/kg, or from 10 µg/kg to 50 µg/kg).

Exemplary doses of an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) include, but are not limited to, 0.01, 0.05, 0.1, 0.5, 1, 2, 2.5, 5, 10, 20, 25, 50, 100, 125, 150, 200, 250, or 500 mg/kg; or 1, 2, 2.5, 5, 10, 20, 25, 50, 100, 125, 150, 200, 250, 500, 750, 900, or 1,000 µg/kg. In particular, compositions including sALP in accordance with the present disclosure can be administered to HPP patients (e.g., children and adolescents with HPP) in doses ranging from about 0.001 mg/kg/day to about 500 mg/kg/day, about 0.01 mg/kg/day to about 100 mg/kg/day, or about 0.01 mg/kg/day to about 20 mg/kg/day.

For example, the sALP compositions (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) can be administered to patients in a weekly dosage ranging, e.g., from about 0.5 mg/kg/week to about 140 mg/kg/week, e.g., about 0.8 mg/kg/week to about 50 mg/kg/week, or about 1 mg/kg/week to about 10 mg/kg/week (e.g., about 6 or about 9 mg/kg/week). In particular, the sALP (such as TNSALP, e.g., asfotase alfa) can be administered at a dosage of 2 mg/kg three times a week (total dose 6 mg/kg/week), 1 mg/kg six times a week (total dose 6 mg/kg/week), 3 mg/kg three times a week (total dose 9 mg/kg/week), 0.5 mg/kg three times a week (total dose of 1.5 mg/kg/week), or 9.3 mg/kg three times a week (total dose 28 mg/kg/week). The dosage will be adapted by the clinician in accordance with conventional factors, such as the extent of the disease, and different parameters from the HPP patient, such as an HPP patient of about 5 to about 15 years of age (e.g., a child or an adolescent having HPP), in particular, those having an mPOMA-G score of less than about 8 (e.g., less than, or about, 5).

Dosages of compositions including sALPs (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) can be provided in either a single or multiple dosage regimens. Doses can be administered, e.g., hourly, bihourly, daily, bidaily, twice a week, three times a week, four times a week, five times a week, six times a week, weekly, biweekly, monthly, bimonthly, or yearly. Alternatively, doses can be administered, e.g., twice, three times, four times, five times, six times, seven times, eight times, nine times, 10 times, 11 times, or 12 times per day. In particular, the dosing regimen is once weekly. The duration of the dosing regimen can be, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 day(s), week(s), or month(s), or even for the remaining lifespan of the HPP patient of about 5 to about 15 years of age (e.g., a child or an adolescent having HPP).

An sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) can be formulated as a solution for injection, which is a clear, colorless to slightly yellow, aqueous solution, pH 7.4. The sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) may be formulated at a concentration of 12mg/0.3mL,18mg/0.45mL, 28mg/0.7mL, 40mg/1ml, or 80mg/0.8mL.

For example, the composition can be formulated as a 40 mg/ml solution for injection, in which each ml of solution contains 40 mg of sALP (e.g., each vial contains 0.3 ml solution and 12 mg of sALP (40 mg/ml), each vial contains 0.45 ml solution and 18 mg of sALP (40 mg/ml), each vial contains 0.7 ml solution and 28 mg of sALP (40 mg/ml), or each vial contains 1.0 ml solution and 40 mg of asfotase alfa (40 mg/ml)). Additionally, an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) can be formulated as a solution for injection at a concentration of 100 mg/ml, in which each 1 ml of solution contains 100 mg of sALP (e.g., each vial contains 0.8 ml solution and 80 mg of asfotase alfa (100 mg/ml)).

For example, the recommended dosage of an sALP (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) is 2 mg/kg of body weight administered subcutaneously three times per week, or a dosage regimen of 1 mg/kg of body weight administered subcutaneously six times per week. Additional dosage information is provided below (Table 1).

According to the present invention, the composition comprising the sALP is used to treat HPP in a subject of 5 to 15 years of age having an average modified Performance-Oriented Mobility Assessment - Gait (mPOMA-G) score of 5 or less, wherein said subject did not exhibit an increase in the average mPOMA-G score of at least 0.6, particularly at least 1.0 or more, after administration of said sALP to the subject at a dose providing 6 mg/kg/week during a treatment period of at least one year, and wherein the dosage of the sALP is increased to 9 mg/kg/week.

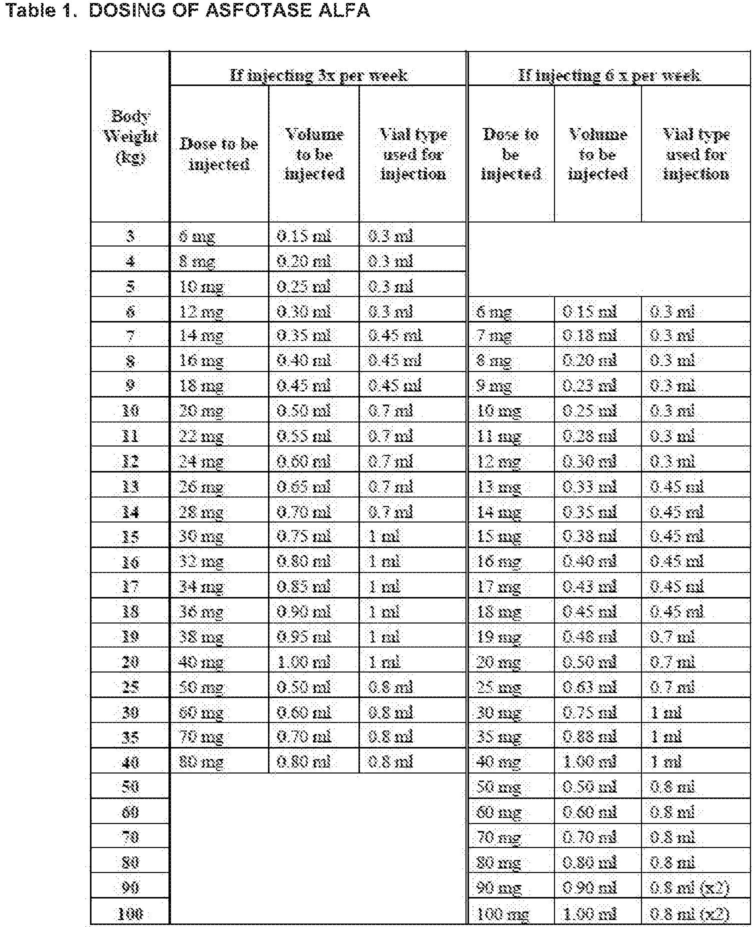

### Formulations

A composition including sALPs (such as TNSALP, for example the sALP polypeptide of SEQ ID NO: 1 or a polypeptide variant having at least 95% sequence identity to the sequence of SEQ ID NO: 1, e.g., asfotase alfa) can be formulated according to standard methods. Pharmaceutical formulation is a well-established art, and is further described in, e.g., Gennaro (2000) Remington: The Science and Practice of Pharmacy, 20th Edition, Lippincott, Williams & Wilkins (ISBN: 0683306472); Ansel et al. (1999) Pharmaceutical Dosage Forms and Drug Delivery Systems, 7th Edition, Lippincott Williams & Wilkins Publishers (ISBN: 0683305727); and Kibbe (2000) Handbook of Pharmaceutical Excipients American Pharmaceutical Association, 3rd Edition (ISBN: 091733096X). For instance, an sALP composition (such as TNSALP, for example the sALP polypeptide of SEQ ID NO: 1 or a polypeptide variant having at least 95% sequence identity to the sequence of SEQ ID NO: 1, e.g., asfotase alfa) can be formulated as a buffered solution at a suitable concentration and suitable for storage at about 2-8°C (e.g., about 4°C). A composition can also be formulated for storage at a temperature below about 0°C (e.g., about -20°C or about -80°C). A composition can further be formulated for storage for up to about 2 years (e.g., one month, two months, three months, four months, five months, six months, seven months, eight months, nine months, 10 months, 11 months, 1 year, 1½ years, or 2 years) at about 2-8°C (e.g., about 4°C). Thus, the compositions described herein can be stable in storage for at least about 1 year at about 2-8°C (e.g., about 4°C).

The compositions including sALPs (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) can be in a variety of forms. These forms include, e.g., liquid, semi-solid and solid dosage forms, such as liquid solutions (e.g., injectable and infusible solutions), dispersions or suspensions, tablets, pills, powders, liposomes and suppositories. The preferred form depends, in part, on the intended mode of administration and therapeutic application.

For example, compositions intended for systemic or local delivery can be in the form of injectable or infusible solutions. Accordingly, the sALP compositions (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) can be formulated for administration by a parenteral mode (e.g., subcutaneous, intravenous, intraperitoneal, or intramuscular injection).

The sALP compositions (such as TNSALP, for example the sALP fusion polypeptide of SEQ ID NO: 1 or a polypeptide variant having at least 95% sequence identity to the sequence of SEQ ID NO: 1, e.g., asfotase alfa) can be formulated as a solution, microemulsion, dispersion, liposome, or other ordered structure suitable for stable storage at high concentration. Sterile injectable solutions can be prepared by incorporating a composition described herein in the required amount in an appropriate solvent with one or a combination of ingredients enumerated above, as required, followed by filter sterilization. Generally, dispersions are prepared by incorporating a composition described herein into a sterile vehicle that contains a basic dispersion medium and the required other ingredients from those enumerated above. In the case of sterile powders for the preparation of sterile injectable solutions, methods for preparation include vacuum drying and freeze-drying that yield a powder of a composition described herein plus any additional desired ingredient (see below) from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition a reagent that delays absorption, for example, monostearate salts, and gelatin.

The sALP compositions (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) described herein can also be formulated in immunoliposome compositions. Such formulations can be prepared by methods known in the art, such the methods described in Epstein et al. (1985) Proc Natl Acad Sci USA 82:3688; Hwang et al. (1980) Proc Natl Acad Sci USA 77:4030; and U.S. Patent Nos. 4,485,045 and 4,544,545. Liposomes with enhanced circulation time are disclosed in, e.g., U.S. Patent No. 5,013,556.

Compositions including an sALP (such as TNSALP, for example the sALP polypeptide of SEQ ID NO: 1 or a polypeptide variant having at least 95% sequence identity to the sequence of SEQ ID NO: 1, e.g., asfotase alfa) can also be formulated with a carrier that will protect the sALP composition against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are known in the art. See, e.g., J.R. Robinson (1978) Sustained and Controlled Release Drug Delivery Systems, Marcel Dekker, Inc., New York.

When sALP compositions (such as TNSALP, for example, an sALP fusion polypeptide, such as the sALP fusion polypeptide of SEQ ID NO: 1 or a polypeptide variant having at least 95% sequence identity to the sequence of SEQ ID NO: 1, e.g., asfotase alfa) are to be used in combination with a second active agent, the compositions can be co-formulated with the second agent, or the compositions can be formulated separately from the second agent formulation. For example, the respective pharmaceutical compositions can be mixed, e.g., just prior to administration, and administered together or can be administered separately, e.g., at the same or different times.

### Carriers/vehicles

Preparations containing an sALP (such as TNSALP, for example the sALP polypeptide of SEQ ID NO: 1 or a polypeptide variant having at least 95% sequence identity to the sequence of SEQ ID NO: 1, e.g., asfotase alfa) can be provided to an HPP patient of about 5 to about 15 years of age, such as a child or an adolescent having HPP, in combination with pharmaceutically acceptable sterile aqueous or non-aqueous solvents, suspensions or emulsions. Examples of non-aqueous solvents are propylene glycol, polyethylene glycol, vegetable oil, fish oil, and injectable organic esters. Aqueous carriers include water, water-alcohol solutions, emulsions or suspensions, including saline and buffered medical parenteral vehicles including sodium chloride solution, Ringer's dextrose solution, dextrose plus sodium chloride solution, Ringer's solution containing lactose, or fixed oils. For example, the pharmaceutically acceptable carrier can include sodium chloride and/or sodium phosphate, in which the composition includes, e.g., about 150 mM sodium chloride and/or about 25 mM sodium phosphate, pH 7.4.

Intravenous vehicles can include fluid and nutrient replenishers, electrolyte replenishers, such as those based upon Ringer's dextrose, and the like. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, can be present in such vehicles. A thorough discussion of pharmaceutically acceptable carriers is available in Remington's Pharmaceutical Sciences (Mack Pub. Co., N.J. 1991).

The following examples are intended to illustrate, rather than limit, the disclosure. These studies feature the administration of asfotase alfa (SEQ ID NO: 1) to a patient of about 5 to about 15 years of age, such as a child or an adolescent, to treat HPP, its symptoms, and gait impairments associated therewith for an extended period of time.

### Example 1. Modification of Performance-Oriented Mobility Assessment - Gait (modified POMA-G,

### mPOMA-G) to evaluate gait in HPP patients

An analysis of gait was performed to compare children and adolescent HPP patients treated with asfotase alfa (SEQ ID NO: 1) to children and adolescent HPP patients that were not treated with asfotase alfa (the historical HPP control ("HC")). Gait impairment has a high prevalence as measured at baseline in asfotase alfa clinical trials, i.e., 100% (8/8) in ages 5-12 and 83% (10/12) in ages ≥13. Gait performance was assessed using a version of the 12-point Performance-Oriented Mobility Assessment - Gait (POMA-G) which was modified in order to provide improved sensitivity for HPP-related impairments (the modified POMA-G or mPOMA-g). The POMA-G had been used previously to evaluate function in adult populations, including patients with amyotrophic lateral sclerosis, normal pressure hydrocephalus, Parkinson's disease, and stroke, and had been validated in elderly and community-dwelling adults (Tinetti, M.E., et al., Am J Med 80(3):429-34 (1986); Faber, M.J., et al., Arch Phys Med Rehabil. 87(7):885-96 (2006); Means K.M., et al., Arch Phys Med Rehabil. 79(12):1570-6 (1998)). The POMA-G has been also been used in deaf children to evaluate balance and gait (Melo et al. Rev. Paul. Pediatr. 30(3): 385-91, 2012). Components of the POMA-G can be used to directly or indirectly measure performance deficits exhibited in HPP patients of about 5 to about 15 years of age; however, the POMA-G, without these modifications, was less appropriate for adequate assessment of HPP patients of about 5 to about 15 years of age.

In particular, the POMA-G can be used to measure step length and height, step symmetry and continuity, trunk sway, and stance, all of which may be abnormal in HPP patients. The POMA-G includes assessments of initiation of gait, step length and step clearance height, symmetry, and continuity; path deviation; trunk sway; and walking stance (Table 2). The lowest score is 0 per item, while 1 or 2 is the highest score per item. Individual item scores are summed to generate a 0 to 12 scale of disability, with lower scores indicating more significant gait impairments and higher scores indicating fewer gait impairments, which would be indicative of, e.g., the gait of a healthy subject.

**Table 2. Unmodified Performance Oriented Mobility Assessment - Gait (POMA-G) Assessments**

| **Assessment** | | **Observation** | **Score** |
|---|---|---|---|
| 1 | Initiation of gait | Any hesitancy or multiple attempts | = 0 |
| | | No hesitancy | = 1 |
| 2 | Step length and height | | |
| | a) Right swing foot | Does not pass the left stance foot with step | = 0 |
| | | Passes the left stance foot | = 1 |
| | b) Right foot clear | Does not clear floor completely with step | = 0 |
| | | Right foot completely clears floor | = 1 |
| | c) Left swing foot | Does not pass the right stance foot with step | = 0 |
| | | Passes the right stance foot | = 1 |
| | d) Left foot clear | Does not clear floor completely with step | = 0 |
| | | Left foot completely clears floor | = 1 |
| 3 | Step symmetry | Right and left step length not equal | = 0 |
| | | Left foot completely clears floor | = 1 |
| 4 | Step continuity | Stopping or discontinuity between steps | = 0 |
| | | Steps appear continuous | = 1 |
| 5 | Path | Marked deviation | = 0 |
| | | Mild/moderate deviation or uses walking aid | = 1 |
| | | Straight without walking aid | = 2 |
| 6 | Trunk | Marked sway or uses walking aid | = 0 |
| | | No sway but flexion of knees or back or spreads arms out while walking | |
| | | No sway, no flexion, no use of arms, and no walking aid | = 2 |
| 7 | Walk stance | Heels apart | = 0 |
| | | Heels almost touching while walking | = 1 |
| Gait score | | | /12 |

The validity of each component of the POMA-G as a measure of clinically important musculoskeletal defects in HPP patients was confirmed by evaluating each component of the scale in the context of physical disabilities specific to HPP. The measures most relevant to pathology in patients with HPP are those that reflect proximal and lower extremity muscle weakness, stability, and base of support. A total of six assessments in the POMA-G specifically measure criteria that indicate proximal and lower extremity muscle weakness, stability, and/or base of support. The specific features of the POMA-G that are relevant to HPP are described below.
- Trunk sway and walking stance (wide base of support to increase postural stability) are indicators of proximal muscle weakness. Abnormalities in these parameters are associated with difficulty navigating obstacles, poor balance, and increased risk of collisions or falls.
- Step length and height are important indicators of both proximal and lower extremity strength. Abnormalities in these parameters interfere with the ability to navigate irregular surfaces and increase the risk of falls and injury.
- Gait asymmetry reflects structural discrepancies between limbs commonly seen in HPP. Gait asymmetry is an important functional measure, because it compromises the ability to navigate obstacles and increases the risk of collisions and falls.
- Gait continuity reflects balance, stability, and fluidity of movement. Gait continuity can affect the patient safety, as forward movement is delayed when step continuity is not present, affecting the stability of gait and risk of fall.

On the other hand, gait initiation is indicative of neurologic abnormality (e.g., Parkinson's disease) and is less likely to be secondary to musculoskeletal defects; therefore, gait initiation was not expected to be affected by the physical disabilities specific to HPP. Additionally, potential issues identified for the item "path" included a possible lack of focused and purposeful gait in children and the potential difficulty in quantifying path deviation in the historical videos in the absence of specific floor markers. The following HPP-specific modifications of the POMA-G were made to optimize the discriminant value of the assessment in patients with HPP, while preserving the clinometric properties of the underlying instrument.
- Item 1 (initiation of gait) was removed, because patients with HPP do not typically have difficulty initiating gait.
- Item 5 (path) was removed, because the nature and quality of the clinical video footage was not expected to permit a reliable assessment of path deviations, as well as the potential for the absence of focused and purposeful movement as typical of young children.
- Item 2 (step length), the scale was changed from a 2 point (0, 1) rating scale to a 3 point (0, 1, 2) scale with detailed definitions for each score to provide greater sensitivity and precision to detect change. Clinical relevance applied to increased risk of trips and falls, decreased ability to climb standard stairs or to climb school bus stairs with an increased vertical rise. Similar to ambulation, stair climbing with a mature pattern of alternating feet requires adequate step length and hip extension on the stance limb. A reduced step size requires more steps to ambulate a given distance, a possible increased fatigue and difficulties keeping up with peers.
- Item 4 (step continuity), the scale was changed from a 2 point (0, 1) rating scale to a 3 point (0, 1, 2) scale to have a partial credit score of 2 for emerging step continuity and greater sensitivity and precision to detect change. A score of two was given if step continuity was not seen bilaterally and was only present on one side or if bilaterally a heel strike was present at initial contact with a foot flat position on the stance limb. Clinical relevance applied to slow and inefficient ambulation and reduced stability that could impact ability to move between classes in the allotted time and difficulties with participation in school activities.

In addition, the resulting mPOMA-G analysis included clarifying descriptions of all items, including trunk sway and walk stance, to increase sensitivity and consistency among raters were provided. A scoring key was also created to provide additional detail on item rating and to include examples of a child demonstrating step continuity (Fig. 1) and for step clearance, evaluation of hip and knee flexion at midswing (Fig. 2) for reference during rating.

The mPOMA-G, optimized for analysis of HPP patients, is shown below (Table 3). Similarly to the POMA-G, 0 is the lowest score per item, and 1 or 2 is the highest score per item. As in the POMA-G, individual item scores are summed to generate a 0 to 12 scale of disability, with lower scores indicating greater disability. The modifications improved the HPP-specific sensitivity to change and the discriminant capacity, while preserving the historical clinometric properties of the unmodified POMA-G. Additionally, the mPOMA-G required no special instrumentation or camera angles, showed robust inter-rater reliability, and was well-correlated with other measures of clinically meaningful functionality.

**Table 3. Modified Performance Oriented Mobility Assessment - Gait (mPOMA-G) Assessment**

| **Assessment** | | **Observation** | **Score** |
|---|---|---|---|
| 1 | Step length and height | | |
| | a) Right swing foot | Does not pass the left stance foot with step | = 0 |
| | | Right heel passes the left stance foot | = 1 |
| | | Right foot passes the left stance foot by at least the length of individual's foot between the stance toe and swing heel | = 2 |
| | b) Right foot clear | Right foot does not clear floor completely with step or raises foot by more than 1 - 2 inches | = 0 |
| | | Right foot completely clears floor | = 1 |
| | c) Left swing foot | Does not pass the right stance foot with step | = 0 |
| | | Left heel passes the right stance foot | = 1 |
| | | Left foot passes the right stance foot by at least the length of individual's foot between the stance toe and swing heel | = 2 |
| | d) Left foot clear | Left foot does not clear floor completely with step or raises foot by more than 1 - 2 inches | = 0 |
| | | Left foot completely clears floor | = 1 |
| 2 | Step symmetry | Right and left step length not equal (estimate) | = 0 |
| | | Right and left step appear equal | = 1 |
| 3 | Step continuity | Stopping or discontinuity between steps | = 0 |
| | | Steps appear continuous unilaterally (observe raising heel of 1 foot as heel of other foot touches the floor, unilaterally) or fiat foot contact on stance limb when heel of other foot touches the floor bilaterally, no breaks or stops in stride | = 1 |
| | | Steps appear continuous bilaterally (observe raising heel of 1 foot as heel of other foot touches the floor, bilaterally), no breaks or stops in stride, step lengths equal | = 2 |
| 4 | Trunk stability | Marked sway or uses walking aid. Marked sway = moderate lateral flexion as the result of instability bilateral or unilateral | = 0 |
| | | No marked sway but compensatory patterns such as trunk flexion, knee flexion, arm abduction or retraction to increase postural stability while walking | = 1 |
| | | No sway, no flexion, no use of arms, and no walking aid | = 2 |
| 5 | Walk stance | Heels always apart, wide base of support utilized to increase postural stability | = 0 |
| | | Heels intermittently apart | = 1 |
| Gait score | | | /12 |

### Example 2. HPP patients treated with asfotase alfa and historical HPP controls

All HPP patients included in the mPOMA-G analyses were required to have: 1) documented diagnosis of HPP with onset of symptoms ≥6 months of age (i.e., childhood HPP), and 2) ≥2 videos of sufficient quality to assess gait between the ages of 5 and 15 years. The either asfotase-alfa treated patients were enrolled in an ongoing, phase 2, open-label study, in which asfotase alfa was administered at a dosage of 6 mg/kg/week to these patients. Of the treated HPP patients, five HPP patients had data from videos both prior to and after initiation of treatment with asfotase alfa, and thus, a pre-treatment change in gait was compared to the on-treatment change in gait. The six HC HPP patients were enrolled in a non-interventional functional natural history study and served as the non-concurrent control group for the eight juvenile-onset patients treated with asfotase alfa for these gait analyses. All historical HPP control patients either declined participation in the treatment trials, or no longer met the age criteria.

Demographic and baseline characteristics for the patient groups are shown in Table 4. Across the groups, the majority of patients were white males. Overall, the HC patients and the all treated patients were similar with respect to age at HPP onset, baseline, and last gait assessment. HPP patients (n=5) with available pre-treatment data tended to be slightly younger at the onset of HPP symptoms and in their initial assessment compared to the HC group.

**Table 4. Baseline characteristics for historical HPP control patients, patients with pre-treatment and on-treatment gait data, and all treated patients (treatment with asfotase alfa).**

| **Variable** | **Juvenile-Onset Historical HPP control (N=6)** | **Juvenile-Onset Pre-& On-Treatment (N=5)** | **Juvenile-Onset All Treated with Asfotase Alfa (N=8)** |
|---|---|---|---|
| Age at Enrollment (months), n | ND^{a} | ND^{b} | 8 |
| Mean (SD) | NA | NA | 101.70 (23.936) |
| Median | NA | NA | 100.35 |
| Min, Max | NA | NA | 72.3, 144.5 |

| **Age at Onset of HPP (months) n** | **6** | **5** | **8** |
|---|---|---|---|
| Mean (SD) | 16.5 (11.24) | 14.59 (4.319) | 15.25 (4.027) |
| Median | 11.5 | 12.00 | 13.50 |
| Min, Max | 8, 36 | 12.0, 22.0 | 12.0, 22.0 |

| **Age at Baseline Gait Assessment (years), n** | **6** | **5** | **8** |
|---|---|---|---|
| Mean (SD) | 7.10 (2.208) | 6.68 (1.839) | 8.58 (1.972) |
| Median | 6.20 | 6.30 | 8.45 |
| Min, Max | 5.3, 10.7 | 4.3, 9.1 | 6.2, 12.1 |

| **Age at Last Gait Assessment (years)** | **6** | **5** | **8** |
|---|---|---|---|
| Mean (SD) | 10.95 (2.279) | 8.86 (2.033) | 10.31 (1.840) |
| Median | 11.05 | 8.70 | 10.20 |
| Min, Max | 8.2, 14.9 | 6.5, 12.1 | 8.2, 14.1 |

| **Sex** | | | |
|---|---|---|---|
| Male, n (%) | 6 (100.0) | 4 (80.0) | 6 (75.0) |
| Female, n (%) | 0 (0.0) | 1 (20.0) | 2 (25.0) |

| **Ethnicity** | | | |
|---|---|---|---|
| Hispanic or Latino, n (%) | 2 (33.3) | 0 | 1 (12.5) |
| Not Hispanic or Latino, n (%) | 4 (66.7) | 5(100.0) | 7 (87.5) |

| **Race** | | | |
|---|---|---|---|
| American Indian or Alaskan Native, n (%) | 0 | 0 | 0 |
| Asian, n (%) | 0 | 0 | 0 |
| Black or African American, n (%) | 0 | 0 | 0 |
| Native Hawaiian or Other Pacific Islander, n (%) | 0 | 0 | 0 |
| White, n (%) | 6 (100.0) | 5 (100.0) | 8 (100.0) |
| Other, n (%) | 0 | 0 | 0 |

| | | | |
|---|---|---|---|
| Abbreviations: max = maximum, min = minimum, ND = not done, SD = standard deviation ^{a} Age at enrollment was not applicable for the historical HPP control patients because data were abstracted retrospectively from medical records. ^{b} Age at enrollment was not calculated for the sub-set of treated patients with pre- and post-treatment data. | | | |

The medical history most relevant to gait for the treated and HC patients is shown in Table 5. All eight HPP patients treated with asfotase alfa and all six HC patients had a history of gait disturbance. Bone deformity (bowing of the long bones), bone pain, and muscular weakness were the most frequent symptoms reported across the patient groups. Gait deficits were also noted at the baseline assessment of the mPOMA-G The most common deficits (foot clearance, stance, proximal sway/trunk, and step length) implicate loss of proximal muscle strength in the pathophysiology of HPP and the subsequent development of compensatory strategies in HPP patients (e.g., waddling gait pattern and increased base of support).

**Table 5. Relevant HPP-specific medical history for patient populations.**

| | **Juvenile-Onset Historical HPP (N=6)** | **Juvenile-Onset Pre-& On-Treatment (N=5)** | **Juvenile-Onset All Treated (N=8)** |
|---|---|---|---|
| | **n(%)** | **n(%)** | **n (%)** |
| **Arthralgia** / **Joint Pain** | **3 (50.0)** | **1 (20.0)** | **2 (25.0)** |
| Joint pain that limited daily activities | 3 (50.0) | - | - |
| Joint pain that required pain medications for management | 3 (50.0) | - | - |
| **Bone deformity (Bowing of the long bones)** | **6 (100.0)** | **2 (40.0)** | **3 (37.5)** |
| **Bone pain** | **3 (50.0)** | **2 (40.0)** | **4 (50.0)** |
| Bone pain that limited daily activities | 3 (50.0) | 1 (20.0) | 2 (25.0) |
| Bone pain that required pain medications for management | 2 (33.3) | 1 (20.0) | 2 (25.0) |
| **Fracture** | **3 (50.0)** | **1 (20.0)** | **2 (25.0)** |
| **Gait disturbance** | **6 (100.0)** | **5 (100.0)** | **8 (100.0)** |
| Unusual gait | 5 (83.3) | - | - |
| Waddling gait | 4 (66.7) | - | - |
| **Muscular weakness** | **3 (50.0)** | **3 (60.0)** | **5 (62.5)** |
| Muscle weakness that limited daily activities | 3 (50.0) | - | - |
| **Myalgia** / **Muscle Pain** | **2 (33.3)** | **3 (60.0)** | **4 (50.0)** |
| Muscle pain that limited daily activities | 2 (33.3) | | |
| Muscle pain that required pain medications for management | 1 (16.7) | | |
| Scoliosis | 1 (16.7) | | |
| **Gait Deficits (mPOMA-G)**^{a} | **6 (100.0) ^{a}** | **5 (100.0) ^{b}** | **8 (100.0) ^{a}** |
| Step length (swing) | 4 (66.7) | 4 (80.0) | 7 (87.5) |
| Steppage (foot clearance) | 5 (83.3) | 4 (80.0) | 8 (100.0) |
| Proximal sway/trunk | 6 (100.0) | 5 (100.0) | 8 (100.0) |
| Stance | 2 (33.3) | 2 (40.0) | 6 (75.0) |

| | | | |
|---|---|---|---|
| ^{a} Gait deficit data based on mPOMA-G scores at baseline. ^{b} Gait deficit data based on mPOMA-G scores at historical time point. | | | |

### Example 3. Collection of gait data to determine mPOMA-G of patient populations

Three qualified and trained physical therapists scored videos of walking using the mPOMA-G. The raters did not participate in caring for enrolled patients and were masked to patient identifiers and the dates of video recording. All raters were provided an Independent Rater Manual describing the process for scoring the functional videos in support of their training. Visible faces on the videos were permanently blurred, and videos were masked and randomized prior to scoring

The mPOMA-G score for each available time point was obtained by summing the median score (across the 3 raters) for each individual component at that time point. For the HC patients, baseline was defined as the earliest available mPOMA-G score within the period from 5 to 15 years of age (allowing for a 2-week window around these ages). Change from baseline was computed for each post-baseline time point. The last available assessment was used for the comparison.

For asfotase alfa-treated patients, the last available assessment was defined as the last time point for which the time from baseline was less than or equal to the median time from baseline among mPOMA-G analysis results from HC patients. The time point for the last assessment in treated patients was always less than the median time from baseline in the HC patients; therefore, no data were censored.

Rate of change in mPOMA-G score was compared between treated and HC patients (primary endpoint) using a Wilcoxon rank-sum test with a 2-sided alpha of 0.05, an exact method for determining the *P*-value. For each patient, the rate of change was computed by dividing the change from baseline to last assessment available in mPOMA-G score by the time (in years) from baseline to last assessment. For asfotase alfa-treated HPP patients with a video available prior to the baseline assessment, rate of change per year in mPOMA-G score was determined for the time period between pre-baseline and the baseline assessment in addition to between baseline and the last assessment on treatment. Within-group changes from baseline and from pre-treatment assessments were evaluated using the Wilcoxon signed-rank statistical test.

### Example 4. Validation of mPOMA-G to assess gait improvements in HPP patients

Intraclass correlation coefficients (ICCs) were calculated to assess overall inter-rater and intra-rater agreement. Raters scored videos of children with childhood and adolescent HPP (≥6 months of age) from 2 clinical studies (asfotase alfa clinical trial participants, n=8; natural history study participants, n=6), as is described in Example 2. For inter-rater ICC, a two-way random effects analysis of variance model with terms for subject-visit, rater, and the interaction between subject-visit and rater was used. For intra-rater ICC, a two-way random effects analysis of variance model with terms for subject-visit-rater, review (original or retest), and the interaction between subject visit-rater and review was used. The null hypothesis was tested with a 0.025 level of significance, and was rejected only if ICC>0. Inter-rater agreement across all visits was excellent (ICC=0.76, p<0.0001, number of observations=192). Likewise, intra-rater agreement across all visits was also excellent (ICC=0.76, p<0.001, number of observations=192).

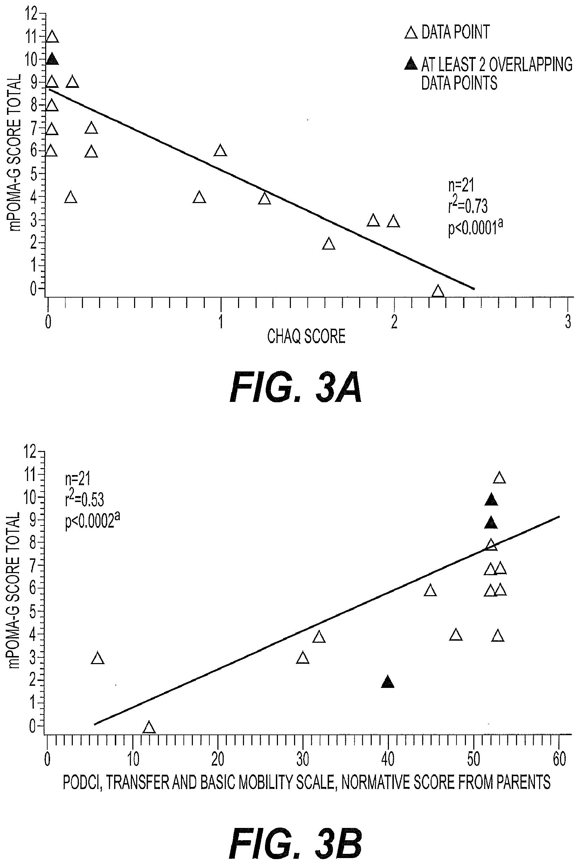

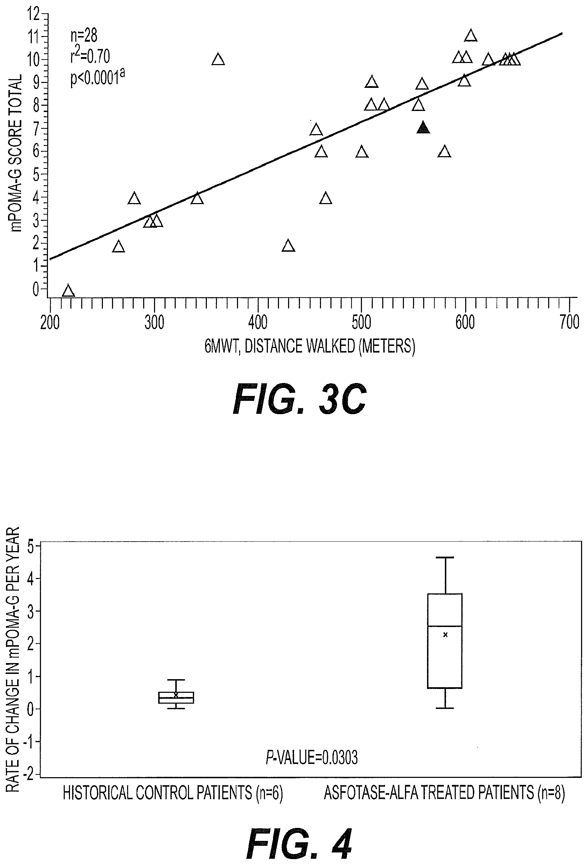

For concurrent validity analysis, linear regression was performed to determine the relationship between mPOMA-G scores and other clinical outcome measures in children and adolescents treated with asfotase alfa (n=5), which included the Childhood Health Assessment Questionnaire (CHAQ) Disability Index, Pediatric Outcomes Data Collection Instrument (PODCI) Transfer and Mobility Scale (normative score reported by parents), and the Six Minute Walk Test (6MWT; distance walked in meters). The 6MWT was chosen because this metric measure ambulatory capacity, and the PODCI and CHAQ were chosen because these metrics measure ability to participate in normal activities of daily living (e.g., climbing stairs and getting onto a bus) and community sports and recreation. There were strong correlations between the mPOMA-G scores of HPP patients and HC patients with measures of ability to perform activities of daily living as assessed using the CHAQ (Fig. 3A) and PODCI (Fig. 3B) in addition to measures of physical function as assessed using the 6MWT (Fig. 3C).

### Example 5. mPOMA-G Minimum Clinically Important Difference

A minimum clinically meaningful difference (MCID) in mPOMA-G score was prospectively determined based on the relationship between mPOMA-G score and other endpoints for which reports of clinically meaningful changes were available, i.e., the CHAQ disability index (change of -0.13) (Dempster, H., et al., Arthritis & Rheumatism 44(8):1768-74 (2001)), PODCI transfer and basic mobility scale normative score from parents (change of 4.5) (see Henricson, E., et al., PLOS Currents Muscular Dystrophy 2013, 1-21, 6MWT and Duchenne muscular dystrophy; Oeffinger, D., et al., Dev Med & Child Neurol 50: 918-925 (2008), ambulatory cerebral palsy in children), and 6MWT distance walked (30 meters) (McDonald, C.M. et al., Muscle Nerve 48:357-68 (2013)). Using data from the 5 infantile-onset HPP patients (prior to analyzing the juvenile-onset data), separate linear regressions were performed for the mPOMA-G with the CHAQ (r² = 0.73 [21 data points]), PODCI (r² = 0.53 [21 data points]), and 6MWT (r² = 0.70 [28 data points]).

Using the resulting linear equations, a clinically meaningful change of -0.13 on the CHAQ corresponded with a change of 0.45 in mPOMA-G score, a clinically meaningful change of 4.5 on the PODCI Test of Motor Proficiency corresponded with a change of 0.76 in mPOMA-G score, and a clinically meaningful change of 30 meters on the 6MWT correlated with a change of 0.59 in mPOMA-G score. Of these, the CHAQ and PODCI were used because they are disability measures for which minimally clinically important differences in pediatric populations were expected to be relatively disease-independent, and therefore better for correlative analysis. The correlations with minimal clinically important 6MWT changes were also included for completeness. Based on results of all three analyses, a change of 0.6 in mPOMA-G score was prospectively identified as a clinically meaningful change.

These data demonstrate that mPOMA-G analysis is a reliable method for detecting clinically significant impairments in gait, as well as clinically significant changes in impairments in gait, using videos of children and adolescents with HPP. Accordingly, mPOMA-G score showed strong concurrent association with physical function and patient-reported disability when performing activities of daily living.

### Example 6. Analysis of physical function and gait impairments in historical HPP controls

The HC patients varied in mobility from the most severe patient, who required a walker or wheelchair up to 50% of the time, to the highest functioning patient, who could participate in community sports. For all patients, activity was limited by fatigue and/or pain. Some patients had challenges in keeping up with healthy peers. Muscle weakness was noted using manual muscle testing in these patients and was most commonly seen in hip extensors and hip abductors.

Three raters assessed videos of the HC patients for gait descriptors including genu varum/valgus, position of knees and hips at terminal stance, and use of orthotics; running descriptors including arm swing, presence of Trendelenburg, steppage, circumduction or scissoring, foot contact and push off pattern, and presence/absence of period of flight; the ability to stand on one foot; and the ability to rise from floor to standing without use of hands.

Genu valgum or "knock-knee" was observed in 67% (4/6) HC patients at first assessment, and all six HC patients exhibited genu valgum at last assessment. A running pattern that utilized strategies to compensate for decreased proximal stability and weakness was observed in all six HC patients. At last assessment, 67% of HC patients were unable to achieve a period of flight, indicating that actual running was not achieved. According to the Peabody Developmental Motor Scales (PDMS-2), the mean normative age to acquire running with a period of flight with both feet in the air is approximately 1.6 years, while a mature running pattern is typically acquired by approximately 3.4 years of age. For a description of the PDMS-2 scales, see van Hartingsveldt et al. (Occup. Ther. Int. 12(1): 1-13, 2005).

Time standing on one foot was lower in all HCs than has been reported previously in healthy age-matched peers, which was 1.5 to 5.6 sec in all six (100%) HCs, which were older than 5.3 years at first assessment. For reference, the PDMS-2 mean normative age to achieve standing on one foot for 5 seconds is approximately 3.8 years. Trendelenburg signs (indicative of weak abductor muscles of the hip) were noted in all six HCs. All HCs required self-support with one or two hands to transition from the floor to standing, further indicating proximal instability or weakness.

Gait of the HCs was then assessed using the mPOMA-G, as described in Examples 1-3. Each HC had one pair of videos for gait evaluation. The median (min, max) time between first assessment and last assessment was 49.2 (24, 71) months. All HCs demonstrated measurable gait impairments at first assessment, with mPOMA-G scores ranging from 3 to 11 (median 6; Table 6). All six HCs retained gait impairments from first assessment to last assessment, with mPOMA-G scores ranging from 4 to 11 (median 7.5) at last assessment. There was no consistent pattern of change in mPOMA-G components (Table 7).

**Table 6. Gait assessment of HPP historical HPP controls.**

| **Median (min, max), n=6** | **First Assessment** | **Last Assessment** | **Change from First Assessment** |
|---|---|---|---|
| Aqe at assessment, years | 6.2 (5.3, 10.7) | 11.1 (8.2, 14.9) | 4.1 (2.1, 5.9) |
| mPOMA-G score | 6.00 (3.0, 11.0) | 7.50 (4.0, 11.0) | 1.50 (0.0, 2.0), *P*=0.0625 |

| | | | |
|---|---|---|---|
| *P*-value based on nonparametric sign test to determine whether the median change from baseline differed from 0. | | | |

**Table 7. Change in mPOMA-G components* of historical HPP controls.**

| **Assessment (min, max possible points)** | **Median score (min, max)** | | **Number of patients with change (n=6)** |
|---|---|---|---|
| | **First Assessment** | **Last Assessment** | |
| Right foot clear (0, 1) | 0 (0, 1) | 0 (0, 1) | 0 |
| Left foot clear (0, 1) | 0 (0, 1) | 0 (0, 1) | 0 |
| Right swing foot (0, 2) | 1 (1, 2) | 1.5 (1, 2) | 1 |
| Left swing foot (0, 2) | 1 (1, 2) | 1.5 (1, 2) | 1 |
| Step symmetry (0, 1) | 1 (0, 1) | 1 (0, 1) | 0 |
| Step continuity (0, 2) | 1 (1, 2) | 1.5 (1, 2) | 1 |
| Trunk (0, 2) | 1 (0, 1) | 1 (0, 2) | 2 |
| Walk stance (0, 1) | 1 (0, 1) | 1 (0, 1) | 2 |

| | | | |
|---|---|---|---|
| *Component score for each patient is determined as the median score of 3 raters. | | | |

Step length and continuity, steppage gait pattern, and marked trunk sway were among commonly observed gait deviations for the HCs, which persisted from first assessment to last assessment (Table 8).

**Table 8. Individual components of mPOMA-G scores observed by raters at First and Last Assessments of historical HPP controls.**

| **Patient** | **First Assessment** | **Last Assessment** |
|---|---|---|
| **1** | • Gait deviations in step length and continuity, and a steppage gait pattern | • Gait deviations in step length and continuity, and a steppage gait pattern |
| | • Widened stance and marked trunk sway | **• Narrower stance and mild trunk sway** |
| **2** | • Gait deviations in step length and continuity | • Gait deviations in step length and continuity |
| | • Steppage gait pattern | • Steppage gait pattern |
| | • Marked trunk sway | • Marked trunk sway |
| | • Widening of the base of support to increase stability | • Widening of the base of support to increase stability |
| | • Step length asymmetry (2/3 raters) | • **No step length asymmetry (3 raters)** |
| **3** | • Mild trunk sway or presence of compensatory arm patterns to compensate for proximal instability | • Mild trunk sway or presence of compensatory arm patterns to compensate for proximal instability |
| **4** | • Steppage gait pattern | • Steppage gait pattern |
| | • Mild trunk sway | • Mild trunk sway |
| | • Stepping just past the stationary foot bilaterally | **• Stepping past the stance foot by at least 1 foot length bilaterally** |
| **5** | • Gait deviations in step length and continuity | • Gait deviations in step length and continuity |
| | • Steppage gait pattern | • Steppage gait pattern |
| | • Marked trunk sway | • Marked trunk sway |
| | • Widening of the base of support | • **Heels intermittently widened or almost touching while walking** |
| **6** | • Steppage gait pattern | • Steppage gait pattern (2/3 raters) |
| | • Lack of step continuity (1/3 raters) | **• Normal step continuity (3 raters)** |
| | • Mild trunk sway (1/3 raters) | **• Absence of trunk sway (3 raters)** |

| | | |
|---|---|---|
| Bold text, an improvement has been observed from First to Last Assessment. | | |

These results indicate that proximal muscle weakness was prevalent and persistent in the HCs, as indicated by gait descriptors, functional mobility items, and mPOMA-G scores. All HCs had clinically significant gait impairment that persisted throughout the study.

### Example 7. Comparative gait analyses using mPOMA-G of HPP patients and historical HPP controls

Children and adolescent HPP patients treated with asfotase alfa demonstrated a statistically significant improvement in rate of change per year in mPOMA-G score from baseline to last assessment compared with HCs (Fig. 4 and Table 9). The median (range) rate of change per year in asfotase alfa-treated patients was 2.51 (0.0, 4.6) compared with 0.33 (0.0, 0.9) in HCs (p=0.0303, Wilcoxon rank sum test (exact method)). Rate of change using the POMA-G score was directionally the same as for the mPOMA-G score, with treated patients demonstrating a greater rate of change compared to the HCs.

**Table 9. Rate of change per year from baseline to last assessment of mPOMA-G score in asfotase alfa-treated versus historical HPP control patients with juvenile-onset HPP.**

| **Statistic** | **Juvenile-Onset Historical HPP control (N=6)** | **Juvenile-Onset Treated (N=8)** |
|---|---|---|
| **Baseline mPOMA-G** | | |
| N | | 8 |
| Mean (SD) | 6.3 (2.94) | 4.6 (2.33) |
| Median (min, max) | 6.0 (3, 11) | 4.0 (2, 9) |

| **mPOMA-G Rate of Change (per year)** | | |
|---|---|---|
| N | 6 | 8 |
| Mean (SD) | 0.37 (0.306) | 2.25 (1.672) |
| Median (min, max) | 0.33 (0.0, 0.9) | 2.51 (0.0, 4.6) |
| p-value (within group) ^{a} | 0.0625 | 0.0156 |
| p-value (between group) ^{b} | 0.0303 | |

| | | |
|---|---|---|
| Abbreviations: max = maximum, min = minimum, SD = standard deviation a Within group comparison of change from Baseline to Last Assessment based on Wilcoxon signed rank test. b Between group comparison of change from Baseline to Last Assessment based on Wilcoxon rank sum test (exact method). | | |

For HPP patients treated with asfotase alfa that had a pre-treatment video available, rate of change per year in mPOMA-G score was determined for the time period prior to and after receiving treatment with asfotase alfa. Deterioration in gait was observed prior to starting treatment with asfotase alfa (median rate of change/year of -0.72); whereas, the median on-treatment rate of change per year was 3.01 (0.0, 3.6) (Fig. 5 and Table 10). These results were consistent with the results of the comparison with HCs, with the treated group showing improvements in gait when compared with either historical or pre-treatment controls.

**Table 10: Rate of change per year in mPOMA-G score pre-treatment versus on-treatment in juvenile-onset HPP patients (N=5).**

| **Statistic** | **Pre-Treatment (N=5)** | **On-Treatment (N=5)** |
|---|---|---|
| **mPOMA-G Rate of Change (per year)** | | |
| N | 5 | 5 |
| Mean (SD) | -0.67 (0.401) | 2.14 (1.669) |
| Median (min, max) | -0.72 (-1.0, 0.0) | 3.01 (0.0, 3.6) |
| p-value | 0.1250 | 0.1250 |

| | | |
|---|---|---|
| Abbreviations: max = maximum, min = minimum, SD = standard deviation Within group comparison of change from Baseline to Last Assessment based on Wilcoxon signed rank test. | | |

Patients treated with asfotase alfa had an absolute median per patient change from baseline in mPOMA-G score that was twice as great as HCs (median change of 3.0 versus 1.5, respectively; Fig. 6 and Table 11). While both groups exceeded the pre-identified mPOMA-G minimally clinical important difference (MCID) of 0.6, patients treated with asfotase alfa had a median improvement in score twice as great as the HCs.

**Table 11: Change from baseline to last assessment of mPOMA-G score for asfotase alfa-treated versus historical HPP controls**

| **Statistic** | **Juvenile-Onset Historical HPP control (N=6)** | | **Juvenile-Onset Treated (N=8)** | |
|---|---|---|---|---|
| | **Actual Score** | **Change from BL** | **Actual Score** | **Change from BL** |
| **Baseline** | | | | |
| N | 6 | - | 8 | - |
| Mean (SD) | 6.3 (2.94) | - | 4.6 (2.33) | - |
| Median (min, max) | 6.0 (3, 11) | - | 4.0 (2, 9) | - |

| **Last Assessment** | | | | |
|---|---|---|---|---|
| N | 6 | 6 | 8 | 8 |
| Mean (SD) | 7.7 (2.73) | 1.3 (0.82) | 7.9 (2.17) | 3.3 (2.60) |
| Median (min, max) | 7.5 (4, 11) | 1.5 (0, 2) | 9.0 (4, 10) | 3.0 (0, 7) |
| p-value (within group)^{a} | 0.0625 | | 0.0156 | |
| p-value (between group) ^{b} | 0.2561 | | | |

| | | | | |
|---|---|---|---|---|
| ^{a} Within group comparison of change from Baseline to Last Assessment based on Wilcoxon signed rank test. ^{b} Between group comparison of change from Baseline to Last Assessment based on Wilcoxon rank sum test (exact method) Abbreviations: BL = Baseline: max = maximum, min = minimum, SD = standard deviation | | | | |

The most common deficits in gait observed at baseline for treated patients and HCs (foot clearance, stance, proximal sway/trunk, and step length) implicate lack of proximal muscle strength as a complication of HPP, which results in compensatory gait abnormalities (e.g., waddling gait pattern and increased base of support). Following asfotase alfa treatment, components of the gait analysis showing the greatest incidence of improvement were step length and stance (Table 12).

**Table 12. Incidence of the separate components of mPOMA-G prior to and following treatment of HPP patients with asfotase alfa.**

| Gait Deficits (mPOMA-G) | Baseline Historical Controls (n=6) n (%) | Treated Patients (n=8) n (%) | |
|---|---|---|---|
| | | Baseline | Post-Asfotase Alfa |
| Reduced step length (swing) | 4 (66.7) | 7 (87.5) | 1 (12.5) |
| Steppage (foot clearance) | 5 (83.3) | 8 (100.0) | 7 (87.5) |
| Widened stance | 2 (33.3) | 6 (75.0) | 2 (25) |

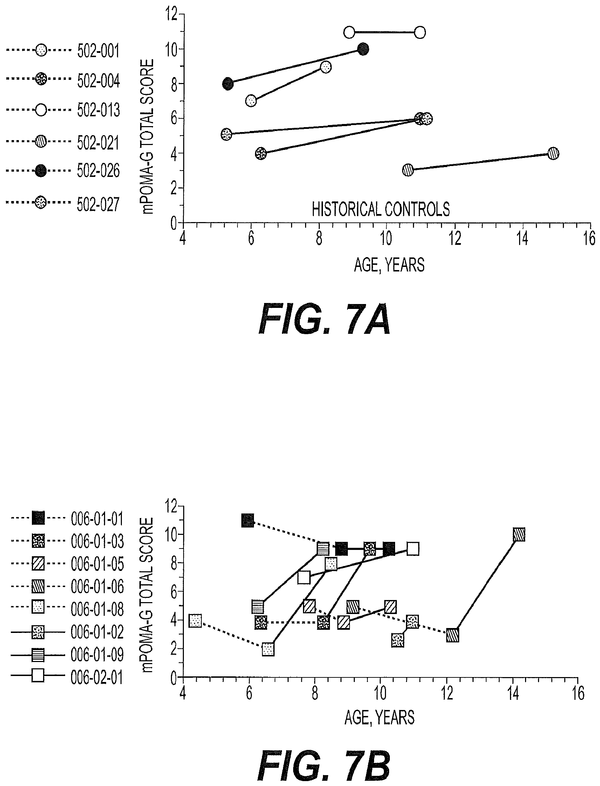

Baseline to last assessment for each patient at the two time points was also compared relative to patient age (Figs. 7A-7B). The range of ages was similar for both the asfotase alfa-treated and historical HPP control patients with the minimum and maximum ages at baseline ranging from 6 to 12 years in treated patients compared with 5 to 11 years in HCs and at last assessment ranging from 8 to 14 years for asfotase alfa-treated patients compared to 8 to 15 years for historical HPP control patients. Neither age at baseline nor age at last assessment was significantly different between the two groups.

Importantly, comparison of individual changes in mPOMA-G from baseline supports the conclusion that treatment with asfotase alfa improves performance-oriented mobility in child and adolescent patients with juvenile-onset HPP. As shown in Figs. 7A-7B, one patient in the historical HPP control group and one patient in the asfotase-alfa treated group maintained the same score at baseline and last assessment. Changes from baseline in the remaining five historical HPP control patients were relatively small, with two patients increasing by 1 point, 3 patients increasing by 2 points, and no patients experiencing improvements greater than 2 points, for a median change from baseline of 1.5 points. In contrast, for the remaining seven (88%) asfotase alfa-treated patients, two patients improved from baseline to last assessment by 1 point, two patients improved by 4 points, one patient improved by 5 points, and two patients improved by 6 points, for a median change from baseline of 3.0 points. POMA-G results were consistent in trend but were of less magnitude than those from the HPP-specific mPOMA-G.

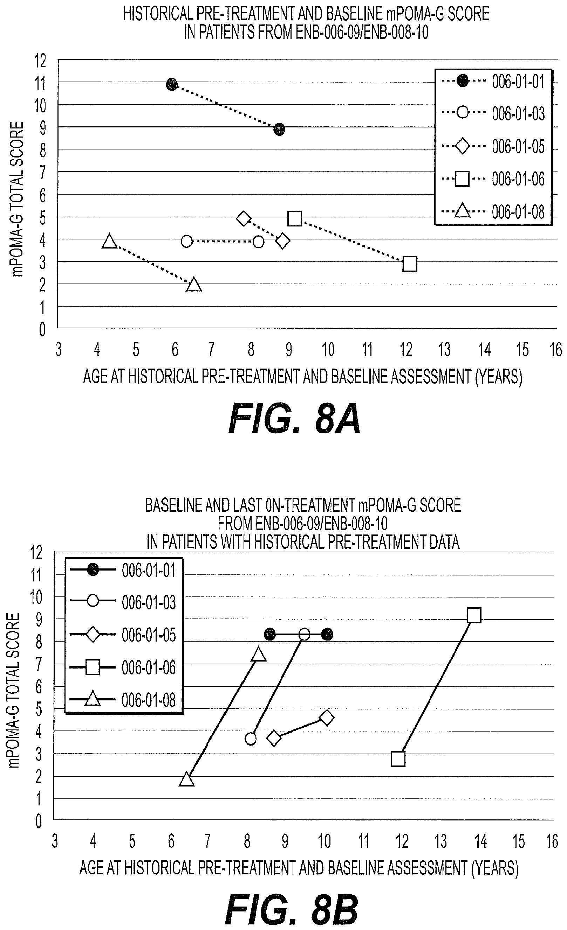

In contrast to the historical HPP control data, where five out of six HCs demonstrated some improvement in gait over time, four out of five asfotase alfa-treated patients with pre-treatment gait data demonstrated increased disability prior to treatment (Fig. 8A). Prior to administration of asfotase alfa, the median change in mPOMA-G score was -2.0 (range: -2, 0); whereas, after starting treatment the median improvement was 5.0 (range: 0, 7) at the last assessment (Fig. 8B and Table 13). Both the median pre-treatment deterioration and the on-treatment improvement in gait markedly exceeded the pre-identified MCID of 0.6.

**Table 13: Change in mPOMA-G score pre-treatment versus on-treatment in asfotase alfa-treated patients.**

| **Statistic** | **Pre-Treatment (N=5)** | | **On-Treatment (N=5)** | |
|---|---|---|---|---|
| | **Actual Score** | **Change from BL** | **Actual Score** | **Change from BL** |
| n | **Earliest Historical Result** | | **Treatment Baseline** | |
| | 5 | - | 5 | - |
| Mean (SD) | 5.8 (2.95) | - | 4.4 (2.70) | - |
| Median (min, max) | 5.0 (4, 11) | - | 4.0 (2, 9) | - |
| n | **Treatment Baseline** | | **Treatment Last Assessment** | |
| | 5 | 5 | 5 | 5 |
| Mean (SD) | 4.4 (2.70) | -1.4 (0.89) | 8.2 (1.92) | 3.8 (3.11) |
| Median (min, max) | 4.0 (2, 9) | -2.0 (-2, 0) | 9.0 (5, 10) | 5.0 (0,7) |
| p-value (within group) ^{a} | 0.1250 | | 0.1250 | |

| | | | | |
|---|---|---|---|---|
| ^{a} Within group comparison of change from Baseline to Last Assessment based on Wilcoxon signed rank test. Abbreviations: BL = Baseline; max = maximum, min = minimum, SD = standard deviation | | | | |

Regardless of age at the earliest historical assessment, four (80.0%) asfotase-alfa treated patients exhibited increased gait disability prior to treatment and one patient remained stable. Additionally, regardless of age at treatment-onset, four (80.0%) asfotase-alfa treated patients demonstrated improved gait after treatment with asfotase alfa and one patient remained stable. In addition, the improvement observed in gait score exceeded the MCID for all four asfotase-alfa treated patients.

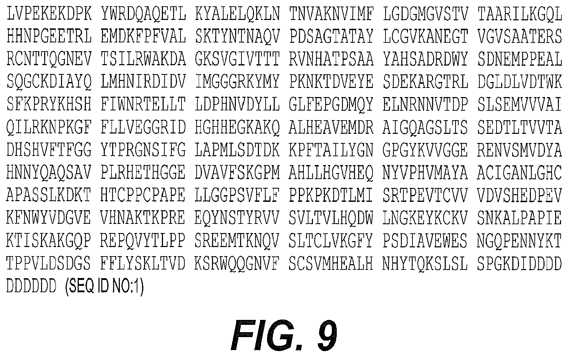

These data show that the mPOMA-G analysis and score accurately quantify both deficits and changes in gait in children and adolescents with HPP. Moreover, children and adolescents with impaired gait due to HPP demonstrated clinically significant improvement in functional mobility when treated with asfotase alfa compared to these HPP patients prior to treatment with asfotase alfa and HCs. Thus, asfotase alfa (SEQ ID NO: 1; Fig. 9) can be used effectively to treat HPP, its symptoms, and decreased physical function associated therewith in patients of about 5 to about 15 years of age for an extended treatment period, such as at least one year, at least two years, at least three years, at least four years, at least five years, at least six years, at least seven years, at least eight years, at least nine years, at least ten years, or longer than ten years, such as for the lifetime of the patient.

### Example 8. Optimization of asfotase alfa dosage to treat gait impairments in HPP patients

The dosage of asfotase alfa (SEQ ID NO: 1) can be increased to, e.g., 9 mg/kg/wk, when a child or adolescent with HPP has an average mPOMA-G score of less than about 12 after administration of asfotase alfa at a dosage of 6 mg/kg/wk for a treatment period of at least one year. For instance, the average mPOMA-G score of the child or adolescent will increase to about 8 to about 11 (e.g., 8, 9, 10, or 11) after being treated with an sALP at a dosage of 6 mg/kg/wk for a treatment period of at least one year. The sALP can then be administered at a dosage of, e.g., 9 mg/kg/wk until the child or adolescent exhibits further improvements in gait, as determining using the mPOMA-G analysis and score. In particular, the average mPOMA-G score of the child or adolescent can increase from about 8 to about 12 after administration of an sALP at a dosage of 9 mg/kg/wk for a treatment period of at least one year, such as at least two years, at least three years, at least four years, at least five years, at least six years, at least seven years, at least eight years, at least nine years, at least ten years, or longer than ten years (e.g., the lifetime of the patient).

## Claims

1. A composition comprising a soluble alkaline phosphatase (sALP) comprising an amino acid sequence having at least 95% sequence identity to the amino acid sequence of SEQ ID NO: 1 for use in treating hypophosphatasia (HPP) in a subject of 5 to 15 years of age having an average modified Performance-Oriented Mobility Assessment - Gait (mPOMA-G) score of 5 or less, wherein said subject did not exhibit an increase in the average mPOMA-G score of at least 0.6, particularly at least 1.0 or more, after administration of said sALP to the subject at a dose providing 6 mg/kg/week during a treatment period of at least one year, and wherein the dosage of the sALP is increased to 9 mg/kg/week.

2. The composition for use according to claim 1, wherein at least one of:
(a) the average mPOMA-G score of the subject is determined relative to an average mPOMA-G score of an untreated subject of 5 to 15 years of age having HPP;
(b) the average mPOMA-G score of the subject is determined relative to an average mPOMA-G score of a healthy subject of 5 to 15 years of age; and
(c) said use further comprises performing an mPOMA-G analysis.

3. The composition for use according to claim 2, wherein at least one of:
(a) the mPOMA-G analysis is performed daily, one or more times per week, weekly, one or more times per month, monthly, every six months, one or more times per year, yearly, every two years, or every three years; and
(b) the mPOMA-G analysis comprises one or more gait assessments selected from the group consisting of trunk sway, walking stance, step length and height, step symmetry, and step continuity.

4. The composition for use according to claim 3, wherein at least one of:
(a) trunk sway comprises measuring at least one of marked sway, use of walking aids, arm abduction, trunk flexion, and excessive knee flexion;
(b) walking stance comprises measuring distance of heels;
(c) step length and height comprise measuring right swing foot, right foot clear, left swing foot, and left foot clear;
(d) step symmetry comprises measuring right step length and left step length and comparing right step length to left step length; and
(e) step continuity comprises measuring stopping between steps or discontinuity between steps, wherein preferably discontinuity between steps is measured through evaluation of heel off in terminal stance on one foot at the same time as initial contact of heel strike on the opposite foot.

5. The composition for use according to any one of claims 1 to 4, wherein at least one of:
(a) the treatment period is at least two years, at least three years, at least four years, at least five years, at least six years, at least seven years, at least eight years, at least nine years, at least ten years, or longer; and
(b) the average mPOMA-G score of the subject increases to 7.5, 8, 8.5, 9, 9.5, 10, 10.5, 11, 11.5, or 12 after administration of the composition.

6. The composition for use according to claim 5, wherein at least one of:
(a) prior to administration of the composition the subject exhibits one or more gait impairments selected from the group consisting of reduced step length, reduced step continuity, reduced foot clearance, foot clearance that exceeds 1 or 2 inches off surface, and widened stance; and
(b) after administration of the composition the subject exhibits an improvement in one or more gait impairments selected from the group consisting of reduced step length, reduced step continuity, reduced foot clearance, foot clearance that exceeds 1 or 2 inches off surface, and widened stance.

7. The composition for use according to any one of claims 1 to 6, wherein at least one of:
(a) an increase in the average mPOMA-G score is sustained throughout a period during which the subject is treated with the composition;
(b) a rate of change per year of the average mPOMA-G score during which the subject is treated with the composition is 2.5;
(c) the subject has juvenile-onset HPP;
(d) the use further comprises performing at least one of a Six Minute Walk Test (6MWT), a Child Health Assessment Questionnaire (CHAQ), and a Pediatric Outcomes Data Collection Instrument (PODCI);
(e) the subject exhibits an increase in activities of daily living (ADL) after administration of the composition; and
(f) the subject exhibits an improvement in walking ability after administration of the composition.

8. The composition for use according to claim 7, wherein at least one of:
(a) the increase in ADL is determined from a CHAQ disability index score or PODCI transfer and mobility scale score of the subject; and
(b) the improvement in walking ability is determined from a 6MWT distance of the subject.

9. The composition for use according to any one of claims 1 to 8, wherein at least one of:
(a) the composition is administered daily or weekly;
(b) the composition is administered twice a week, three times a week, four times a week, five times a week, six times a week, or seven times a week;
(c) the composition is administered at a sALP dosage of 3 mg/kg three times a week;
(d) the composition is administered on consecutive or alternating days;
(e) the sALP comprises or consists of the amino acid sequence of SEQ ID NO: 1;
(f) the composition is administered as a pharmaceutical composition, with at least one pharmaceutically acceptable carrier; and
(g) the composition is administered by at least one of subcutaneous, intramuscular, intravenous, oral, nasal, sublingual, intrathecal, and intradermal routes.

10. The composition for use according to any one of claims 1 to 9, wherein the subject exhibits one or more symptoms of HPP selected from the group consisting of gait disturbance, bone deformity, joint pain, bone pain, bone fracture, muscle weakness, muscle pain, rickets, premature loss of deciduous teeth, incomplete bone mineralization, elevated blood and/or urine levels of phosphoethanolamine (PEA), elevated blood and/or urine levels of inorganic pyrophosphate (PPi), elevated blood and/or urine levels of pyridoxal 5'-phosphate (PLP), hypomineralization, rachitic ribs, hypercalciuria, short stature, HPP-related seizure, inadequate weight gain, craniosynostosis, and calcium pyrophosphate dihydrate crystal deposition.

11. The composition for use according to claim 10, wherein the subject exhibits an improvement in the one or more symptoms of HPP after administration of the composition.

12. The composition for use according to any one of claims 9 to 11, wherein the at least one pharmaceutically acceptable carrier at least one of:
(a) is saline; and
(b) comprises sodium chloride and sodium phosphate, wherein, optionally, the pharmaceutically acceptable carrier comprises 150 mM sodium chloride and 25 mM sodium phosphate.

13. The composition for use according to any one of claims 1 to 12, wherein at least one of:
(a) the sALP is physiologically active toward PEA, PPi, and PLP;
(b) the sALP is catalytically competent to improve skeletal mineralization in bone;
(c) the sALP is the soluble extracellular domain of an alkaline phosphatase;
(d) the use further comprises determining sALP activity in a serum and/or blood sample from the subject of 5 to 15 years of age;
(e) administration of the composition results in an increase in the average mPOMA-G score of at least 2.5 or more;
(f) the mPOMA-G analysis is performed before administration of the composition; and
(g) the mPOMA-G analysis is performed after administration of the composition.

14. The composition for use according to claim 13, wherein the determination of sALP activity comprises measuring at least one of phosphoethanolamine (PEA), inorganic pyrophosphate (PPi), and pyridoxal 5'-phosphate (PLP) in at least one of serum and blood from the subject of 5 to 15 years of age.

15. The composition for use according to any one of claims 1 to 14, wherein the subject exhibits decreased reliance on an assistive device for mobility after administration of the composition, wherein, optionally, the assistive device for mobility is selected from the group consisting of a wheelchair, braces, crutches, and orthotics.

## Patentansprüche

1. Zusammensetzung, umfassend eine lösliche alkalische Phosphatase (sALP), die eine Aminosäuresequenz mit wenigstens 95 % Sequenzidentität mit der Aminosäuresequenz von SEQ ID NO:1 umfasst, zur Verwendung bei der Behandlung von Hypophosphatasie (HPP) bei einem Subjekt mit einem Alter von 5 bis 15 Jahren mit einem mittleren modifizierten "Performance Oriented Mobility Assessment - Gait (mPOMA-G) Score" von 5 oder weniger, wobei das Subjekt nach Verabreichung der sALP an das Subjekt mit einer Dosis, die 6 mg/kg/Woche bereitstellt, während einer Behandlungsdauer von wenigstens einem Jahr keine Verbesserung des mittleren mPOMA-G-Scores von wenigstens 0,6, insbesondere wenigstens 1,0 oder mehr, zeigte, und wobei die Dosierung der sALP auf 9 mg/kg/Woche erhöht wird.

2. Zusammensetzung zur Verwendung gemäß Anspruch 1, wobei wenigstens eines von:
(a) der mittlere mPOMA-G-Score des Subjekts relativ zu einem mittleren mPOMA-G-Score eines unbehandelten Subjekts mit einem Alter von 5 bis 15 Jahren, das HPP aufweist, bestimmt wird;
(b) der mittlere mPOMA-G-Score des Subjekts relativ zu einem mittleren mPOMA-G-Score eines gesunden Subjekts mit einem Alter von 5 bis 15 Jahren bestimmt wird; und
(c) die Verwendung ferner Durchführen einer mPOMA-G-Analyse umfasst.

3. Zusammensetzung zur Verwendung gemäß Anspruch 2, wobei wenigstens eines von:
(a) die mPOMA-G-Analyse täglich, ein- oder mehrmals pro Woche, wöchentlich, ein- oder mehrmals pro Monat, monatlich, alle sechs Monate, ein- oder mehrmals pro Jahr, jährlich, alle zwei Jahre oder alle drei Jahre durchgeführt wird; und
(b) die mPOMA-G-Analyse eine oder mehrere Prüfungen des Gehvermögens ausgewählt aus der Gruppe bestehend aus Rumpfschwanken, Gehhaltung, Schrittlänge und -höhe, Schrittsymmetrie und Schrittkontinuität umfasst.

4. Zusammensetzung zur Verwendung gemäß Anspruch 3, wobei wenigstens eines von:
(a) Rumpfschwanken Messen von wenigstens einem von ausgeprägtem Schwanken, Verwendung von Gehhilfen, Armabduktion, Rumpfbeugung und übermäßige Kniebeugung umfasst;
(b) Gehhaltung Messen des Abstands der Fersen umfasst;
(c) Schrittlänge und -höhe Messen von Fußschwingbewegung rechts, Fuß-Boden-Abstand rechts, Fußschwingbewegung links, Fuß-Boden-Abstand links umfasst;
(d) Schrittsymmetrie Messen der rechten Schrittlänge und der linken Schrittlänge und Vergleichen der rechten Schrittlänge mit der linken Schrittlänge umfasst; und
(e) Schrittkontinuität Messen von Unterbrechung zwischen Schritten oder Diskontinuität zwischen Schritten umfasst, wobei vorzugsweise Diskontinuität zwischen Schritten durch Auswertung der Fersenhebung in der Endstellung eines Fußes gleichzeitig mit dem ersten Kontakt des Fersenaufsetzens des anderen Fußes gemessen wird.

5. Zusammensetzung zur Verwendung gemäß einem der Ansprüche 1 bis 4, wobei wenigstens eines von:
(a) der Behandlungszeitraum wenigstens zwei Jahre, wenigstens drei Jahre, wenigstens vier Jahre, wenigstens fünf Jahre, wenigstens sechs Jahre, wenigstens sieben Jahre, wenigstens acht Jahre, wenigstens neun Jahre, wenigstens zehn Jahre oder länger beträgt; und
(b) der mittlere mPOMA-G-Score des Subjekts nach Verabreichung der Zusammensetzung auf 7,5, 8, 8,5, 9, 9,5, 10, 10,5, 11, 11,5 oder 12 zunimmt.

6. Zusammensetzung zur Verwendung gemäß Anspruch 5, wobei wenigstens eines von:
(a) das Subjekt vor Verabreichung der Zusammensetzung eine oder mehrere Gehbeeinträchtigungen ausgewählt aus der Gruppe bestehend aus verringerter Schrittlänge, verringerter Schrittkontinuität, verringertem Fuß-Boden-Abstand, Fuß-Boden-Abstand, der 1 oder 2 Zoll über der Oberfläche übersteigt, und verbreiterter Haltung zeigt; und
(b) das Subjekt nach Verabreichung der Zusammensetzung eine Verbesserung einer oder mehrerer Gehbeeinträchtigungen ausgewählt aus der Gruppe bestehend aus verringerter Schrittlänge, verringerter Schrittkontinuität, verringertem Fuß-Boden-Abstand, Fuß-Boden-Abstand, der 1 oder 2 Zoll über der Oberfläche übersteigt, und verbreiterter Haltung zeigt.

7. Zusammensetzung zur Verwendung gemäß einem der Ansprüche 1 bis 6, wobei wenigstens eines von:
(a) eine Verbesserung des mittleren mPOMA-G-Scores über einen Zeitraum aufrecht erhalten bleibt, während dessen das Subjekt mit der Zusammensetzung behandelt wird;
(b) eine Veränderungsrate des mittleren mPOMA-G-Scores pro Jahr, während dessen das Subjekt mit der Zusammensetzung behandelt wird, 2,5 beträgt;
(c) das Subjekt HPP mit Ausbruch im Jugendalter aufweist;
(d) die Verwendung ferner die Durchführung von wenigstens einem von einem Sechs-Minuten-Gehtest (6MWT), einem "Child Health Assessment Questionnaire" (CHAQ) und einem "Pedriatric Outcomes Data Collection Instrument" (PODCI) umfasst;
(e) das Subjekt eine Zunahme der Aktivität des täglichen Lebens (ADL) nach Verabreichung der Zusammensetzung zeigt; und
(f) das Subjekt eine Verbesserung der Gehfähigkeit nach Verabreichung der Zusammensetzung zeigt.

8. Zusammensetzung zur Verwendung gemäß Anspruch 7, wobei wenigstens eines von:
(a) die Zunahme der ADL aus einem "CHAQ Disability Index Score" oder "PODCI Transfer and Mobility Scale Score" des Subjekts bestimmt wird;
(b) die Verbesserung der Gehfähigkeit aus einer 6MWT-Distanz des Subjekts bestimmt wird.

9. Zusammensetzung zur Verwendung gemäß einem der Ansprüche 1 bis 8, wobei wenigstens eines von:
(a) die Zusammensetzung täglich oder wöchentlich verabreicht wird;
(b) die Zusammensetzung zweimal pro Woche, dreimal pro Woche, viermal pro Woche, fünfmal pro Woche, sechsmal pro Woche oder siebenmal pro Woche verabreicht wird;
(c) die Zusammensetzung als sALP-Dosierung von 3 mg/kg dreimal pro Woche verabreicht wird;
(d) die Zusammensetzung an aufeinanderfolgenden oder alternierenden Tagen verabreicht wird;
(e) die sALP die Aminosäuresequenz von SEQ ID NO:1 umfasst oder daraus besteht;
(f) die Zusammensetzung als pharmazeutische Zusammensetzung mit wenigstens einem pharmazeutisch verträglichen Träger verabreicht wird; und
(g) die Zusammensetzung über wenigstens einen von einem subkutanen, intramuskulären, intravenösen, oralen, nasalen, sublingualen, intrathekalen und intradermalen Weg verabreicht wird.

10. Zusammensetzung zur Verwendung gemäß einem der Ansprüche 1 bis 9, wobei das Subjekt ein oder mehrere Symptome von HPP ausgewählt aus der Gruppe bestehend aus Gehstörung, Knochendeformation, Gelenkschmerz, Knochenschmerz, Knochenfraktur, Muskelschwäche, Muskelschmerz, Rachitis, frühzeitigem Verlust von Milchzähnen, unvollständiger Knochenmineralisierung, erhöhten Blut- und/oder Urinspiegeln von Phosphoethanolamin (PEA), erhöhten Blut- und/oder Urinspiegeln von anorganischem Pyrophosphat (PPi), erhöhten Blut- und/oder Urinspiegeln von Pyridoxal-5'-phosphat (PLP), Hypomineralisierung, rachitischen Rippen, Hypercalciurie, Kleinwuchs, mit HPP verbundenen Krampfanfällen, unzureichender Gewichtszunahme, Craniosynostosis und Calciumpyrophosphatdihydrat-Kristallabscheidung.

11. Zusammensetzung zur Verwendung gemäß Anspruch 10, wobei das Subjekt eine Verbesserung des einen oder der mehreren Symptome von HPP nach Verabreichung der Zusammensetzung zeigt.

12. Zusammensetzung zur Verwendung gemäß einem der Ansprüche 9 bis 11, wobei der wenigstens eine pharmazeutisch verträgliche Träger wenigstens eines von:
(a) Kochsalzlösung ist; und
(b) Natriumchlorid und Natriumphosphat umfasst, wobei gegebenenfalls der pharmazeutisch verträgliche Träger 150 mM Natriumchlorid und 25 mM Natriumphosphat umfasst.

13. Zusammensetzung zur Verwendung gemäß einem der Ansprüche 1 bis 12, wobei wenigstens eines von:
(a) die sALP physiologisch aktiv für PEA, PPi und PLP ist;
(b) die sALP katalytisch kompetent zum Verbessern der Skelettmineralisierung von Knochen ist;
(c) die sALP die lösliche extrazelluläre Domäne einer alkalischen Phosphatase ist;
(d) die Verwendung ferner Bestimmen der sALP-Aktivität in einer Serum- und/oder Blutprobe des Subjekts mit einem Alter von 5 bis 15 Jahren umfasst;
(e) Verabreichung der Zusammensetzung zu einer Zunahme des mittleren mPOMA-G-Scores von wenigstens 2,5 oder mehr führt;
(f) die mPOMA-G-Analyse vor Verabreichung der Zusammensetzung durchgeführt wird; und
(g) die mPOMA-G-Analyse nach Verabreichung der Zusammensetzung durchgeführt wird.

14. Zusammensetzung zur Verwendung gemäß Anspruch 13, wobei die Bestimmung der sALP-Aktivität Messen von wenigstens einem von Phosphoethanolamin (PEA), anorganischem Pyrophosphat (PPi) und Pyridoxal-5'-phosphat (PLP) in wenigstens einem von Serum und Blut des Subjekts mit einem Alter von 5 bis 15 Jahren umfasst.

15. Zusammensetzung zur Verwendung gemäß einem der Ansprüche 1 bis 14, wobei das Subjekt nach Verabreichung der Zusammensetzung einen verringerten Bedarf an einer Hilfsvorrichtung für die Mobilität zeigt, wobei die Hilfsvorrichtung für die Mobilität gegebenenfalls ausgewählt ist aus der Gruppe bestehend aus einem Rollstuhl, Stützen, Krücken und Orthesen.

## Revendications

1. Composition comprenant une phosphatase alcaline soluble (sALP) comprenant une séquence d'acides aminés ayant au moins 95 % d'identité de séquence avec la séquence d'acides aminés de SEQ ID NO : 1 pour utilisation dans le traitement de l'hypophosphatasie (HPP) chez un sujet âgé de 5 à 15 ans ayant un score d'évaluation de la mobilité axée sur la performance - démarche (mPOMA-G) modifié moyen de 5 ou moins, où ledit sujet ne présente pas une augmentation du score mPOMA-G moyen d'au moins 0,6, en particulier d'au moins 1,0 ou plus, après l'administration de ladite sALP au sujet à une dose fournissant 6 mg/kg/semaine pendant une période de traitement d'au moins un an, et où la dose de la sALP est augmentée à 9 mg/kg/semaine.

2. Composition pour utilisation selon la revendication 1, présentant au moins l'une des caractéristiques suivantes :
(a) le score mPOMA-G moyen du sujet est déterminé par rapport à un score mPOMA-G moyen d'un sujet non traité âgé de 5 à 15 ans atteint de HPP ;
(b) le score mPOMA-G moyen du sujet est déterminé par rapport à un score mPOMA-G moyen d'un sujet sain âgé de 5 à 15 ans ; et
(c) ladite utilisation comprend en outre la conduite d'une analyse mPOMA-G.

3. Composition pour utilisation selon la revendication 2, présentant au moins l'une des caractéristiques suivantes :
(a) l'analyse mPOMA-G est effectuée chaque jour, une ou plusieurs fois par semaine, chaque semaine, une ou plusieurs fois par mois, chaque mois, tous les six mois, une ou plusieurs fois par an, chaque année, tous les deux ans, ou tous les trois ans ; et
(b) l'analyse mPOMA-G comprend une ou plusieurs évaluations de démarche choisies dans le groupe constitué du balancement du tronc, de la position de marche, de la longueur et la hauteur de pas, de la symétrie des pas et de la continuité des pas.

4. Composition pour utilisation selon la revendication 3, présentant au moins l'une des caractéristiques suivantes :
(a) le balancement du tronc comprend la mesure d'au moins l'un parmi un balancement marqué, l'utilisation de dispositifs d'aide à la marche, l'abduction des bras, la flexion du tronc, et une flexion excessive des genoux ;
(b) la position de marche comprend la mesure de distance des talons ;
(c) la longueur et la hauteur de pas comprennent la mesure du balancement du pied droit, du dégagement du pied droit, du balancement du pied gauche et du dégagement du pied gauche ;
(d) la symétrie des pas comprend la mesure de longueur de pas droit et de longueur de pas gauche et la comparaison de la longueur de pas droit à la longueur de pas gauche ; et
(e) la continuité des pas comprend la mesure de l'arrêt entre les pas ou la discontinuité entre les pas, où, de préférence, la discontinuité entre les pas est mesurée par l'évaluation du décollement du talon dans la position terminale sur un pied en même temps que le contact initial d'attaque du talon sur le côté opposé.

5. Composition pour utilisation selon l'une quelconque des revendications 1 à 4, présentant au moins l'une des caractéristiques suivantes :
(a) la période de traitement est au moins deux ans, au moins trois ans, au moins quatre ans, au moins cinq ans, au moins six ans, au moins sept ans, au moins huit ans, au moins neuf ans, au moins dix ans, ou plus longtemps ; et
(b) le score mPOMA-G moyen du sujet augmente à 7,5, 8, 8,5, 9, 9,5, 10, 10,5, 11, 11,5, ou 12 après l'administration de la composition.

6. Composition pour utilisation selon la revendication 5, présentant au moins l'une des caractéristiques suivantes :
(a) avant l'administration de la composition, le sujet présente une ou plusieurs altérations de la marche choisies dans le groupe constitué de : une longueur de pas réduite, une continuité de pas réduite, un dégagement du pied réduit, un dégagement du pied qui dépasse 1 ou 2 pouces depuis la surface, et une position des pieds élargie ; et
(b) après l'administration de la composition le sujet présente une amélioration d'une ou plusieurs altérations de la marche choisies dans le groupe constitué de : une longueur de pas réduite, une continuité de pas réduite, un dégagement du pied réduit, un dégagement du pied qui dépasse 1 ou 2 pouces depuis la surface, et une position des pieds élargie.

7. Composition pour utilisation selon l'une quelconque des revendications 1 à 6, présentant au moins l'une des caractéristiques suivantes :
(a) une augmentation du score mPOMA-G moyen est prolongée tout au long d'une période pendant laquelle le sujet est traité avec la composition ;
(b) un taux de changement par an du score mPOMA-G moyen pendant lequel le sujet est traité avec la composition est 2,5 ;
(c) le sujet présente une HPP juvénile ;
(d) l'utilisation comprend en outre la conduite d'au moins l'un parmi un test de marche de six minutes (6MWT), un questionnaire d'évaluation de la santé des enfants (CHAQ), et un instrument de collecte de données de résultats pédiatrique (PODCI) ;
(e) le sujet présente une augmentation des activités de la vie quotidienne (ADL) après l'administration de la composition ; et
(f) le sujet présente une amélioration de la capacité de marche après l'administration de la composition.

8. Composition pour utilisation selon la revendication 7, présentant au moins l'une des caractéristiques suivantes :
(a) l'augmentation d'ADL est déterminée à partir d'un score d'indice d'incapacité CHAQ ou d'un score d'échelle de transfert et de mobilité PODCI du sujet ; et
(b) l'amélioration de la capacité de marche est déterminée à partir d'une distance 6MWT du sujet.

9. Composition pour utilisation selon l'une quelconque des revendications 1 à 8, présentant au moins l'une des caractéristiques suivantes :
(a) la composition est administrée chaque jour ou chaque semaine ;
(b) la composition est administrée deux fois par semaine, trois fois par semaine, quatre fois par semaine, cinq fois par semaine, six fois par semaine, ou sept fois par semaine ;
(c) la composition est administrée à une dose de sALP de 3 mg/kg trois fois par semaine ;
(d) la composition est administrée à des jours consécutifs ou alternés ;
(e) la sALP comprend ou est constituée de la séquence d'acides aminés de SEQ ID NO : 1 ;
(f) la composition est administrée sous la forme d'une composition pharmaceutique, avec au moins un véhicule pharmaceutiquement acceptable ; et
(g) la composition est administrée par au moins l'une des voies sous-cutanée, intramusculaire, intraveineuse, orale, nasale, sublinguale, intrathécale et intradermique.

10. Composition pour utilisation selon l'une quelconque des revendications 1 à 9, où le sujet présente un ou plusieurs symptômes de HPP choisis dans le groupe constitué de : un trouble de la démarche, une difformité osseuse, une douleur articulaire, une douleur osseuse, une fracture osseuse, une faiblesse musculaire, une douleur musculaire, le rachitisme, la chute prématurée des dents temporaires, une minéralisation osseuse incomplète, des taux sanguins et/ou urinaires élevés de phosphoéthanolamine (PEA), des taux sanguins et/ou urinaires élevés de pyrophosphate inorganique (PPi), des taux sanguins et/ou urinaires élevés de pyridoxal-5'-phosphate (PLP), une hypominéralisation, des côtes rachitiques, une hypercalciurie, une petite taille, des crises épileptiques liées à l'HPP, un gain de poids insuffisant, une craniosynostose et un dépôt de cristaux de pyrophosphate de calcium dihydraté.

11. Composition pour utilisation selon la revendication 10, où le sujet présente une amélioration des un ou plusieurs symptômes de HPP après l'administration de la composition.

12. Composition pour utilisation selon l'une quelconque des revendications 9 à 11, dans laquelle l'au moins un véhicule pharmaceutiquement acceptable présente au moins une des caractéristiques suivantes :
(a) est salin ; et
(b) comprend du chlorure de sodium et du phosphate de sodium, dans laquelle, facultativement, le véhicule pharmaceutiquement acceptable comprend du chlorure de sodium 150 mM et du phosphate de sodium 25 mM.

13. Composition pour utilisation selon l'une quelconque des revendications 1 à 12, présentant au moins l'une des caractéristiques suivantes :
(a) la sALP est physiologiquement active sur PEA, PPi, et PLP ;
(b) la sALP est catalytiquement compétente pour améliorer la minéralisation squelettique dans les os ;
(c) la sALP est le domaine extracellulaire soluble d'une phosphatase alcaline ;
(d) l'utilisation comprend en outre la détermination de l'activité sALP dans un échantillon de sérum et/ou de sang provenant du sujet âgé de 5 à 15 ans ;
(e) l'administration de la composition conduit à une augmentation du score mPOMA-G moyen d'au moins 2,5 ou plus ;
(f) l'analyse mPOMA-G est effectuée avant l'administration de la composition ; et
(g) l'analyse mPOMA-G est effectuée après l'administration de la composition.

14. Composition pour utilisation selon la revendication 13, où la détermination de l'activité sALP comprend la mesure d'au moins l'un parmi la phosphoéthanolamine (PEA), le pyrophosphate inorganique (PPi), et le pyridoxal-5'-phosphate (PLP) dans au moins l'un parmi le sérum et le sang du sujet âgé de 5 à 15 ans.

15. Composition pour utilisation selon l'une quelconque des revendications 1 à 14, où le sujet présente une dépendance réduite vis-à-vis d'un dispositif d'assistance pour la mobilité après l'administration de la composition, où, facultativement, le dispositif d'assistance pour la mobilité est choisi dans le groupe constitué d'une chaise roulante, d'appareils orthopédiques, de béquilles et d'orthèses.


### SEQUENCE LISTING

<110> Alexion Pharmaceuticals, Inc.
<120> METHODS FOR TREATING HYPOPHOSPHATASIA IN CHILDREN AND ADOLESCENTS
<130> 50694-061WO1
<160> 20
<170> PatentIn version 3.5
<210> 1
   <211> 726
   <212> PRT
   <213> Artificial Sequence
<220>
   <223> Synthetic Construct
<400> 1
   Leu Val Pro Glu Lys Glu Lys Asp Pro Lys Tyr Trp Arg Asp Gln Ala
   1               5                   10                  15
   Gln Glu Thr Leu Lys Tyr Ala Leu Glu Leu Gln Lys Leu Asn Thr Asn
               20                  25                  30
   Val Ala Lys Asn Val Ile Met Phe Leu Gly Asp Gly Met Gly Val Ser
           35                  40                  45
   Thr Val Thr Ala Ala Arg Ile Leu Lys Gly Gln Leu His His Asn Pro
       50                  55                  60
   Gly Glu Glu Thr Arg Leu Glu Met Asp Lys Phe Pro Phe Val Ala Leu
   65                  70                  75                  80
   Ser Lys Thr Tyr Asn Thr Asn Ala Gln Val Pro Asp Ser Ala Gly Thr
                   85                  90                  95
   Ala Thr Ala Tyr Leu Cys Gly Val Lys Ala Asn Glu Gly Thr Val Gly
               100                 105                 110
   Val Ser Ala Ala Thr Glu Arg Ser Arg Cys Asn Thr Thr Gln Gly Asn
           115                 120                 125
   Glu Val Thr Ser Ile Leu Arg Trp Ala Lys Asp Ala Gly Lys Ser Val
       130                 135                 140
   Gly Ile Val Thr Thr Thr Arg Val Asn His Ala Thr Pro Ser Ala Ala
   145                 150                 155                 160
   Tyr Ala His Ser Ala Asp Arg Asp Trp Tyr Ser Asp Asn Glu Met Pro
                   165                 170                 175
   Pro Glu Ala Leu Ser Gln Gly Cys Lys Asp Ile Ala Tyr Gln Leu Met
               180                 185                 190
   His Asn Ile Arg Asp Ile Asp Val Ile Met Gly Gly Gly Arg Lys Tyr
           195                 200                 205
   Met Tyr Pro Lys Asn Lys Thr Asp Val Glu Tyr Glu Ser Asp Glu Lys
       210                 215                 220
   Ala Arg Gly Thr Arg Leu Asp Gly Leu Asp Leu Val Asp Thr Trp Lys
   225                 230                 235                 240
   Ser Phe Lys Pro Arg Tyr Lys His Ser His Phe Ile Trp Asn Arg Thr
                   245                 250                 255
   Glu Leu Leu Thr Leu Asp Pro His Asn Val Asp Tyr Leu Leu Gly Leu
               260                 265                 270
   Phe Glu Pro Gly Asp Met Gln Tyr Glu Leu Asn Arg Asn Asn Val Thr
           275                 280                 285
   Asp Pro Ser Leu Ser Glu Met Val Val Val Ala Ile Gln Ile Leu Arg
       290                 295                 300
   Lys Asn Pro Lys Gly Phe Phe Leu Leu Val Glu Gly Gly Arg Ile Asp
   305                 310                 315                 320
   His Gly His His Glu Gly Lys Ala Lys Gln Ala Leu His Glu Ala Val
                   325                 330                 335
   Glu Met Asp Arg Ala Ile Gly Gln Ala Gly Ser Leu Thr Ser Ser Glu
               340                 345                 350
   Asp Thr Leu Thr Val Val Thr Ala Asp His Ser His Val Phe Thr Phe
           355                 360                 365
   Gly Gly Tyr Thr Pro Arg Gly Asn Ser Ile Phe Gly Leu Ala Pro Met
       370                 375                 380
   Leu Ser Asp Thr Asp Lys Lys Pro Phe Thr Ala Ile Leu Tyr Gly Asn
   385                 390                 395                 400
   Gly Pro Gly Tyr Lys Val Val Gly Gly Glu Arg Glu Asn Val Ser Met
                   405                 410                 415
   Val Asp Tyr Ala His Asn Asn Tyr Gln Ala Gln Ser Ala Val Pro Leu
               420                 425                 430
   Arg His Glu Thr His Gly Gly Glu Asp Val Ala Val Phe Ser Lys Gly
           435                 440                 445
   Pro Met Ala His Leu Leu His Gly Val His Glu Gln Asn Tyr Val Pro
       450                 455                 460
   His Val Met Ala Tyr Ala Ala Cys Ile Gly Ala Asn Leu Gly His Cys
   465                 470                 475                 480
   Ala Pro Ala Ser Ser Leu Lys Asp Lys Thr His Thr Cys Pro Pro Cys
                   485                 490                 495
   Pro Ala Pro Glu Leu Leu Gly Gly Pro Ser Val Phe Leu Phe Pro Pro
               500                 505                 510
   Lys Pro Lys Asp Thr Leu Met Ile Ser Arg Thr Pro Glu Val Thr Cys
           515                 520                 525
   Val Val Val Asp Val Ser His Glu Asp Pro Glu Val Lys Phe Asn Trp
       530                 535                 540
   Tyr Val Asp Gly Val Glu Val His Asn Ala Lys Thr Lys Pro Arg Glu
   545                 550                 555                 560
   Glu Gln Tyr Asn Ser Thr Tyr Arg Val Val Ser Val Leu Thr Val Leu
                   565                 570                 575
   His Gln Asp Trp Leu Asn Gly Lys Glu Tyr Lys Cys Lys Val Ser Asn
               580                 585                 590
   Lys Ala Leu Pro Ala Pro Ile Glu Lys Thr Ile Ser Lys Ala Lys Gly
           595                 600                 605
   Gln Pro Arg Glu Pro Gln Val Tyr Thr Leu Pro Pro Ser Arg Glu Glu
       610                 615                 620
   Met Thr Lys Asn Gln Val Ser Leu Thr Cys Leu Val Lys Gly Phe Tyr
   625                 630                 635                 640
   Pro Ser Asp Ile Ala Val Glu Trp Glu Ser Asn Gly Gln Pro Glu Asn
                   645                 650                 655
   Asn Tyr Lys Thr Thr Pro Pro Val Leu Asp Ser Asp Gly Ser Phe Phe
               660                 665                 670
   Leu Tyr Ser Lys Leu Thr Val Asp Lys Ser Arg Trp Gln Gln Gly Asn
   675 680 685
   Val Phe Ser Cys Ser Val Met His Glu Ala Leu His Asn His Tyr Thr
   690 695 700
   Gln Lys Ser Leu Ser Leu Ser Pro Gly Lys Asp Ile Asp Asp Asp Asp
   705 710 715 720
   Asp Asp Asp Asp Asp Asp
   725
<210> 2
   <211> 524
   <212> PRT
   <213> Homo sapiens
<400> 2
   Met Ile Ser Pro Phe Leu Val Leu Ala Ile Gly Thr Cys Leu Thr Asn
   1 5 10 15
   Ser Leu Val Pro Glu Lys Glu Lys Asp Pro Lys Tyr Trp Arg Asp Gln
   20 25 30
   Ala Gln Glu Thr Leu Lys Tyr Ala Leu Glu Leu Gln Lys Leu Asn Thr
   35 40 45
   Asn Val Ala Lys Asn Val Ile Met Phe Leu Gly Asp Gly Met Gly Val
   50 55 60
   Ser Thr Val Thr Ala Ala Arg Ile Leu Lys Gly Gln Leu His His Asn
   65 70 75 80
   Pro Gly Glu Glu Thr Arg Leu Glu Met Asp Lys Phe Pro Phe Val Ala
   85 90 95
   Leu Ser Lys Thr Tyr Asn Thr Asn Ala Gln Val Pro Asp Ser Ala Gly
   100 105 110
   Thr Ala Thr Ala Tyr Leu Cys Gly Val Lys Ala Asn Glu Gly Thr Val
   115 120 125
   Gly Val Ser Ala Ala Thr Glu Arg Ser Arg Cys Asn Thr Thr Gln Gly
   130 135 140
   Asn Glu Val Thr Ser Ile Leu Arg Trp Ala Lys Asp Ala Gly Lys Ser
   145 150 155 160
   Val Gly Ile Val Thr Thr Thr Arg Val Asn His Ala Thr Pro Ser Ala
                   165                 170                 175
   Ala Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
           195                 200                 205
   Met His Asn Ile Arg Asp Ile Asp Val Ile Met Gly Gly Gly Arg Lys
       210                 215                 220
   Tyr Met Tyr Pro Lys Asn Lys Thr Asp Val Glu Tyr Glu Ser Asp Glu
   225                 230                 235                 240
   Lys Ala Arg Gly Thr Arg Leu Asp Gly Leu Asp Leu Val Asp Thr Trp
                   245                 250                 255
   Lys Ser Phe Lys Pro Arg Tyr Lys His Ser His Phe Ile Trp Asn Arg
               260                 265                 270
   Thr Glu Leu Leu Thr Leu Asp Pro His Asn Val Asp Tyr Leu Leu Gly
           275                 280                 285
   Leu Phe Glu Pro Gly Asp Met Gln Tyr Glu Leu Asn Arg Asn Asn Val
       290                 295                 300
   Thr Asp Pro Ser Leu Ser Glu Met Val Val Val Ala Ile Gln Ile Leu
   305                 310                 315                 320
   Arg Lys Asn Pro Lys Gly Phe Phe Leu Leu Val Glu Gly Gly Arg Ile
                   325                 330                 335
   Asp His Gly His His Glu Gly Lys Ala Lys Gln Ala Leu His Glu Ala
               340                 345                 350
   Val Glu Met Asp Arg Ala Ile Gly Gln Ala Gly Ser Leu Thr Ser Ser
           355                 360                 365
   Glu Asp Thr Leu Thr Val Val Thr Ala Asp His Ser His Val Phe Thr
       370                 375                 380
   Phe Gly Gly Tyr Thr Pro Arg Gly Asn Ser Ile Phe Gly Leu Ala Pro
   385                 390                 395                 400
   Met Leu Ser Asp Thr Asp Lys Lys Pro Phe Thr Ala Ile Leu Tyr Gly
                   405                 410                 415
   Asn Gly Pro Gly Tyr Lys Val Val Gly Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Ser Lys
   450 455 460
   Gly Pro Met Ala His Leu Leu His Gly Val His Glu Gln Asn Tyr Val
   465 470 475 480
   Pro His Val Met Ala Tyr Ala Ala Cys Ile Gly Ala Asn Leu Gly His
   485 490 495
   Cys Ala Pro Ala Ser Ser Ala Gly Ser Leu Ala Ala Gly Pro Leu Leu
   500 505 510
   Leu Ala Leu Ala Leu Tyr Pro Leu Ser Val Leu Phe
   515 520
<210> 3
   <211> 524
   <212> PRT
   <213> Homo sapiens
<400> 3
<210> 4
   <211> 524
   <212> PRT
   <213> Homo sapiens
<400> 4
   Met Ile Ser Pro Phe Leu Val Leu Ala Ile Gly Thr Cys Leu Thr Asn
   1               5                   10                  15
   Ser Leu Val Pro Glu Lys Glu Lys Asp Pro Lys Tyr Trp Arg Asp Gln
               20                  25                  30
   Ala Gln Glu Thr Leu Lys Tyr Ala Leu Glu Leu Gln Lys Leu Asn Thr
           35                  40                  45
   Asn Val Ala Lys Asn Val Ile Met Phe Leu Gly Asp Gly Met Gly Val
       50                  55                  60
   Ser Thr Val Thr Ala Ala Arg Ile Leu Lys Gly Gln Leu His His Asn
   65                  70                  75                  80
   Pro Gly Glu Glu Thr Arg Leu Glu Met Asp Lys Phe Pro Phe Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Glu Arg Ser Arg Cys Asn Thr Thr Gln Gly
       130                 135                 140
   Asn Glu Val Thr Ser Ile Leu Arg Trp Ala Lys Asp Ala Gly Lys Ser
   145                 150                 155                 160
   Val Gly Ile Val Thr Thr Thr Arg Val Asn His Ala Thr Pro Ser Ala
                   165                 170                 175
   Ala Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
           195                 200                 205
   Met His Asn Ile Arg Asp Ile Asp Val Ile Met Gly Gly Gly Arg Lys
       210                 215                 220
   Tyr Met Tyr Pro Lys Asn Lys Thr Asp Val Glu Tyr Glu Ser Asp Glu
   225                 230                 235                 240
   Lys Ala Arg Gly Thr Arg Leu Asp Gly Leu Asp Leu Val Asp Thr Trp
                   245                 250                 255
   Lys Ser Phe Lys Pro Arg Tyr Lys His Ser His Phe Ile Trp Asn Arg
               260                 265                 270
   Thr Glu Leu Leu Thr Leu Asp Pro His Asn Val Asp Tyr Leu Leu Gly
           275                 280                 285
   Leu Phe Glu Pro Gly Asp Met Gln Tyr Glu Leu Asn Arg Asn Asn Val
       290                 295                 300
   Thr Asp Pro Ser Leu Ser Glu Met Val Val Val Ala Ile Gln Ile Leu
   305                 310                 315                 320
   Arg Lys Asn Pro Lys Gly Phe Phe Leu Leu Val Glu Gly Gly Arg Ile
                   325                 330                 335
   Asp His Gly His His Glu Gly Lys Ala Lys Gln Ala Leu His Glu Ala
               340                 345                 350
   Val Glu Met Asp Arg Ala Ile Gly Gln Ala Gly Ser Leu Thr Ser Ser
           355                 360                 365
   Glu Asp Thr Leu Thr Val Val Thr Ala Asp His Ser His Val Phe Thr
       370                 375                 380
   Phe Gly Gly Tyr Thr Pro Arg Gly Asn Ser Ile Phe Gly Leu Ala Pro
   385                 390                 395                 400
   Met Leu Ser Asp Thr Asp Lys Lys Pro Phe Thr Ala Ile Leu Tyr Gly
                   405                 410                 415
   Asn Gly Pro Gly Tyr Lys Val Val Gly Gly Glu Arg Glu Asn Val Ser
               420                 425                 430
   Met Val Asp Tyr Ala His Asn Asn Tyr Gln Ala Gln Ser Ala Val Pro
           435                 440                 445
   Leu Arg His Glu Thr His Gly Gly Glu Asp Val Ala Val Phe Ser Lys
       450                 455                 460
   Gly Pro Met Ala His Leu Leu His Gly Val His Glu Gln Asn Tyr Val
   465                 470                 475                 480
   Pro His Val Met Ala Tyr Ala Ala Cys Ile Gly Ala Asn Leu Gly His
                   485                 490                 495
   Cys Ala Pro Ala Ser Ser Ala Gly Ser Leu Ala Ala Gly Pro Leu Leu
               500                 505                 510
   Leu Ala Leu Ala Leu Tyr Pro Leu Ser Val Leu Phe
           515                 520
<210> 5
   <211> 524
   <212> PRT
   <213> Homo sapiens
<400> 5
   Met Ile Ser Pro Phe Leu Val Leu Ala Ile Gly Thr Cys Leu Thr Asn
   1               5                   10                  15
   Ser Leu Val Pro Glu Lys Glu Lys Asp Pro Lys Tyr Trp Arg Asp Gln
               20                  25                  30
   Ala Gln Glu Thr Leu Lys Tyr Ala Leu Glu Leu Gln Lys Leu Asn Thr
           35                  40                  45
   Asn Val Ala Lys Asn Val Ile Met Phe Leu Gly Asp Gly Met Gly Val
       50                  55                  60
   Ser Thr Val Thr Ala Ala Arg Ile Leu Lys Gly Gln Leu His His Asn
   65                  70                  75                  80
   Pro Gly Glu Glu Thr Arg Leu Glu Met Asp Lys Phe Pro Phe Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Glu Arg Ser Arg Cys Asn Thr Thr Gln Gly
       130                 135                 140
   Asn Glu Val Thr Ser Ile Leu Arg Trp Ala Lys Asp Ala Gly Lys Ser
   145                 150                 155                 160
   Val Gly Ile Val Thr Thr Thr Arg Val Asn His Ala Thr Pro Ser Ala
                   165                 170                 175
   Ala Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
           195                 200                 205
   Met His Asn Ile Arg Asp Ile Asp Val Ile Met Gly Gly Gly Arg Lys
       210                 215                 220
   Tyr Met Tyr Pro Lys Asn Lys Thr Asp Val Glu Tyr Glu Ser Asp Glu
   225                 230                 235                 240
   Lys Ala Arg Gly Thr Arg Leu Asp Gly Leu Asp Leu Val Asp Thr Trp
                   245                 250                 255
   Lys Ser Phe Lys Pro Arg Tyr Lys His Ser His Phe Ile Trp Asn Arg
               260                 265                 270
   Thr Glu Leu Leu Thr Leu Asp Pro His Asn Val Asp Tyr Leu Leu Gly
           275                 280                 285
   Leu Phe Glu Pro Gly Asp Met Gln Tyr Glu Leu Asn Arg Asn Asn Val
       290                 295                 300
   Thr Asp Pro Ser Leu Ser Glu Met Val Val Val Ala Ile Gln Ile Leu
   305                 310                 315                 320
   Arg Lys Asn Pro Lys Gly Phe Phe Leu Leu Val Glu Gly Gly Arg Ile
                   325                 330                 335
   Asp His Gly His His Glu Gly Lys Ala Lys Gln Ala Leu His Glu Ala
               340                 345                 350
   Val Glu Met Asp Arg Ala Ile Gly Gln Ala Gly Ser Leu Thr Ser Ser
           355                 360                 365
   Glu Asp Thr Leu Thr Val Val Thr Ala Asp His Ser His Val Phe Thr
       370                 375                 380
   Phe Gly Gly Tyr Thr Pro Arg Gly Asn Ser Ile Phe Gly Leu Ala Pro
   385                 390                 395                 400
   Met Leu Ser Asp Thr Asp Lys Lys Pro Phe Thr Ala Ile Leu Tyr Gly
                   405                 410                 415
   Asn Gly Pro Gly Tyr Lys Val Val Gly Gly Glu Arg Glu Asn Val Ser
               420                 425                 430
   Met Val Asp Tyr Ala His Asn Asn Tyr Gln Ala Gln Ser Ala Val Pro
           435                 440                 445
   Leu Arg His Glu Thr His Gly Gly Glu Asp Val Ala Val Phe Ser Lys
       450                 455                 460
   Gly Pro Met Ala His Leu Leu His Gly Val His Glu Gln Asn Tyr Val
   465                 470                 475                 480
   Pro His Val Met Ala Tyr Ala Ala Cys Ile Gly Ala Asn Leu Gly His
                   485                 490                 495
   Cys Ala Pro Ala Ser Ser Ala Gly Ser Leu Ala Ala Gly Pro Leu Leu
               500                 505                 510
   Leu Ala Leu Ala Leu Tyr Pro Leu Ser Val Leu Phe
   515 520
<210> 6
   <211> 524
   <212> PRT
   <213> Homo sapiens
<400> 6
<210> 7
   <211> 652
   <212> PRT
   <213> Macaca mulatta
<400> 7
   Met Pro Thr Val Lys Thr Lys Gln Glu Ser His Ala Gly Ser Gly Ser
   1               5                   10                  15
   Gly Pro Arg Leu Ala Glu Arg Lys Gly Arg Val Gly Ala Ala Arg Arg
               20                  25                  30
   Gln Ser Pro Arg Ala Pro Gly Gly Gly Leu Pro Gly Pro Arg Ser Gly
           35                  40                  45
   Pro Ala Ala Ala Phe Ile Arg Arg Arg Gly Arg Trp Pro Gly Pro Arg
       50                  55                  60
   Cys Ala Pro Ala Thr Pro Arg Pro Arg Ser Arg Leu Cys Ala Pro Thr
   65                  70                  75                  80
   Arg Leu Cys Leu Asp Glu Pro Ser Ser Val Leu Cys Ala Gly Leu Glu
                   85                  90                  95
   His Gln Leu Thr Ser Asp His Cys Gln Pro Thr Pro Ser His Pro Arg
               100                 105                 110
   Arg Ser His Leu Trp Ala Ser Gly Ile Lys Gln Val Leu Gly Cys Thr
           115                 120                 125
   Met Ile Ser Pro Phe Leu Val Leu Ala Ile Gly Thr Cys Leu Thr Asn
       130                 135                 140
   Ser Leu Val Pro Glu Lys Glu Lys Asp Pro Lys Tyr Trp Arg Asp Gln
   145 150 155 160
   Ala Gln Glu Thr Leu Lys Tyr Ala Leu Glu Leu Gln Lys Leu Asn Thr
   165 170 175
   Asn Val Ala Lys Asn Val Ile Met Phe Leu Gly Asp Gly Met Gly Val
   180 185 190
   Ser Thr Val Thr Ala Thr Arg Ile Leu Lys Gly Gln Leu His His Asn
   195 200 205
   Pro Gly Glu Glu Thr Arg Leu Glu Met Asp Lys Phe Pro Phe Val Ala
   210 215 220
   Leu Ser Lys Thr Tyr Asn Thr Asn Ala Gln Val Pro Asp Ser Ala Gly
   225 230 235 240
   Thr Ala Thr Ala Tyr Leu Cys Gly Val Lys Ala Asn Glu Gly Thr Val
   245 250 255
   Gly Val Ser Ala Ala Thr Glu Arg Ser Arg Cys Asn Thr Thr Gln Gly
   260 265 270
   Asn Glu Val Thr Ser Ile Leu Arg Trp Ala Lys Asp Ala Gly Lys Ser
   275 280 285
   Val Gly Ile Val Thr Thr Thr Arg Val Asn His Ala Thr Pro Ser Ala
   290 295 300
   Ala Tyr Ala His Ser Ala Asp Arg Asp Trp Tyr Ser Asp Asn Glu Met
   305 310 315 320
   Pro Pro Glu Ala Leu Ser Gln Gly Cys Lys Asp Ile Ala Tyr Gln Leu
   325 330 335
   Val His Asn Ile Arg Asp Ile Asp Val Ile Met Gly Gly Gly Arg Lys
   340 345 350
   Tyr Met Tyr Pro Lys Asn Lys Thr Asp Val Glu Tyr Glu Ile Asp Glu
   355 360 365
   Lys Ala Arg Gly Thr Arg Leu Asp Gly Leu Asp Leu Val Asn Ile Trp
   370 375 380
   Lys Ser Phe Lys Pro Arg His Lys His Ser His Phe Ile Trp Asn Arg
   385 390 395 400
   Thr Glu Leu Leu Thr Leu Asp Pro His Asn Val Asp Tyr Leu Leu Gly
                   405                 410                 415
   Leu Phe Glu Pro Gly Asp Met Glu Tyr Glu Leu Asn Arg Asn Asn Val
               420                 425                 430
   Thr Asp Pro Ser Leu Ser Glu Met Val Val Val Ala Ile Gln Ile Leu
           435                 440                 445
   Arg Lys Asn Pro Lys Gly Phe Phe Leu Leu Val Glu Gly Gly Arg Ile
       450                 455                 460
   Asp His Gly His His Glu Gly Lys Ala Lys Gln Ala Leu His Glu Ala
   465                 470                 475                 480
   Val Glu Met Asp Arg Ala Ile Gly Gln Ala Gly Ser Met Thr Ser Leu
                   485                 490                 495
   Glu Asp Thr Leu Thr Val Val Thr Ala Asp His Ser His Val Phe Thr
               500                 505                 510
   Phe Gly Gly Tyr Thr Pro Arg Gly Asn Ser Ile Phe Gly Leu Ala Pro
           515                 520                 525
   Met Leu Ser Asp Thr Asp Lys Lys Pro Phe Thr Ala Ile Leu Tyr Gly
       530                 535                 540
   Asn Gly Pro Gly Tyr Lys Val Val Gly Gly Glu Arg Glu Asn Val Ser
   545                 550                 555                 560
   Met Val Asp Tyr Ala His Asn Asn Tyr Gln Ala Gln Ser Ala Val Pro
                   565                 570                 575
   Leu Arg His Glu Thr His Gly Gly Glu Asp Val Ala Val Phe Ser Lys
               580                 585                 590
   Gly Pro Met Ala His Leu Leu His Gly Val His Glu Gln Asn Tyr Ile
           595                 600                 605
   Pro His Val Met Ala Tyr Ala Ala Cys Ile Gly Ala Asn Leu Asp His
       610                 615                 620
   Cys Ala Pro Ala Ser Ser Ala Gly Ser Leu Ala Ala Gly Pro Leu Leu
   625                 630                 635                 640
   Leu Pro Leu Ala Leu Phe Pro Leu Ser Ile Leu Phe
                   645                 650
<210> 8
   <211> 524
   <212> PRT
   <213> Rattus norvegicus
<400> 8
   Met Ile Leu Pro Phe Leu Val Leu Ala Ile Gly Pro Cys Leu Thr Asn
   1               5                   10                  15
   Ser Phe Val Pro Glu Lys Glu Lys Asp Pro Ser Tyr Trp Arg Gln Gln
               20                  25                  30
   Ala Gln Glu Thr Leu Lys Asn Ala Leu Lys Leu Gln Lys Leu Asn Thr
           35                  40                  45
   Asn Val Ala Lys Asn Ile Ile Met Phe Leu Gly Asp Gly Met Gly Val
       50                  55                  60
   Ser Thr Val Thr Ala Ala Arg Ile Leu Lys Gly Gln Leu His His Asn
   65                  70                  75                  80
   Thr Gly Glu Glu Thr Arg Leu Glu Met Asp Lys Phe Pro Phe Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Glu Arg Thr Arg Cys Asn Thr Thr Gln Gly
       130                 135                 140
   Asn Glu Val Thr Ser Ile Leu Arg Trp Ala Lys Asp Ala Gly Lys Ser
   145                 150                 155                 160
   Val Gly Ile Val Thr Thr Thr Arg Val Asn His Ala Thr Pro Ser Ala
                   165                 170                 175
   Ala Tyr Ala His Ser Ala Asp Arg Asp Trp Tyr Ser Asp Asn Glu Met
               180                 185                 190
   Arg Pro Glu Ala Leu Ser Gln Gly Cys Lys Asp Ile Ala Tyr Gln Leu
           195                 200                 205
   Met His Asn Ile Lys Asp Ile Asp Val Ile Met Gly Gly Gly Arg Lys
       210                 215                 220
   Tyr Met Tyr Pro Lys Asn Arg Thr Asp Val Glu Tyr Glu Leu Asp Glu
   225                 230                 235                 240
   Lys Ala Arg Gly Thr Arg Leu Asp Gly Leu Asp Leu Ile Ser Ile Trp
                   245                 250                 255
   Lys Ser Phe Lys Pro Arg His Lys His Ser His Tyr Val Trp Asn Arg
               260                 265                 270
   Thr Glu Leu Leu Ala Leu Asp Pro Ser Arg Val Asp Tyr Leu Leu Gly
           275                 280                 285
   Leu Phe Glu Pro Gly Asp Met Gln Tyr Glu Leu Asn Arg Asn Asn Leu
       290                 295                 300
   Thr Asp Pro Ser Leu Ser Glu Met Val Glu Val Ala Leu Arg Ile Leu
   305                 310                 315                 320
   Thr Lys Asn Pro Lys Gly Phe Phe Leu Leu Val Glu Gly Gly Arg Ile
                   325                 330                 335
   Asp His Gly His His Glu Gly Lys Ala Lys Gln Ala Leu His Glu Ala
               340                 345                 350
   Val Glu Met Asp Glu Ala Ile Gly Lys Ala Gly Thr Met Thr Ser Gln
           355                 360                 365
   Lys Asp Thr Leu Thr Val Val Thr Ala Asp His Ser His Val Phe Thr
       370                 375                 380
   Phe Gly Gly Tyr Thr Pro Arg Gly Asn Ser Ile Phe Gly Leu Ala Pro
   385                 390                 395                 400
   Met Val Ser Asp Thr Asp Lys Lys Pro Phe Thr Ala Ile Leu Tyr Gly
                   405                 410                 415
   Asn Gly Pro Gly Tyr Lys Val Val Asp Gly Glu Arg Glu Asn Val Ser
               420                 425                 430
   Met Val Asp Tyr Ala His Asn Asn Tyr Gln Ala Gln Ser Ala Val Pro
           435                 440                 445
   Leu Arg His Glu Thr His Gly Gly Glu Asp Val Ala Val Phe Ala Lys
       450                 455                 460
   Gly Pro Met Ala His Leu Leu His Gly Val His Glu Gln Asn Tyr Ile
   465                 470                 475                 480
   Pro His Val Met Ala Tyr Ala Ser Cys Ile Gly Ala Asn Leu Asp His
   485 490 495
   Cys Ala Trp Ala Ser Ser Ala Ser Ser Pro Ser Pro Gly Ala Leu Leu
   500 505 510
   Leu Pro Leu Ala Leu Phe Pro Leu Arg Thr Leu Phe
   515 520
<210> 9
   <211> 502
   <212> PRT
   <213> Canis lupus familiaris
<400> 9
   Glu Lys Asp Pro Lys Tyr Trp Arg Asp Gln Ala Gln Gln Thr Leu Lys
   1 5 10 15
   Tyr Ala Leu Arg Leu Gln Asn Leu Asn Thr Asn Val Ala Lys Asn Val
   20 25 30
   Ile Met Phe Leu Gly Asp Gly Met Gly Val Ser Thr Val Thr Ala Thr
   35 40 45
   Arg Ile Leu Lys Gly Gln Leu His His Asn Pro Gly Glu Glu Thr Arg
   50 55 60
   Leu Glu Met Asp Lys Phe Pro Tyr Val Ala Leu Ser Lys Thr Tyr Asn
   65 70 75 80
   Thr Asn Ala Gln Val Pro Asp Ser Ala Gly Thr Ala Thr Ala Tyr Leu
   85 90 95
   Cys Gly Val Lys Ala Asn Glu Gly Thr Val Gly Val Ser Ala Ala Thr
   100 105 110
   Gln Arg Thr His Cys Asn Thr Thr Gln Gly Asn Glu Val Thr Ser Ile
   115 120 125
   Leu Arg Trp Ala Lys Asp Ala Gly Lys Ser Val Gly Ile Val Thr Thr
   130 135 140
   Thr Arg Val Asn His Ala Thr Pro Ser Ala Ala Tyr Ala His Ser Ala
   145 150 155 160
   Asp Arg Asp Trp Tyr Ser Asp Asn Glu Met Pro Pro Glu Ala Leu Ser
   165 170 175
   Gln Gly Cys Lys Asp Ile Ala Tyr Gln Leu Met His Asn Val Lys Asp
               180                 185                 190
   Ile Glu Val Ile Met Gly Gly Gly Arg Lys Tyr Met Phe Pro Lys Asn
           195                 200                 205
   Arg Thr Asp Val Glu Tyr Glu Met Asp Glu Lys Ser Thr Gly Ala Arg
       210                 215                 220
   Leu Asp Gly Leu Asn Leu Ile Asp Ile Trp Lys Asn Phe Lys Pro Arg
   225                 230                 235                 240
   His Lys His Ser His Tyr Val Trp Asn Arg Thr Glu Leu Leu Ala Leu
                   245                 250                 255
   Asp Pro Tyr Thr Val Asp Tyr Leu Leu Gly Leu Phe Asp Pro Gly Asp
               260                 265                 270
   Met Gln Tyr Glu Leu Asn Arg Asn Asn Val Thr Asp Pro Ser Leu Ser
           275                 280                 285
   Glu Met Val Glu Ile Ala Ile Lys Ile Leu Ser Lys Lys Pro Arg Gly
       290                 295                 300
   Phe Phe Leu Leu Val Glu Gly Gly Arg Ile Asp His Gly His His Glu
   305                 310                 315                 320
   Gly Lys Ala Lys Gln Ala Leu His Glu Ala Val Glu Met Asp Arg Ala
                   325                 330                 335
   Ile Gly Lys Ala Gly Val Met Thr Ser Leu Glu Asp Thr Leu Thr Val
               340                 345                 350
   Val Thr Ala Asp His Ser His Val Phe Thr Phe Gly Gly Tyr Thr Pro
           355                 360                 365
   Arg Gly Asn Ser Ile Phe Gly Leu Ala Pro Met Val Ser Asp Thr Asp
       370                 375                 380
   Lys Lys Pro Phe Thr Ala Ile Leu Tyr Gly Asn Gly Pro Gly Tyr Lys
   385                 390                 395                 400
   Val Val Gly Gly Glu Arg Glu Asn Val Ser Met Val Asp Tyr Ala His
                   405                 410                 415
   Asn Asn Tyr Gln Ala Gln Ser Ala Val Pro Leu Arg His Glu Thr His
                   420                 425                 430
   Gly Gly Glu Asp Val Ala Val Phe Ala Lys Gly Pro Met Ala His Leu
           435                 440                 445
   Leu His Gly Val His Glu Gln Asn Tyr Ile Pro His Val Met Ala Tyr
       450                 455                 460
   Ala Ala Cys Ile Gly Ala Asn Gln Asp His Cys Ala Ser Ala Ser Ser
   465                 470                 475                 480
   Ala Gly Gly Pro Ser Pro Gly Pro Leu Leu Leu Leu Leu Ala Leu Leu
                   485                 490                 495
   Pro Val Gly Ile Leu Phe
               500
<210> 10
   <211> 252
   <212> PRT
   <213> Sus scrofa
<400> 10
   Ala Glu Leu Leu Ala Leu Asp Pro His Thr Val Asp Tyr Leu Leu Gly
   1               5                   10                  15
   Leu Phe Glu Pro Gly Asp Met Gln Tyr Glu Leu Asn Arg Asn Asn Val
               20                  25                  30
   Thr Asp Pro Ser Leu Ser Glu Met Val Glu Met Ala Ile Arg Ile Leu
           35                  40                  45
   Ile Lys Asn Pro Lys Gly Phe Phe Leu Leu Val Glu Gly Gly Arg Ile
       50                  55                  60
   Asp His Gly His His Glu Gly Lys Ala Lys Gln Ala Leu His Glu Ala
   65                  70                  75                  80
   Val Glu Met Asp Arg Ala Ile Glu Gln Ala Gly Ser Met Thr Ser Val
                   85                  90                  95
   Glu Asp Thr Leu Thr Val Val Thr Ala Asp His Ser His Val Phe Thr
               100                 105                 110
   Phe Gly Gly Tyr Thr Pro Arg Gly Asn Ser Ile Phe Gly Leu Ala Pro
           115                 120                 125
   Met Val Ser Asp Thr Asp Lys Lys Pro Phe Thr Ala Ile Leu Tyr Gly
           130                 135                 140
   Asn Gly Pro Gly Tyr Lys Val Val Gly Gly Glu Arg Glu Asn Val Ser
   145                 150                 155                 160
   Met Val Asp Tyr Ala His Asp Asn Tyr Gln Ala Gln Ser Ala Val Pro
                   165                 170                 175
   Leu Arg His Glu Thr His Gly Gly Glu Asp Val Ala Ile Phe Ala Arg
               180                 185                 190
   Gly Pro Met Ala His Leu Leu His Gly Val His Glu Gln Asn Tyr Ile
           195                 200                 205
   Pro His Val Met Ala Tyr Ala Ala Cys Val Gly Ala Asn Arg Asp His
       210                 215                 220
   Cys Ala Ser Ala Ser Ser Ser Gly Ser Pro Ser Pro Gly Pro Leu Leu
   225                 230                 235                 240
   Leu Leu Leu Ala Leu Leu Pro Leu Gly Ile Leu Phe
                   245                 250
<210> 11
   <211> 524
   <212> PRT
   <213> Mus musculus
<400> 11
   Met Ile Ser Pro Phe Leu Val Leu Ala Ile Gly Thr Cys Leu Thr Asn
   1               5                   10                  15
   Ser Phe Val Pro Glu Lys Glu Arg Asp Pro Ser Tyr Trp Arg Gln Gln
               20                  25                  30
   Ala Gln Glu Thr Leu Lys Asn Ala Leu Lys Leu Gln Lys Leu Asn Thr
           35                  40                  45
   Asn Val Ala Lys Asn Val Ile Met Phe Leu Gly Asp Gly Met Gly Val
       50                  55                  60
   Ser Thr Val Thr Ala Ala Arg Ile Leu Lys Gly Gln Leu His His Asn
   65                  70                  75                  80
   Thr Gly Glu Glu Thr Arg Leu Glu Met Asp Lys Phe Pro Phe Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Glu Arg Thr Arg Cys Asn Thr Thr Gln Gly
       130                 135                 140
   Asn Glu Val Thr Ser Ile Leu Arg Trp Ala Lys Asp Ala Gly Lys Ser
   145                 150                 155                 160
   Val Gly Ile Val Thr Thr Thr Arg Val Asn His Ala Thr Pro Ser Ala
                   165                 170                 175
   Ala Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
           195                 200                 205
   Met His Asn Ile Lys Asp Ile Asp Val Ile Met Gly Gly Gly Arg Lys
       210                 215                 220
   Tyr Met Tyr Pro Lys Asn Arg Thr Asp Val Glu Tyr Glu Leu Asp Glu
   225                 230                 235                 240
   Lys Ala Arg Gly Thr Arg Leu Asp Gly Leu Asp Leu Ile Ser Ile Trp
                   245                 250                 255
   Lys Ser Phe Lys Pro Arg His Lys His Ser His Tyr Val Trp Asn Arg
               260                 265                 270
   Thr Glu Leu Leu Ala Leu Asp Pro Ser Arg Val Asp Tyr Leu Leu Gly
           275                 280                 285
   Leu Phe Glu Pro Gly Asp Met Gln Tyr Glu Leu Asn Arg Asn Asn Leu
       290                 295                 300
   Thr Asp Pro Ser Leu Ser Glu Met Val Glu Val Ala Leu Arg Ile Leu
   305                 310                 315                 320
   Thr Lys Asn Leu Lys Gly Phe Phe Leu Leu Val Glu Gly Gly Arg Ile
                   325                 330                 335
   Asp His Gly His His Glu Gly Lys Ala Lys Gln Ala Leu His Glu Ala
               340                 345                 350
   Val Glu Met Asp Gln Ala Ile Gly Lys Ala Gly Ala Met Thr Ser Gln
           355                 360                 365
   Lys Asp Thr Leu Thr Val Val Thr Ala Asp His Ser His Val Phe Thr
       370                 375                 380
   Phe Gly Gly Tyr Thr Pro Arg Gly Asn Ser Ile Phe Gly Leu Ala Pro
   385                 390                 395                 400
   Met Val Ser Asp Thr Asp Lys Lys Pro Phe Thr Ala Ile Leu Tyr Gly
                   405                 410                 415
   Asn Gly Pro Gly Tyr Lys Val Val Asp Gly Glu Arg Glu Asn Val Ser
               420                 425                 430
   Met Val Asp Tyr Ala His Asn Asn Tyr Gln Ala Gln Ser Ala Val Pro
           435                 440                 445
   Leu Arg His Glu Thr His Gly Gly Glu Asp Val Ala Val Phe Ala Lys
       450                 455                 460
   Gly Pro Met Ala His Leu Leu His Gly Val His Glu Gln Asn Tyr Ile
   465                 470                 475                 480
   Pro His Val Met Ala Tyr Ala Ser Cys Ile Gly Ala Asn Leu Asp His
                   485                 490                 495
   Cys Ala Trp Ala Gly Ser Gly Ser Ala Pro Ser Pro Gly Ala Leu Leu
               500                 505                 510
   Leu Pro Leu Ala Val Leu Ser Leu Arg Thr Leu Phe
           515                 520
<210> 12
   <211> 524
   <212> PRT
   <213> Bos taurus
<400> 12
   Met Ile Ser Pro Phe Leu Leu Leu Ala Ile Gly Thr Cys Phe Ala Ser
   1               5                   10                  15
   Ser Leu Val Pro Glu Lys Glu Lys Asp Pro Lys Tyr Trp Arg Asp Gln
               20                  25                  30
   Ala Gln Gln Thr Leu Lys Asn Ala Leu Arg Leu Gln Thr Leu Asn Thr
           35                  40                  45
   Asn Val Ala Lys Asn Val Ile Met Phe Leu Gly Asp Gly Met Gly Val
       50                  55                  60
   Ser Thr Val Thr Ala Ala Arg Ile Leu Lys Gly Gln Leu His His Ser
   65                  70                  75                  80
   Pro Gly Glu Glu Thr Lys Leu Glu Met Asp Lys Phe Pro Tyr Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Gln Arg Ser Gln Cys Asn Thr Thr Gln Gly
       130                 135                 140
   Asn Glu Val Thr Ser Ile Leu Arg Trp Ala Lys Asp Ala Gly Lys Ser
   145                 150                 155                 160
   Val Gly Ile Val Thr Thr Thr Arg Val Asn His Ala Thr Pro Ser Ala
                   165                 170                 175
   Ser Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
           195                 200                 205
   Met His Asn Ile Lys Asp Ile Glu Val Ile Met Gly Gly Gly Arg Lys
       210                 215                 220
   Tyr Met Phe Pro Lys Asn Arg Thr Asp Val Glu Tyr Glu Leu Asp Glu
   225                 230                 235                 240
   Lys Ala Arg Gly Thr Arg Leu Asp Gly Leu Asn Leu Ile Asp Ile Trp
                   245                 250                 255
   Lys Ser Phe Lys Pro Lys His Lys His Ser His Tyr Val Trp Asn Arg
               260                 265                 270
   Thr Asp Leu Leu Ala Leu Asp Pro His Ser Val Asp Tyr Leu Leu Gly
           275                 280                 285
   Leu Phe Glu Pro Gly Asp Met Gln Tyr Glu Leu Asn Arg Asn Asn Ala
       290                 295                 300
   Thr Asp Pro Ser Leu Ser Glu Met Val Glu Met Ala Ile Arg Ile Leu
   305                 310                 315                 320
   Asn Lys Asn Pro Lys Gly Phe Phe Leu Leu Val Glu Gly Gly Arg Ile
                   325                 330                 335
   Asp His Gly His His Glu Gly Lys Ala Lys Gln Ala Leu His Glu Ala
               340                 345                 350
   Val Glu Met Asp Gln Ala Ile Gly Gln Ala Gly Ala Met Thr Ser Val
           355                 360                 365
   Glu Asp Thr Leu Thr Val Val Thr Ala Asp His Ser His Val Phe Thr
       370                 375                 380
   Phe Gly Gly Tyr Thr Pro Arg Gly Asn Ser Ile Phe Gly Leu Ala Pro
   385                 390                 395                 400
   Met Val Ser Asp Thr Asp Lys Lys Pro Phe Thr Ala Ile Leu Tyr Gly
                   405                 410                 415
   Asn Gly Pro Gly Tyr Lys Val Val Gly Gly Glu Arg Glu Asn Val Ser
               420                 425                 430
   Met Val Asp Tyr Ala His Asn Asn Tyr Gln Ala Gln Ser Ala Val Pro
           435                 440                 445
   Leu Arg His Glu Thr His Gly Gly Glu Asp Val Ala Val Phe Ala Lys
       450                 455                 460
   Gly Pro Met Ala His Leu Leu His Gly Val His Glu Gln Asn Tyr Ile
   465                 470                 475                 480
   Pro His Val Met Ala Tyr Ala Ala Cys Ile Gly Ala Asn Arg Asp His
                   485                 490                 495
   Cys Ala Ser Ala Ser Ser Ser Gly Ser Pro Ser Pro Gly Pro Leu Leu
               500                 505                 510
   Leu Leu Leu Ala Leu Leu Pro Leu Gly Ser Leu Phe
           515                 520
<210> 13
   <211> 524
   <212> PRT
   <213> Bos taurus
<400> 13
   Met Ile Ser Pro Phe Leu Leu Leu Ala Ile Gly Thr Cys Phe Ala Ser
   1               5                   10                  15
   Ser Leu Val Pro Glu Lys Glu Lys Asp Pro Lys Tyr Trp Arg Asp Gln
               20                  25                  30
   Ala Gln Gln Thr Leu Lys Asn Ala Leu Arg Leu Gln Thr Leu Asn Thr
           35                  40                  45
   Asn Val Ala Lys Asn Val Ile Met Phe Leu Gly Asp Gly Met Gly Val
       50                  55                  60
   Ser Thr Val Thr Ala Ala Arg Ile Leu Lys Gly Gln Leu His His Ser
   65                  70                  75                  80
   Pro Gly Glu Glu Thr Lys Leu Glu Met Asp Lys Phe Pro Tyr Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Gln Arg Ser Gln Cys Asn Thr Thr Gln Gly
       130                 135                 140
   Asn Glu Val Thr Ser Ile Leu Arg Trp Ala Lys Asp Ala Gly Lys Ser
   145                 150                 155                 160
   Val Gly Ile Val Thr Thr Thr Arg Val Asn His Ala Thr Pro Ser Ala
                   165                 170                 175
   Ser Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
           195                 200                 205
   Met His Asn Ile Lys Asp Ile Glu Val Ile Met Gly Gly Gly Arg Lys
       210                 215                 220
   Tyr Met Phe Pro Lys Asn Arg Thr Asp Val Glu Tyr Glu Leu Asp Glu
   225                 230                 235                 240
   Lys Ala Arg Gly Thr Arg Leu Asp Gly Leu Asn Leu Ile Asp Ile Trp
                   245                 250                 255
   Lys Ser Phe Lys Pro Lys His Lys His Ser His Tyr Val Trp Asn Arg
               260                 265                 270
   Thr Asp Leu Leu Ala Leu Asp Pro His Ser Val Asp Tyr Leu Leu Gly
           275                 280                 285
   Leu Phe Glu Pro Gly Asp Met Gln Tyr Glu Leu Asn Arg Asn Asn Ala
       290                 295                 300
   Thr Asp Pro Ser Leu Ser Glu Met Val Glu Met Ala Ile Arg Ile Leu
   305                 310                 315                 320
   Asn Lys Asn Pro Lys Gly Phe Phe Leu Leu Val Glu Gly Gly Arg Ile
                   325                 330                 335
   Asp His Gly His His Glu Gly Lys Ala Lys Gln Ala Leu His Glu Ala
               340                 345                 350
   Val Glu Met Asp Gln Ala Ile Gly Gln Ala Gly Ala Met Thr Ser Val
           355                 360                 365
   Glu Asp Thr Leu Thr Val Val Thr Ala Asp His Ser His Val Phe Thr
       370                 375                 380
   Phe Gly Gly Tyr Thr Pro Arg Gly Asn Ser Ile Phe Gly Leu Ala Pro
   385                 390                 395                 400
   Met Val Ser Asp Thr Asp Lys Lys Pro Phe Thr Ala Ile Leu Tyr Gly
                   405                 410                 415
   Asn Gly Pro Gly Tyr Lys Val Val Gly Gly Glu Arg Glu Asn Val Ser
               420                 425                 430
   Met Val Asp Tyr Ala His Asn Asn Tyr Gln Ala Gln Ser Ala Val Pro
           435                 440                 445
   Leu Arg His Glu Thr His Gly Gly Glu Asp Val Ala Val Phe Ala Lys
       450                 455                 460
   Gly Pro Met Ala His Leu Leu His Gly Val His Glu Gln Asn Tyr Ile
   465                 470                 475                 480
   Pro His Val Met Ala Tyr Ala Ala Cys Ile Gly Ala Asn Arg Asp His
                   485                 490                 495
   Cys Ala Ser Ala Ser Ser Ser Gly Ser Pro Ser Pro Gly Pro Leu Leu
   500 505 510
   Leu Leu Leu Ala Leu Leu Pro Leu Gly Ser Leu Phe
   515 520
<210> 14
   <211> 124
   <212> PRT
   <213> Bos taurus
<400> 14
   Asp Pro Lys Tyr Trp Arg Asp Gln Ala Gln Gln Thr Leu Lys Asn Ala
   1 5 10 15
   Leu Gly Leu Gln Lys Leu Asn Thr Lys Val Ala Lys Asn Val Ile Leu
   20 25 30
   Phe Leu Gly Asp Gly Met Gly Val Ser Thr Val Thr Ala Ala Arg Ile
   35 40 45
   Leu Lys Gly Gln Leu His His Asn Pro Gly Glu Glu Thr Arg Leu Glu
   50 55 60
   Met Asp Lys Phe Pro Phe Val Ala Leu Ser Lys Thr Tyr Asn Thr Asn
   65 70 75 80
   Ala Gln Val Pro Asp Ser Ala Gly Thr Ala Pro His Pro Val Arg Val
   85 90 95
   Lys Ala Met Arg Ala Pro Trp Gly Glu Pro His Gln Arg Gln Cys Asn
   100 105 110
   Thr Arg Arg Ala Thr Ser Thr His Leu Leu Ala Gly
   115 120
<210> 15
   <211> 524
   <212> PRT
   <213> Felis catus
<400> 15
   Met Ile Ser Pro Phe Leu Val Leu Ala Ile Gly Thr Cys Leu Thr Asn
   1               5                   10                  15
   Ser Leu Val Pro Glu Lys Glu Lys Asp Pro Lys Tyr Trp Arg Asp Gln
               20                  25                  30
   Ala Gln Gln Thr Leu Lys Asn Ala Leu Arg Leu Gln Lys Leu Asn Thr
           35                  40                  45
   Asn Val Val Lys Asn Val Ile Met Phe Leu Gly Asp Gly Met Gly Val
       50                  55                  60
   Ser Thr Val Thr Ala Ala Arg Ile Leu Lys Gly Gln Leu His His Asn
   65                  70                  75                  80
   Pro Gly Glu Glu Thr Arg Leu Glu Met Asp Lys Phe Pro Tyr Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Gln Arg Thr Gln Cys Asn Thr Thr Gln Gly
       130                 135                 140
   Asn Glu Val Thr Ser Ile Leu Arg Trp Ala Lys Asp Ser Gly Lys Ser
   145                 150                 155                 160
   Val Gly Ile Val Thr Thr Thr Arg Val Asn His Ala Thr Pro Ser Ala
                   165                 170                 175
   Ala Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
           195                 200                 205
   Met His Asn Val Arg Asp Ile Glu Val Ile Met Gly Gly Gly Arg Lys
       210                 215                 220
   Tyr Met Phe Pro Lys Asn Arg Thr Asp Val Glu Tyr Glu Met Asp Glu
   225                 230                 235                 240
   Lys Ala Arg Gly Thr Arg Leu Asp Gly Leu Asn Leu Val Asp Ile Trp
                   245                 250                 255
   Lys Ser Phe Lys Pro Arg His Lys His Ser His Tyr Val Trp Asn Arg
               260                 265                 270
   Thr Glu Leu Leu Thr Leu Asp Pro Tyr Gly Val Asp Tyr Leu Leu Gly
           275                 280                 285
   Leu Phe Glu Pro Gly Asp Met Gln Tyr Glu Leu Asn Arg Asn Ser Thr
   290 295 300
   Thr Asp Pro Ser Leu Ser Glu Met Val Glu Ile Ala Ile Lys Ile Leu
   305 310 315 320
   Ser Lys Asn Pro Lys Gly Phe Phe Leu Leu Val Glu Gly Gly Arg Ile
   325 330 335
   Asp His Gly His His Glu Gly Lys Ala Lys Gln Ala Leu His Glu Ala
   340 345 350
   Val Glu Met Asp Gln Ala Ile Gly Arg Ala Gly Ala Met Thr Ser Val
   355 360 365
   Glu Asp Thr Leu Thr Ile Val Thr Ala Asp His Ser His Val Phe Thr
   370 375 380
   Phe Gly Gly Tyr Thr Pro Arg Gly Asn Ser Ile Phe Gly Leu Ala Pro
   385 390 395 400
   Met Val Ser Asp Thr Asp Lys Lys Pro Phe Thr Ser Ile Leu Tyr Gly
   405 410 415
   Asn Gly Pro Gly Tyr Lys Val Val Gly Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Ala Lys
   450 455 460
   Gly Pro Met Ala His Leu Leu His Gly Val His Glu Gln Asn Tyr Ile
   465 470 475 480
   Pro His Val Met Ala Tyr Ala Ala Cys Ile Gly Ala Asn Leu Asp His
   485 490 495
   Cys Ala Ser Ala Ser Ser Ala Gly Gly Pro Ser Pro Gly Pro Leu Phe
   500 505 510
   Leu Leu Leu Ala Leu Pro Ser Leu Gly Ile Leu Phe
   515 520
<210> 16
   <211> 532
   <212> PRT
   <213> Homo sapiens
<400> 16
   Met Gln Gly Pro Trp Val Leu Leu Leu Leu Gly Leu Arg Leu Gln Leu
   1               5                   10                  15
   Ser Leu Gly Ile Ile Pro Val Glu Glu Glu Asn Pro Asp Phe Trp Asn
               20                  25                  30
   Arg Gln Ala Ala Glu Ala Leu Gly Ala Ala Lys Lys Leu Gln Pro Ala
           35                  40                  45
   Gln Thr Ala Ala Lys Asn Leu Ile Ile Phe Leu Gly Asp Gly Met Gly
       50                  55                  60
   Val Ser Thr Val Thr Ala Ala Arg Ile Leu Lys Gly Gln Lys Lys Asp
   65                  70                  75                  80
   Lys Leu Gly Pro Glu Thr Phe Leu Ala Met Asp Arg Phe Pro Tyr Val
                   85                  90                  95
   Ala Leu Ser Lys Thr Tyr Ser Val Asp Lys His Val Pro Asp Ser Gly
               100                 105                 110
   Ala Thr Ala Thr Ala Tyr Leu Cys Gly Val Lys Gly Asn Phe Gln Thr
           115                 120                 125
   Ile Gly Leu Ser Ala Ala Ala Arg Phe Asn Gln Cys Asn Thr Thr Arg
       130                 135                 140
   Gly Asn Glu Val Ile Ser Val Met Asn Arg Ala Lys Lys Ala Gly Lys
   145                 150                 155                 160
   Ser Val Gly Val Val Thr Thr Thr Arg Val Gln His Ala Ser Pro Ala
                   165                 170                 175
   Gly Ala Tyr Ala His Thr Val Asn Arg Asn Trp Tyr Ser Asp Ala Asp
               180                 185                 190
   Val Pro Ala Ser Ala Arg Gln Glu Gly Cys Gln Asp Ile Ala Thr Gln
           195                 200                 205
   Leu Ile Ser Asn Met Asp Ile Asp Val Ile Leu Gly Gly Gly Arg Lys
       210                 215                 220
   Tyr Met Phe Pro Met Gly Thr Pro Asp Pro Glu Tyr Pro Asp Asp Tyr
   225                 230                 235                 240
   Ser Gln Gly Gly Thr Arg Leu Asp Gly Lys Asn Leu Val Gln Glu Trp
                   245                 250                 255
   Leu Ala Lys His Gln Gly Ala Arg Tyr Val Trp Asn Arg Thr Glu Leu
               260                 265                 270
   Leu Gln Ala Ser Leu Asp Pro Ser Val Thr His Leu Met Gly Leu Phe
           275                 280                 285
   Glu Pro Gly Asp Met Lys Tyr Glu Ile His Arg Asp Ser Thr Leu Asp
       290                 295                 300
   Pro Ser Leu Met Glu Met Thr Glu Ala Ala Leu Leu Leu Leu Ser Arg
   305                 310                 315                 320
   Asn Pro Arg Gly Phe Phe Leu Phe Val Glu Gly Gly Arg Ile Asp His
                   325                 330                 335
   Gly His His Glu Ser Arg Ala Tyr Arg Ala Leu Thr Glu Thr Ile Met
               340                 345                 350
   Phe Asp Asp Ala Ile Glu Arg Ala Gly Gln Leu Thr Ser Glu Glu Asp
           355                 360                 365
   Thr Leu Ser Leu Val Thr Ala Asp His Ser His Val Phe Ser Phe Gly
       370                 375                 380
   Gly Tyr Pro Leu Arg Gly Ser Ser Ile Phe Gly Leu Ala Pro Gly Lys
   385                 390                 395                 400
   Ala Arg Asp Arg Lys Ala Tyr Thr Val Leu Leu Tyr Gly Asn Gly Pro
                   405                 410                 415
   Gly Tyr Val Leu Lys Asp Gly Ala Arg Pro Asp Val Thr Glu Ser Glu
               420                 425                 430
   Ser Gly Ser Pro Glu Tyr Arg Gln Gln Ser Ala Val Pro Leu Asp Gly
           435                 440                 445
   Glu Thr His Ala Gly Glu Asp Val Ala Val Phe Ala Arg Gly Pro Gln
       450                 455                 460
   Ala His Leu Val His Gly Val Gln Glu Gln Thr Phe Ile Ala His Val
   465                 470                 475                 480
   Met Ala Phe Ala Ala Cys Leu Glu Pro Tyr Thr Ala Cys Asp Leu Ala
                   485                 490                 495
   Pro Arg Ala Gly Thr Thr Asp Ala Ala His Pro Gly Pro Ser Val Val
               500                 505                 510
   Pro Ala Leu Leu Pro Leu Leu Ala Gly Thr Leu Leu Leu Leu Gly Thr
           515                 520                 525
   Ala Thr Ala Pro
       530
<210> 17
   <211> 535
   <212> PRT
   <213> Homo sapiens
<400> 17
   Met Leu Gly Pro Cys Met Leu Leu Leu Leu Leu Leu Leu Gly Leu Arg
   1               5                   10                  15
   Leu Gln Leu Ser Leu Gly Ile Ile Pro Val Glu Glu Glu Asn Pro Asp
               20                  25                  30
   Phe Trp Asn Arg Glu Ala Ala Glu Ala Leu Gly Ala Ala Lys Lys Leu
           35                  40                  45
   Gln Pro Ala Gln Thr Ala Ala Lys Asn Leu Ile Ile Phe Leu Gly Asp
       50                  55                  60
   Gly Met Gly Val Ser Thr Val Thr Ala Ala Arg Ile Leu Lys Gly Gln
   65                  70                  75                  80
   Lys Lys Asp Lys Leu Gly Pro Glu Ile Pro Leu Ala Met Asp Arg Phe
                   85                  90                  95
   Pro Tyr Val Ala Leu Ser Lys Thr Tyr Asn Val Asp Lys His Val Pro
               100                 105                 110
   Asp Ser Gly Ala Thr Ala Thr Ala Tyr Leu Cys Gly Val Lys Gly Asn
           115                 120                 125
   Phe Gln Thr Ile Gly Leu Ser Ala Ala Ala Arg Phe Asn Gln Cys Asn
       130                 135                 140
   Thr Thr Arg Gly Asn Glu Val Ile Ser Val Met Asn Arg Ala Lys Lys
   145                 150                 155                 160
   Ala Gly Lys Ser Val Gly Val Val Thr Thr Thr Arg Val Gln His Ala
                   165                 170                 175
   Ser Pro Ala Gly Thr Tyr Ala His Thr Val Asn Arg Asn Trp Tyr Ser
   180 185 190
   Asp Ala Asp Val Pro Ala Ser Ala Arg Gln Glu Gly Cys Gln Asp Ile
   195 200 205
   Ala Thr Gln Leu Ile Ser Asn Met Asp Ile Asp Val Ile Leu Gly Gly
   210 215 220
   Gly Arg Lys Tyr Met Phe Arg Met Gly Thr Pro Asp Pro Glu Tyr Pro
   225 230 235 240
   Asp Asp Tyr Ser Gln Gly Gly Thr Arg Leu Asp Gly Lys Asn Leu Val
   245 250 255
   Gln Glu Trp Leu Ala Lys Arg Gln Gly Ala Arg Tyr Val Trp Asn Arg
   260 265 270
   Thr Glu Leu Met Gln Ala Ser Leu Asp Pro Ser Val Thr His Leu Met
   275 280 285
   Gly Leu Phe Glu Pro Gly Asp Met Lys Tyr Glu Ile His Arg Asp Ser
   290 295 300
   Thr Leu Asp Pro Ser Leu Met Glu Met Thr Glu Ala Ala Leu Arg Leu
   305 310 315 320
   Leu Ser Arg Asn Pro Arg Gly Phe Phe Leu Phe Val Glu Gly Gly Arg
   325 330 335
   Ile Asp His Gly His His Glu Ser Arg Ala Tyr Arg Ala Leu Thr Glu
   340 345 350
   Thr Ile Met Phe Asp Asp Ala Ile Glu Arg Ala Gly Gln Leu Thr Ser
   355 360 365
   Glu Glu Asp Thr Leu Ser Leu Val Thr Ala Asp His Ser His Val Phe
   370 375 380
   Ser Phe Gly Gly Tyr Pro Leu Arg Gly Ser Ser Ile Phe Gly Leu Ala
   385 390 395 400
   Pro Gly Lys Ala Arg Asp Arg Lys Ala Tyr Thr Val Leu Leu Tyr Gly
   405 410 415
   Asn Gly Pro Gly Tyr Val Leu Lys Asp Gly Ala Arg Pro Asp Val Thr
   420 425 430
   Glu Ser Glu Ser Gly Ser Pro Glu Tyr Arg Gln Gln Ser Ala Val Pro
   435 440 445
   Leu Asp Glu Glu Thr His Ala Gly Glu Asp Val Ala Val Phe Ala Arg
   450 455 460
   Gly Pro Gln Ala His Leu Val His Gly Val Gln Glu Gln Thr Phe Ile
   465 470 475 480
   Ala His Val Met Ala Phe Ala Ala Cys Leu Glu Pro Tyr Thr Ala Cys
   485 490 495
   Asp Leu Ala Pro Pro Ala Gly Thr Thr Asp Ala Ala His Pro Gly Arg
   500 505 510
   Ser Val Val Pro Ala Leu Leu Pro Leu Leu Ala Gly Thr Leu Leu Leu
   515 520 525
   Leu Glu Thr Ala Thr Ala Pro
   530 535
<210> 18
   <211> 532
   <212> PRT
   <213> Homo sapiens
<400> 18
   Met Gln Gly Pro Trp Val Leu Leu Leu Leu Gly Leu Arg Leu Gln Leu
   1 5 10 15
   Ser Leu Gly Ile Ile Pro Val Glu Glu Glu Asn Pro Asp Phe Trp Asn
   20 25 30
   Arg Gln Ala Ala Glu Ala Leu Gly Ala Ala Lys Lys Leu Gln Pro Ala
   35 40 45
   Gln Thr Ala Ala Lys Asn Leu Ile Ile Phe Leu Gly Asp Gly Met Gly
   50 55 60
   Val Ser Thr Val Thr Ala Ala Arg Ile Leu Lys Gly Gln Lys Lys Asp
   65 70 75 80
   Lys Leu Gly Pro Glu Thr Phe Leu Ala Met Asp Arg Phe Pro Tyr Val
   85 90 95
   Ala Leu Ser Lys Thr Tyr Ser Val Asp Lys His Val Pro Asp Ser Gly
   100 105 110
   Ala Thr Ala Thr Ala Tyr Leu Cys Gly Val Lys Gly Asn Phe Gln Thr
   115 120 125
   Ile Gly Leu Ser Ala Ala Ala Arg Phe Asn Gln Cys Asn Thr Thr Arg
   130 135 140
   Gly Asn Glu Val Ile Ser Val Met Asn Arg Ala Lys Lys Ala Gly Lys
   145 150 155 160
   Ser Val Gly Val Val Thr Thr Thr Arg Val Gln His Ala Ser Pro Ala
   165 170 175
   Gly Ala Tyr Ala His Thr Val Asn Arg Asn Trp Tyr Ser Asp Ala Asp
   180 185 190
   Val Pro Ala Ser Ala Arg Gln Glu Gly Cys Gln Asp Ile Ala Thr Gln
   195 200 205
   Leu Ile Ser Asn Met Asp Ile Asp Val Ile Leu Gly Gly Gly Arg Lys
   210 215 220
   Tyr Met Phe Pro Met Gly Thr Pro Asp Pro Glu Tyr Pro Asp Asp Tyr
   225 230 235 240
   Ser Gln Gly Gly Thr Arg Leu Asp Gly Lys Asn Leu Val Gln Glu Trp
   245 250 255
   Leu Ala Lys His Gln Gly Ala Arg Tyr Val Trp Asn Arg Thr Glu Leu
   260 265 270
   Leu Gln Ala Ser Leu Asp Pro Ser Val Thr His Leu Met Gly Leu Phe
   275 280 285
   Glu Pro Gly Asp Met Lys Tyr Glu Ile His Arg Asp Ser Thr Leu Asp
   290 295 300
   Pro Ser Leu Met Glu Met Thr Glu Ala Ala Leu Leu Leu Leu Ser Arg
   305 310 315 320
   Asn Pro Arg Gly Phe Phe Leu Phe Val Glu Gly Gly Arg Ile Asp His
   325 330 335
   Gly His His Glu Ser Arg Ala Tyr Arg Ala Leu Thr Glu Thr Ile Met
   340 345 350
   Phe Asp Asp Ala Ile Glu Arg Ala Gly Gln Leu Thr Ser Glu Glu Asp
   355 360 365
   Thr Leu Ser Leu Val Thr Ala Asp His Ser His Val Phe Ser Phe Gly
   370 375 380
   Gly Tyr Pro Leu Arg Gly Ser Ser Ile Phe Gly Leu Ala Pro Gly Lys
   385 390 395 400
   Ala Arg Asp Arg Lys Ala Tyr Thr Val Leu Leu Tyr Gly Asn Gly Pro
   405 410 415
   Gly Tyr Val Leu Lys Asp Gly Ala Arg Pro Asp Val Thr Glu Ser Glu
   420 425 430
   Ser Gly Ser Pro Glu Tyr Arg Gln Gln Ser Ala Val Pro Leu Asp Gly
   435 440 445
   Glu Thr His Ala Gly Glu Asp Val Ala Val Phe Ala Arg Gly Pro Gln
   450 455 460
   Ala His Leu Val His Gly Val Gln Glu Gln Thr Phe Ile Ala His Val
   465 470 475 480
   Met Ala Phe Ala Ala Cys Leu Glu Pro Tyr Thr Ala Cys Asp Leu Ala
   485 490 495
   Pro Arg Ala Gly Thr Thr Asp Ala Ala His Pro Gly Pro Ser Val Val
   500 505 510
   Pro Ala Leu Leu Pro Leu Leu Ala Gly Thr Leu Leu Leu Leu Gly Thr
   515 520 525
   Ala Thr Ala Pro
   530
<210> 19
   <211> 528
   <212> PRT
   <213> Homo sapiens
<400> 19
   Met Gln Gly Pro Trp Val Leu Leu Leu Leu Gly Leu Arg Leu Gln Leu
   1               5                   10                  15
   Ser Leu Gly Val Ile Pro Ala Glu Glu Glu Asn Pro Ala Phe Trp Asn
               20                  25                  30
   Arg Gln Ala Ala Glu Ala Leu Asp Ala Ala Lys Lys Leu Gln Pro Ile
           35                  40                  45
   Gln Lys Val Ala Lys Asn Leu Ile Leu Phe Leu Gly Asp Gly Leu Gly
       50                  55                  60
   Val Pro Thr Val Thr Ala Thr Arg Ile Leu Lys Gly Gln Lys Asn Gly
   65                  70                  75                  80
   Lys Leu Gly Pro Glu Thr Pro Leu Ala Met Asp Arg Phe Pro Tyr Leu
                   85                  90                  95
   Ala Leu Ser Lys Thr Tyr Asn Val Asp Arg Gln Val Pro Asp Ser Ala
               100                 105                 110
   Ala Thr Ala Thr Ala Tyr Leu Cys Gly Val Lys Ala Asn Phe Gln Thr
           115                 120                 125
   Ile Gly Leu Ser Ala Ala Ala Arg Phe Asn Gln Cys Asn Thr Thr Arg
       130                 135                 140
   Gly Asn Glu Val Ile Ser Val Met Asn Arg Ala Lys Gln Ala Gly Lys
   145                 150                 155                 160
   Ser Val Gly Val Val Thr Thr Thr Arg Val Gln His Ala Ser Pro Ala
                   165                 170                 175
   Gly Thr Tyr Ala His Thr Val Asn Arg Asn Trp Tyr Ser Asp Ala Asp
               180                 185                 190
   Met Pro Ala Ser Ala Arg Gln Glu Gly Cys Gln Asp Ile Ala Thr Gln
           195                 200                 205
   Leu Ile Ser Asn Met Asp Ile Asp Val Ile Leu Gly Gly Gly Arg Lys
       210                 215                 220
   Tyr Met Phe Pro Met Gly Thr Pro Asp Pro Glu Tyr Pro Ala Asp Ala
   225                 230                 235                 240
   Ser Gln Asn Gly Ile Arg Leu Asp Gly Lys Asn Leu Val Gln Glu Trp
                   245                 250                 255
   Leu Ala Lys His Gln Gly Ala Trp Tyr Val Trp Asn Arg Thr Glu Leu
               260                 265                 270
   Met Gln Ala Ser Leu Asp Gln Ser Val Thr His Leu Met Gly Leu Phe
           275                 280                 285
   Glu Pro Gly Asp Thr Lys Tyr Glu Ile His Arg Asp Pro Thr Leu Asp
       290                 295                 300
   Pro Ser Leu Met Glu Met Thr Glu Ala Ala Leu Arg Leu Leu Ser Arg
   305                 310                 315                 320
   Asn Pro Arg Gly Phe Tyr Leu Phe Val Glu Gly Gly Arg Ile Asp His
                   325                 330                 335
   Gly His His Glu Gly Val Ala Tyr Gln Ala Leu Thr Glu Ala Val Met
               340                 345                 350
   Phe Asp Asp Ala Ile Glu Arg Ala Gly Gln Leu Thr Ser Glu Glu Asp
           355                 360                 365
   Thr Leu Thr Leu Val Thr Ala Asp His Ser His Val Phe Ser Phe Gly
       370                 375                 380
   Gly Tyr Thr Leu Arg Gly Ser Ser Ile Phe Gly Leu Ala Pro Ser Lys
   385                 390                 395                 400
   Ala Gln Asp Ser Lys Ala Tyr Thr Ser Ile Leu Tyr Gly Asn Gly Pro
                   405                 410                 415
   Gly Tyr Val Phe Asn Ser Gly Val Arg Pro Asp Val Asn Glu Ser Glu
               420                 425                 430
   Ser Gly Ser Pro Asp Tyr Gln Gln Gln Ala Ala Val Pro Leu Ser Ser
           435                 440                 445
   Glu Thr His Gly Gly Glu Asp Val Ala Val Phe Ala Arg Gly Pro Gln
       450                 455                 460
   Ala His Leu Val His Gly Val Gln Glu Gln Ser Phe Val Ala His Val
   465                 470                 475                 480
   Met Ala Phe Ala Ala Cys Leu Glu Pro Tyr Thr Ala Cys Asp Leu Ala
                   485                 490                 495
   Pro Pro Ala Cys Thr Thr Asp Ala Ala His Pro Val Ala Ala Ser Leu
               500                 505                 510
   Pro Leu Leu Ala Gly Thr Leu Leu Leu Leu Gly Ala Ser Ala Ala Pro
           515                 520                 525
<210> 20
   <211> 227
   <212> PRT
   <213> Artificial Sequence
<220>
   <223> Synthetic Construct
<400> 20
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Glu Glu Met Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225